(19) Europäisches Patentamt
European Patent Office
Office européen des brevets

(11) Publication number: **0 341 602 A2**

# (12) EUROPEAN PATENT APPLICATION

(21) Application number: 89108139.0

(22) Date of filing: 05.05.89

(51) Int. Cl.4: C07K 5/06 , C07D 233/64 , C07D 231/12 , C07D 295/00 , C07D 401/12 , C07D 401/14 , A61K 37/64 , A61K 31/395 , C07C 103/38 , C07C 91/14 , C07C 103/50

The application is published incomplete as filed (Article 93 (2) EPC). The point in the description at which the omission obviously occurs has been left blank. (Page 46 of the originally filed description is missing)

The title of the invention has been amended (Guidelines for Examination in the EPO, A-III, 7.3).

A request for correction of the description has been filed pursuant to Rule 88 EPC. A decision on the request will be taken during the proceedings before the Examining Division (Guidelines for Examination in the EPO, A-V, 2.2).

(30) Priority: 09.05.88 US 192737

(43) Date of publication of application: 15.11.89 Bulletin 89/46

(84) Designated Contracting States:
ES GR

(71) Applicant: **ABBOTT LABORATORIES**
One Abbott Park Road
Abbott Park, IL 60064-3500(US)

(72) Inventor: Rosenberg, Saul H.
1110 Fairlawn Drive
Libertyville, Illinois 60048(US)
Inventor: Dellaria, Joseph F.
2512 Timber Lane
Lindenhurst, Illinois 60046(US)
Inventor: Plattner, Jacob John
1301 St. William Drive
Libertyville, Illinois 60048(US)
Inventor: Baker, William R.
1408 Juliet Lane
Libertyville, Illinois 60048(US)

(74) Representative: Modiano, Guido et al
MODIANO, JOSIF, PISANTY & STAUB
Modiano & Associati Via Meravigli, 16
I-20123 Milan(IT)

## (54) Renin-inhibiting functionalized peptidyl aminodiols and - triols.

(57) A renin inhibiting compound of the formula:

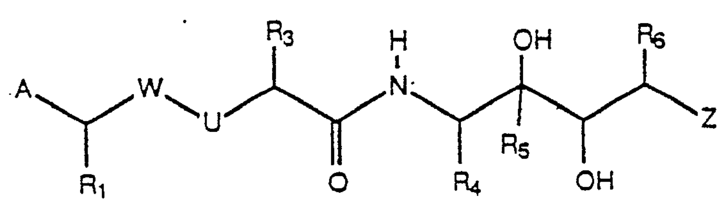

I

EP 0 341 602 A2

or a pharmaceutically acceptable salt, ester or prodrug thereof.

# FUNCTIONALIZED PEPTIDYL AMINODIOLS AND -TRIOLS

## Technical Field

This application is a continuation-in-part of U.S. patent application Serial No. 192,737, filed May 9, 1988 which is a continuation-in-part of U.S. patent application Serial No. 943,566, filed December 31, 1986, now abandoned, which is a continuation-in-part of U.S. patent application Serial No. 818,715, filed January 16, 1986, now abandoned, which is a continuation-in-part of U.S. patent application Serial No. 693,951, filed January 23, 1985, now abandoned.

The present invention relates to novel compounds and compositions which inhibit renin, processes for making such compounds, synthetic intermediates employed in these processes and a method of treating hypertension, congestive heart failure and glaucoma with such compounds.

## Background Art

Renin is a proteolytic enzyme synthesized and stored principally in a specific part of the kidney called the juxtaglomerular apparatus. Any of three different physiologic circumstances may cause the release of renin into the circulation: (a) a decrease in the blood pressure entering or within the kidney itself; (b) a decrease in the blood volume in the body; or (c) a fall in the concentration of sodium in the distal tubules of the kidney.

When renin is released into the blood from the kidney, the renin-angiotensin system is activated, leading to vasoconstriction and conservation of sodium, both of which result in increased blood pressure. The renin acts on a circulating protein, angiotensinogen, to cleave out a fragment called angiotensin I (AI). AI itself has only slight pharamacologic activity but, after additional cleavage by a second enzyme, angiotensin converting enzyme (ACE), forms the potent molecule angiotensin II (AII). The major pharmacological effects of AII are vasoconstriction and stimulation of the adrenal cortex to release aldosterone, a hormone which causes sodium retention. Sodium retention causes blood volume to increase, which leads to hypertension. AII is cleaved by an aminopeptidase to form angiotensin II (AIII), which, compared to AII, is a less potent vasoconstrictor but a more potent inducer of aldosterone release.

Inhibitors of renin have been sought as agents for control of hypertension and as diagnostic agents for identification of cases of hypertension due to renin excess.

With these objectives in mind, the renin-angiotensin system has been modulated or manipulated, in the past, with ACE inhibitors. However, ACE acts on several substrates other than angiotensin I (AI), most notably the kinins which cause such undesirable side effects as pain, "leaky" capillaries, prostaglandin release and a variety of behavorial and neurologic effects. Further, ACE inhibition leads to the accumulation of AI. Although AI has much less vasoconstrictor activity than AII, its presence may negate some of the hypotensive effects of the blockade of AII synthesis.

Inhibition of other targets in the renin-angiotensin system such as AII with compounds such as saralasin can block AII activity, but would leave unimpaired and perhaps enhance the hypertensive effects of AIII.

On the other hand, there are no known side effects which result when renin is inhibited from acting on its substrate. Considerable research efforts have thus been carried out to develop useful inhibitors of renin. Past research efforts have been directed to renin antibodies, pepstatin, phospholipids and substrate analogs such as tetrapeptides and octapeptides to tridecapeptides. These inhibitors either demonstrate poor activity in inhibiting renin production or poor specificity for inhibiting renin only. However, Boger et al. have reported that statine-containing peptides possess potent and specific renin-inhibiting activity (Nature, Vol. 303, p. 81, 1983). In addition, Szelke and co-workers have described polypeptide analogs containing a non-peptide link (Nature, Vol. 299, p. 555, 1982) which also cause potent renin inhibition and show a high specificity for this enzyme.

Copending U.S. patent application, USSN 191,714, filed May 9, 1988, which is hereby incorporated by reference, discloses carboxy-terminal $-NH_2$ and -NH(loweralkyl) substituted-diol peptides as renin inhibitors.

Copending U.S. patent application, USSN 132,356, filed December 18, 1987, which is hereby incorporated by reference, discloses carboxy-terminal substituted-diol peptides as renin inhibitors.

Morishima, Biochem. Biophys. Res. Commun. 159 999 (1989), discloses (2S, 3R, 4S)-4-(L-N-((2S)3-ethylsulfonyl-2-(1-naphthylmethyl)propionyl)norleucyl)amino-5-cyclohexyl-1-morpholino-2,3-pentanediol as a renin inhibitor.

In addition, the following references disclose renin inhibitors incorporating a substituted-diol functional

group:
Hanson, Biochem. Biophys. Res. Commun. 132155 (1985);
Matsueda, U.S. Patent No. 4,548,926, issued October 22, 1985;
Luly, European Patent Application No. EP0189203, published July 30, 1986;
Rosenberg, European Patent Application No. EP0230266, published July 29, 1987;
Luly, U.S. Patent No. 4,680,284, issued July 14, 1987; and
Fung, PCT Patent Application No. WO88/05050, published July 14, 1988.

Watkins, PCT Patent Application No. WO87/02581, published May 7,1987, discloses the use of renin inhibitors for the treatment of glaucoma.

Disclosure of the Invention

In accordance with the present invention, there are renin inhibiting compounds of the formula:

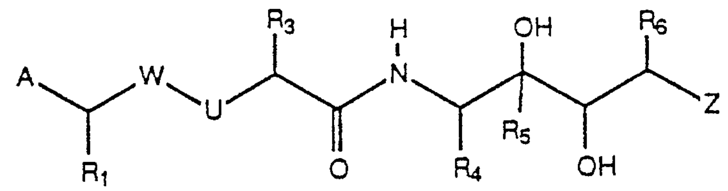

I

or a pharmaceutically acceptable salt, ester or prodrug thereof.

A is
(1) hydrogen,
(2) loweralkyl,
(3) carboxy,
(4) alkoxycarbonyl,
(5) $-OR_8$ or $-SR_8$

wherein $R_8$ is
(i) hydrogen,
(ii) loweralkyl,
(iii) aminoalkyl,
(iv) (alkyl)aminoalkyl,
(v) dialkylaminoalkyl,
(vi) (alkoxy)aminoalkyl,
(vii) (alkoxy) (alkyl)aminoalkyl,
(viii) phenylalkyl,
(ix) (substituted phenyl)alkyl wherein substituted phenyl is as defined above,
(x) naphthylalkyl,
(xi) (substituted naphthyl)alkyl wherein substituted naphthyl is as defined above,
(xii) heterocyclic,
(xiii) (heterocyclic)alkyl, or
(xiv) (heterocyclic)C(O)-,

(6) heterocyclic,
(7) functionalized alkyl,
(8) functionalized amino,
(9) functionalized carbonyl,
(10) functionalized sulfonyl or
(11) functionalized hydroxyalkyl.

Functionalized alkyl groups are selected from the group consisting of:
(1) aminoalkyl,
(2) (alkyl)aminoalkyl,

(3) dialkylaminoalkyl,
(4) (alkoxy)aminoalkyl,
(5) (alkoxy) (alkyl)aminoalkyl,
(6) phenylalkyl,
(7) (substituted phenyl)alkyl wherein the phenyl ring is substituted with one, two or three substituents independently selected from loweralkoxy, loweralkyl, amino, alkylamino, dialkylamino, hydroxy, halo, mercapto, nitro, thioalkoxy, carboxaldehyde, carboxy, alkoxycarbonyl and carboxamide,
(8) naphthylalkyl,
(9) (substituted naphthyl)alkyl wherein the naphthyl ring is substituted with one, two or three substituents independently selected from loweralkoxy, loweralkyl, amino, alkylamino, dialkylamino, hydroxy, halo, mercapto, nitro, thioalkoxy, carboxaldehyde, carboxy, alkoxycarbonyl and carboxamide, and
(10) (heterocyclic)alkyl.

Functionalized amino groups include $-NR_9R_{10}$ wherein $R_9$ and $R_{10}$ are independently selected from
(i) hydrogen,
(ii) hydroxy,
(iii) alkoxy,
(iv) loweralkyl,
(v) aminoalkyl,
(vi) cyanoalkyl and
(vii) hydroxyalkyl.

Functionalized carbonyl and functionalized sulfonyl groups include $R_{11}C(O)B-$ and $R_{11}S(O)_2B-$, respectively,
wherein B is
(i) NH,
(ii) -N(alkyl)-,
(iii) S,
(iv) O,
(v) $-CH_2-$,
(vi) $-NHCH_2-$ or
(vii) $-CH(OR_{52})-$ wherein $R_{52}$ is hydrogen, loweralkyl or loweralkylcarbonyl, and
$R_{11}$ is
(i) hydrogen,
(ii) loweralkyl,
(iii) haloalkyl,
(iv) cycloalkyl,
(v) phenyl,
(vi) substituted phenyl independently as defined above,
(vii) naphthyl,
(viii) substituted naphthyl independently as defined above,
(ix) phenylalkyl,
(x) (substituted phenyl)alkyl wherein substituted phenyl is indepedently as defined above,
(xi) naphthylalkyl,
(xii) (substitutued naphthyl)alkyl wherein substituted naphthyl is independently as defined above,
(xiii) alkoxy,
(xiv) alkenyloxy,
(xv) hydroxyalkoxy,
(xvi) dihydroxyalkoxy,
(xvii) phenylalkoxy,
(xviii) (substituted phenyl)alkoxy wherein substituted phenyl is independently as defined above,
(xix) naphthylalkoxy,
(xx) (substituted naphthyl)alkoxy wherein substituted naphthyl is independently as defined above,
(xxi) phenylalkoxyalkyl,
(xxii) (substituted phenyl)alkoxyalkyl wherein substituted phenyl is independently as defined above,
(xxiii) naphthylalkoxyalkyl,
(xxiv) (substituted naphthyl)alkoxyalkyl wherein substituted naphthyl is independently as defined above,

(xxv) thioalkoxyalkyl,
(xxvi) loweralkylsulfinylalkyl,
(xxvii) loweralkylsulfonylalkyl,
(xxviii) phenylthioalkyl,
(xxix) (substituted phenyl)thioalkyl wherein substituted phenyl is independently as defined above,
(xxx) naphthylthioalkyl,
(xxxi) (substituted naphthyl)thioalkyl wherein substituted naphthyl is independently as defined above,
(xxxii) phenylsulfonylalkyl,
(xxxiii) (substituted phenyl)sulfonylalkyl wherein substituted phenyl is independently as defined above,
(xxxiv) naphthylsulfonylalkyl,
(xxxv) (substituted naphthyl)sulfonylalkyl wherein substituted naphthyl is independently as defined above,
(xxxvi) amino,
(xxxvii) alkylamino,
(xxxviii) dialkylamino,
(xxxix) (hydroxyalkyl) (alkyl)amino,
(xl) (dihydroxyalkyl) (alkyl)amino,
(xli) aminoalkyl,
(xlii) alkoxycarbonylalkyl,
(xliii) carboxyalkyl,
(xliv) (N-protected)aminoalkyl,
(xlv) alkylaminoalkyl,
xlvi) (N-protected) (alkyl)aminoalkyl,
(xlvii) dialkylaminoalkyl,
(xlviii) (heterocyclic)alkyl,
(xlix) heterocyclic,
(l) aminocycloalkyl,
(li) aminoalkylamino,
(lii) (dialkylaminoalkyl) (alkyl)amino,
(liii) phenylalkylamino,
(liv) (substituted phenyl)alkylamino wherein substituted phenyl is independently as defined above,
(lv) naphthylalkylamino,
(lvi) (substituted naphthyl)alkylamino wherein substituted naphthyl is independently as defined above,
(lvii) (phenylalkyl) (alkyl)amino,
(lviii) ((substituted phenyl)alkyl)(alkyl)amino wherein substituted phenyl is independently as defined above,
(lix) (naphthylalkyl) (alkyl)amino,
(lx) ((substituted naphthyl)alkyl)(alkyl)amino wherein substituted naphthyl is independently as defined above,
(lxi) alkoxyalkyl(alkyl)amino,
(lxii) (polyalkoxy)alkyl(alkyl)amino,
(lxiii) di-(alkoxyalkyl)amino,
(lxiv) di-(hydroxyalkyl)amino,
(lxv) di-((polyalkoxy)alkyl)amino,
(lxvi) ((heterocyclic)alkyl) (alkyl)amino,
(lxvii) ((heterocyclic)alkyl)amino,
(lxviii) (heterocyclic) (alkyl)amino,
(lxix) (alkylaminoalkyl) (alkyl)amino,
(lxx) dialkylaminoalkyl) (alkyl)amino,
(lxxi) ((alkoxy) (alkyl)aminoalkyl)(alkyl)amino,
(lxxii) ((alkoxy)aminoalkyl) (alkyl)amino,
(lxxiii) polyalkoxy,
(lxxiv) (polyalkoxy)alkyl,
(lxxv) carboxyalkoxyalkyl,
(lxxvi) (alkoxycarbonyl)alkoxyalkyl),
(lxxvii) carboxyalkylamino,
(lxxvii) alkoxycarbonylalkylamino,

(lxxviii) (amino)carboxyalkyl,
(lxxix) (amino)carboxyalkylamino,
(lxxx) ((N-protected)amino)carboxyalkyl,
(lxxxi) ((N-protected)amino)carboxyalkylamino,
(lxxxii) (alkylamino)carboxyalkyl,
(lxxxiii) (alkylamino)carboxyalkylamino,
(lxxxiv) (((N-protected)alkylamino)carboxyalkyl,
(lxxxv) ((N-protected)alkylamino)carboxyalkylamino,
(lxxxvi) (dialkylamino)carboxyalkyl,
(lxxxvii) (dialkylamino)carboxyalkylamino,
(lxxxviii) (amino)alkoxycarbonylalkyl,
(lxxxix) (amino)alkoxycarbonylalkylamino,
(xc) ((N-protected)amino)alkoxycarbonylalkyl,
(xci) ((N-protected)amino)alkoxycarbonylalkylamino,
(xcii) (alkylamino)alkoxycarbonylalkyl,
(xciii) (alkylamino)alkoxycarbonylalkyl,
(xciv) (alkylamino)alkoxycarbonylalkylamino,
(xcv) (((N-protected)alkylamino)alkoxycarbonylalkyl,
(xcvi) ((N-protected)alkylamino)alkoxycarbonylalkylamino,
(xcvii) (dialkylamino)alkoxycarbonylalkyl or
(xcviii) ((dialkylamino)alkoxycarbonylalkylamino.

Functionalized hydroxyalkyl groups include $R_{41}CH(OH)CH_2$- and $R_{41}CH(OH)CH(OH)$- wherein $R_{41}$ is
(i) loweralkyl,
(ii) cycloalkyl,
(iii) phenyl,
(iv) substituted phenyl independently as defined above,
(v) naphthyl,
(vi) substituted naphthyl independently as defined above,
(vii) phenylalkyl,
(viii) (substituted phenyl)alkyl wherein substituted phenyl is independently as defined above,
(ix) naphthylalkyl,
(x) (substituted naphthyl) alkyl wherein substituted naphthyl is independently as defined above,
(xi) phenylalkoxyalkyl,
(xii) (substituted phenyl)alkoxyalkyl wherein substituted phenyl is independently as defined above,
(xiii) naphthylalkoxyalkyl,
(xiv) (substituted naphthyl)alkoxyalkyl wherein substituted naphthyl is independently as defined above,
(xv) thioalkoxyalkyl,
(xvi) loweralkylsulfinylalkyl,
(xvii) loweralkylsulfonylalkyl,
(xviii) phenylthioalkyl,
(xix) (substituted phenyl)thioalkyl wherein substituted phenyl is independently as defined above,
(xx) naphthylthioalkyl,
(xxi) (substituted naphthyl)thioalkyl wherein substituted naphthyl is independently as defined above,
(xxii) phenylsulfonylalkyl,
(xxiii) (substituted phenyl)sulfonylalkyl wherein substituted phenyl is independently as defined above,
(xxiv) naphthylsulfonylalkyl,
(xxv) (substituted naphthyl)sulfonylalkyl wherein substituted naphthyl is independently as defined above,
(xxvi) aminoalkyl,
(xxvii) alkoxycarbonylalkyl,
(xxviii) carboxyalkyl,
(xxix) (N-protected)aminoalkyl,
(xxx) alkylaminoalkyl,
(xxxi) (N-protected) (alkyl)aminoalkyl,
(xxxii) dialkylaminoalkyl,

(xxxiii) (heterocyclic)alkyl,
(xxxiv) heterocyclic,
(xxxv) aminocycloalkyl or
(xxxvi) (polyalkoxy)alkyl.

W is
(1) -C(O)-,
(2) -CH(OH)- or
(3) $-N(R_2)-$ wherein $R_2$ is hydrogen or loweralkyl.

U is
(1) -C(O)-,
(2) $-CH_2-$ or
(3) $-N(R_2)-$ wherein $R_2$ is hydrogen or loweralkyl, with the proviso that when W is -CH(OH)-then U is $-CH_2-$ and with the proviso that U is -C(O)- or $-CH_2-$ when W is $-N(R_2)-$.

$R_1$ is
(1) loweralkyl,
(2) cycloalkylalkyl,
(3) benzyl,
(4) (alpha, alpha)-dimethylbenzyl,
(5) (susbstituted phenyl)methyl wherein substituted phenyl is independently as defined above,
(6) (alpha, alpha)-dimethyl-(substituted phenyl)methyl wherein substituted phenyl is independently as defined above,
(7) (1-naphthyl)methyl,
(8) (2-naphthyl)methyl,
(9) (heterocyclic)methyl,
(10) phenethyl,
(11) 1-benzyloxyethyl,
(12) phenoxy,
(13) thiophenoxy or
(14) anilino, provided that B is -CH2- or -CH(OH)- or A is hydrogen when $R_1$ is phenoxy, thiophenoxy or anilino.

$R_3$ is
(1) loweralkyl,
(2) loweralkenyl,
(3) hydroxyalkyl,
(4) alkoxyalkyl,
(5) ((alkoxy)alkoxy)alkyl,
(6) carboxyalkyl,
(7) (thioalkoxy)alkyl,
(8) azidoalkyl,
(9) aminoalkyl,
(10) (alkyl)aminoalkyl,
(11) dialkylaminoalkyl,
(12) (alkoxy) (alkyl)aminoalkyl,
(13) (alkoxy)aminoalkyl,
(14) benzyl or
(15) (heterocyclic)methyl.

$R_4$ is
(1) loweralkyl,
(2) cycloalkylmethyl or
(3) benzyl.

$R_5$ and $R_6$ are independently selected from
(1) hydrogen and
(2) loweralkyl.

Z is
(1) $-N(R_7)(R_{12})$ wherein
$R_7$ is
(1) loweralkyl,
(2) haloalkyl or

(3) alkoxy; and

$R_{12}$ is

(1) hydrogen,

(2) loweralkyl or

(3) haloalkyl; or

(2) heterocyclic.

The chiral centers of the compounds of the invention may have either the "R" or "S" configuration. The terms "R" and "S" configuration are as defined by IUPAC 1974 Recommendations for Section E, Fundamental Stereochemistry, Pure Appl. Chem. (1976) 45, 13-30.

The term "loweralkyl" as used herein refers to straight or branched chain alkyl radicals containing from 1 to 7 carbon atoms including but not limited to methyl, ethyl, n-propyl, iso-propyl, n-butyl, iso-butyl, sec-butyl, n-pentyl, 2-methylbutyl, 2,2-dimethylpropyl, n-hexyl, 2-methylpentyl, 2,2-dimethylbutyl, n-heptyl, and the like.

The term "cycloalkyl" as used herein refers to an alicyclic ring having 3 to 7 carbon atoms.

The term "cycloalkylalkyl" as used herein refers to a cycloalkyl residue appended to a loweralkyl radical and includes but is not limited to cyclohexylmethyl and cyclopentylmethyl.

The term "phenylalkyl" as used herein refers to a phenyl group appended to a loweralkyl radical, including, but not limited to benzyl, phenethyl and the like.

The term "(substituted phenyl)alkyl" as used herein refers to a substituted phenyl group appended to a loweralkyl radical wherein the phenyl ring is substituted with one, two or three substituents independently selected from the group loweralkoxy, loweralkyl, amino, loweralkylamino, dialkylamino, hydroxy, halo, mercapto, nitro, thioalkoxy, carboxaldehyde, carboxy, carboalkoxy and carboxamide, including, but not limited to halobenzyl, alkoxybenzyl and the like.

The term "naphthylalkyl" as used herein refers to a naphthyl group appended to a loweralkyl radical, including, but not limited to 1-naphthylmethyl, 2-naphthylmethyl and the like.

The term "(substituted naphthyl)alkyl" as used herein refers to a substituted naphthyl group appended to a loweralkyl radical wherein the naphthyl ring is substituted with one, two or three substituents independently selected from the group loweralkoxy, loweralkyl, amino, loweralkylamino, dialkylamino, hydroxy, halo, mercapto, nitro, thioalkoxy, carboxaldehyde, carboxy, carboalkoxy and carboxamide, including, but not limited to halonaphthylmethyl, alkoxynaphthylmethyl and the like.

The term "(heterocyclic)alkyl" as used herein refers to an unsubstituted or substituted heterocyclic ring (as defined below) appended to a lower alkyl radical, including, but not limited to imidazolylmethyl, thiazolylmethyl and the like.

The term "hydroxyalkyl" as used herein refers to -OH appended to a loweralkyl radical.

The term "alkoxyalkyl" as used herein refers to an alkoxy group appended to a loweralkyl radical.

The term "thioalkoxyalkyl" as used herein refers to a thioalkoxy group appended to a loweralkyl radical.

The term "phenylalkoxy" as used herein refers to $R_{100}O$- wherein $R_{100}$ is a phenylalkyl groupincluding, but not limited to, benzyloxy and the like.

The term "phenylalkoxyalkyl" as used herein refers to a phenylalkoxy group appended to a loweralkyl radical, including, but not limited to phenylmethoxymethyl and the like.

The term "(substituted phenyl)alkoxy" as used herein refers to $R_{101}O$- wherein $R_{101}$ is a (substituted phenyl)alkyl group.

The term "(substituted phenyl)alkoxyalkyl" as used herein refers to a (substituted phenyl)alkoxy group appended to a loweralkyl radical, including, but not limited to 4-chlorophenylmethoxymethyl.

The term "naphthylalkoxy" as used herein refers to $R_{102}O$- wherein $R_{102}$ is a naphthylalkyl group.

The term "naphthylalkoxyalkyl" as used herein refers to a naphthylalkoxy group appended to a loweralkyl radical, including, but not limited to 1-naphthylmethoxymethyl and the like.

The term "(substituted naphthyl)alkoxy" as used herein refers to $R_{103}O$- wherein $R_{103}$ is a (substituted naphthyl)alkyl group.

The term "(substituted naphthyl)alkoxyalkyl" as used herein refers to a (substituted naphthyl)alkoxy group appended to a loweralky radical, including, but not limited to halonaphthylmethoxymethyl and the like.

The term "((alkoxy)alkoxy)alkyl" as used herein refers to an alkoxy group appended to an alkoxy group which is appended to a loweralkyl radical including, but not limited to, methoxymethoxymethyl and the like.

The term "polyalkoxyalkyl" as used herein refers to a polyalkoxy residue appended to a loweralkyl radical including, but not limited to, methoxyethoxymethoxymethyl and the like.

The term "aminoalkyl" as used herein refers to $-NH_2$ appended to a loweralkyl radical.

The term "alkylaminoalkyl" as used herein refers to $-NHR_{104}$ appended to a loweralkyl radical, wherein $R_{104}$ is a loweralkyl radical.

The term "dialkylaminoalkyl" as used herein refers to a dialkylamino group appended to a loweralkyl radical.

The term "aminocycloalkyl" as used herein refers to an $-NH_2$ appended to a cycloalkyl radical.

The term "(N-protected)aminoalkyl" as used herein refers to $-NHR_{13}$ appended to a loweralkyl group, wherein $R_{13}$ is an N-protecting group.

The term "(N-protected)(alkyl)aminoalkyl" as used herein refers to $-NR_{13}R_{14}$, which is appended to a loweralkyl radical, wherein $R_{13}$ is defined as above and $R_{14}$ is a loweralkyl group.

The term "alkoxycarbonylalkyl" as used herein refers to $R_{15}C(O)R_{16}$-, wherein $R_{15}$ is an alkoxy group and $R_{16}$ is a loweralkyl radical.

The term "carboxyalkyl" as used herein refers to a carboxylic acid group (-COOH) appended to a loweralkyl radical.

The term "cyanoalkyl" as used herein refers to -CN appended to a loweralkyl radical.

The term "(alkoxy)aminoalkyl" as used herein refers to an alkoxy group appended to an amino group which in turn is appended to a loweralkyl radical.

The term "(alkoxy) (alkyl)aminoalkyl" as used herein refers to an $-NR_{59}R_{60}$ group appended to a loweralkyl radical wherein $R_{59}$ is an alkoxy group and $R_{60}$ is a loweralkyl group.

The term "haloalkyl" as used herein refers to a loweralkyl radical in which one or more hydrogen atoms are replaced by halogen including, but not limited to, fluoromethyl, 2-chloroethyl, trifluoromethyl, 2,2-dichloroethyl and the like.

The term "azidoalkyl" as used herein refers to a $-N_3$ group appended to a loweralkyl radical.

The term "loweralkylsulfinylalkyl" as used herein refers to a $R_{61}S(O)$- group appended to a loweralkyl radical wherein $R_{61}$ is a loweralkyl group.

The term "loweralkylsulfonylalkyl" as used herein refers to a $R_{62}S(O)_2$- group appended to a loweralkyl radical wherein $R_{62}$ is a loweralkyl group.

The term "phenylthioalkyl" as used herein refers to a $R_{63}S$- group appended to a loweralkyl radical wherein $R_{63}$ is a phenyl group.

The term "(substituted phenyl)thioalkyl" as used herein refers to a $R_{64}S$- group appended to a loweralkyl radical wherein $R_{64}$ is a substituted phenyl group.

The term "naphthyl thioalkyl" as used herein refers to a $R_{65}S$- group appended to a loweralkyl radical wherein $R_{65}$ is a naphthyl group.

The term "(substituted naphthyl)thioalkyl" as used herein refers to a $R_{66}S$- group appended to a loweralkyl radical wherein $R_{66}$ is a substituted naphthyl group.

The term "phenylsulfonylalkyl" as used herein refers to a $R_{67}S(O)_2$- group appended to a loweralkyl radical wherein $R_{67}$ is a phenyl group.

The term "(substituted phenyl)sulfonylalkyl" as used herein refers to a $R_{68}S(O)_2$- group appended to a loweralkyl radical wherein $R_{68}$ is a substituted phenyl group.

The term "naphthylsulfonylalkyl" as used herein refers to a $R_{69}S(O)_2$- group appended to a loweralkyl group wherein $R_{69}$ is a naphthyl group.

The term "(substituted naphthyl)sulfonylalkyl" as used herein refers to a $R_{70}S(O)_2$- group appended to a loweralkyl group wherein $R_{70}$ is a substituted naphthyl group.

The term "amino" as used herein refers to an $-NH_2$ substituent.

The term "alkylamino" as used herein refers to $-NHR_{17}$, wherein $R_{17}$ is a loweralkyl group.

The term "dialkylamino" as used herein refers to $-NR_{18}R_{19}$, wherein $R_{18}$ and $R_{19}$ are independently selected from loweralkyl groups.

The term "phenylalkylamino" as used herein refers to a phenylalkyl group appended to an amino radical, including, but not limited to benzylamino and the like.

The term "(substituted phenyl)alkylamino" as used herein refers to a (substituted phenyl)alkyl group appended to an amino radical, including, but not limited to 4-chlorobenzylamino and the like.

The term "napthylalkylamino" as used herein refers to a naphthylalkyl group appended to an amino radical, including, but not limited to 1-naphthylmethylamino and the like.

The term "(substituted naphthyl)alkylamino" as used herein refers to a (substituted naphthyl)alkyl group appended to an amino radical.

The term "(phenylalkyl) (alkyl)amino" as used herein refers to $R_{21}R_{22}N$-, wherein $R_{21}$ is a phenylalkyl residue and $R_{22}$ is a loweralkyl residue.

The term "((substituted phenyl)alkyl)(alkyl)amino" as used herein refers to $R_{53}R_{54}N$- wherein $R_{53}$ is a (substituted phenyl)alkyl group and $R_{54}$ is a loweralkyl group.

The term "(naphthylalkyl) (alkyl)amino" as used herein refers to $R_{55}R_{56}N$- wherein $R_{55}$ is a naphthylalkyl group and $R_{56}$ is a loweralkyl group.

The term "((substituted naphthyl)alkyl)(alkyl)amino" as used herein refers to $R_{57}R_{58}N$- wherein $R_{57}$ is a (substituted naphthyl)alkyl group and $R_{58}$ is a loweralky group.

The term "aminoalkylamino" as used herein refers to $R_{23}NH$-, where $R_{23}$ is an aminoalkyl residue.

The term "(dialkylaminoalkyl) (alkyl)amino" as used herein refers to $R_{24}R_{25}N$-, wherein $R_{24}$ is a dialkylamino residue appended to a loweralkyl group and $R_{25}$ is a loweralkyl group.

The term "(hydroxyalkyl) (alkyl)amino" as used herein refers to $-NR_{26}R_{27}$ wherein $R_{26}$ is a hydroxyalkyl group and $R_{27}$ is a loweralkyl group.

The term "(di-hydroxyalkyl) (alkyl)amino" as used herein refers to a loweralkyl group which is disubstituted with -OH radicals appended to an amino group, which amino group also has appended another loweralkyl group.

The term "di-(hydroxyalkyl)amino" as used herein refers to $R_{28}R_{29}N$-, wherein $R_{28}$ and $R_{29}$ are hydroxyalkyl residues.

The term "(alkoxyalkyl) (alkyl)amino" as used herein refers to $R_{30}R_{31}N$-, wherein $R_{30}$ is an alkoxyalkyl group and $R_{31}$ is a loweralkyl group.

The term "di-(alkoxyalkyl)amino" as used herein refers to $R_{32}R_{33}N$-, wherein $R_{32}$ and $R_{33}$ are alkoxy groups appended to a loweralkyl residue.

The term "di-(polyalkoxyalkyl)amino" as used herein refers to $R_{34}R_{35}N$-, wherein $R_{34}$ and $R_{35}$ are polyalkoxy residues appended to loweralkyl residues.

The term "((polyalkoxy)alkyl) (alkyl)amino" as used herein refers to $R_{36}R_{37}N$-, wherein $R_{36}$ is a polyalkoxy residue appended to a loweralkyl radical and $R_{37}$ is a loweralkyl residue.

The term "((heterocyclic)alkyl) (alkyl)amino" as used herein refers to $-NR_{71}R_{72}$ wherein $R_{71}$ is a heterocyclic alkyl group and $R_{72}$ is a loweralkyl group.

The term "(heterocyclic alkyl)amino" as used herein refers to $-NHR_{73}$ wherein $R_{73}$ is a heterocyclic alkyl group.

The term "(heterocyclic) (alkyl)amino" as used herein refers to $-NR_{74}R_{75}$ wherein $R_{74}$ is a heterocyclic group and $R_{75}$ is a loweralkyl group.

The term "(alkylaminoalkyl) (alkyl)amino" as used herein refers to $-NR_{76}R_{77}$ wherein $R_{76}$ is an alkylaminoalkyl group and $R_{77}$ is a loweralkyl group.

The term "(dialkylaminoalkyl) (alkyl)amino" as used herein refers to $-NR_{78}R_{79}$ wherein $R_{78}$ is a dialkylaminoalkyl group and $R_{79}$ is a loweralkyl group.

The term "((alkoxy) (alkyl)aminoalkyl)(alkyl)amino" as used herein refers to $-NR_{80}R_{81}$ wherein $R_{80}$ is $-NR_{82}R_{83}$ appended to a loweralkyl radical wherein $R_{82}$ is an alkoxy group and $R_{83}$ is a loweralkyl group and $R_{81}$ is a loweralkyl group.

The term "((alkoxy)aminoalkyl) (alkyl)amino" as used herein refers to $-NR_{84}R_{85}$ wherein $R_{84}$ is $-NHR_{86}$ appended to a loweralkyl group and wherein $R_{86}$ is an alkoxy group and $R_{85}$ is a loweralkyl group.

The term "carboxyalkoxyalkyl" as used herein refers to a carboxy (-COOH) group appended to an alkoxy group which is in turn appended to a loweralkyl radical.

The term "(alkoxycarbonyl)alkoxyalkyl" as used herein refers to an alkoxycarbonyl group (-C(O)OR200 wherein R200 is loweralkyl) appended to an alkoxy group which in turn is appended to a loweralkyl radical.

The term "carboxyalkylamino" as used herein refers to a carboxy group appended to the alkyl portion of an alkylamino radical.

The term "alkoxycarbonylalkylamino" as used herein refers to an alkoxycarbonyl group appended to the alkyl portion of an alkylamino radical.

The term "(amino)carboxyalkyl" as used herein refers to a loweralkyl radical to which is appended an amino group and a carboxy group.

The term "(amino)carboxyalkylamino" as used herein refers to an alkylamino radical, the alkyl portion of which has appended an amino group and a carboxy group.

The term "((N-protected)amino)carboxyalkyl" as used herein refers to a loweralkyl radical to which is appended an N-protected amino group and a carboxy group.

The term "((N-protected)amino)carboxyalkylamino" as used herein refers to an amino radical to which is appended an ((N-protected)amino)carboxyalkyl group.

The term "(alkylamino)carboxyalkyl" as used herein refers to a loweralkyl radical to which is appended an alkylamino group and a carboxy group.

The term "(alkylamino)carboxyalkylamino" as used herein refers to an amino radical to which is appended an (alkylamino)carboxyalkyl group.

The term "((N-protected)alkylamino)carboxyalkyl" as used herein refers to a loweralkyl radical to which

is appended an (N-protected)alkylamino group and a carboxy group.

The term "((N-protected)alkylamino)carboxyalkylamino" as used herein refers to an amino radical to which is appended an ((N-protected)alkylamino)carboxyalkyl group.

The term "(dialkylamino)carboxyalkyl" as used herein refers to a loweralkyl radical to which is appended a dialkylamino group and a carboxy group.

The term "(dialkylamino)carboxyalkylamino" as used herein refers to an amino radical to which is appended an (dialkylamino)carboxyalkyl group.

The term "(amino)alkoxycarbonylalkyl" as used herein refers to a loweralkyl radical to which is appended an amino group and an alkoxycarbonyl group.

The term "(amino)alkoxycarbonylalkylamino" as used herein refers to an amino radical to which is appended an (amino)alkoxycarbonylalkyl group.

The term "(N-protected)amino)alkoxycarbonylalkyl" as used herein refers to a loweralkyl radical to which is appended an (N-protected)amino group and an alkoxycarbonyl group.

The term "(N-protected)amino)alkoxycarbonylalkylamino" as used herein refers to an amino radical to which is appended an (N-protected)amino)alkoxycarbonylalkyl group.

The term "(alkylamino)alkoxycarbonylalkyl" as used herein refers to a loweralkyl radical to which is appended an alkylamino group and an alkoxycarbonyl group.

The term "(alkylamino)alkoxycarbonylalkylamino" as used herein refers to an amino radical to which is appended an (alkylamino)alkoxycarbonylalkyl group.

The term "((N-protected)alkylamino)alkoxycarbonylalkyl" as used herein refers to a loweralkyl radical to which is appended an (N-protected)alkylamino group and an alkoxycarbonyl group.

The term "((N-protected)alkylamino)alkoxycarbonylalkylamino" as used herein refers to an amino radical to which is appended an ((N-protected)alkylamino)alkoxycarbonylalkyl group.

The term "(dialkylamino)alkoxycarbonylalkyl" as used herein refers to a loweralkyl radical to which is appended a dialkylamino group and an alkoxycarbonyl group.

The term "(dialkylamino)alkoxycarbonylalkylamino" as used herein refers to an amino radical to which is appended a (dialkylamino)alkoxycarbonylalkyl group.

The term "loweralkylcarbonyl" as used herein refers to $R_{87}C(O)$- wherein $R_{87}$ is a loweralkyl group, including, but not limited to acetyl, propionyl and the like.

The terms "alkoxy" and "thioalkoxy" as used herein refer to $R_{38}O$- and $R_{38}S$-, respectively, wherein $R_{38}$ is a loweralkyl group.

The term "alkenyloxy" as used herein refers to $R_{39}O$-, wherein $R_{39}$ is a straight or branched chain of 1 to 7 carbon atoms which contains at least one double bond including, but not limited to, allyl, propenyl and the like.

The term "hydroxyalkoxy" as used herein refers to -OH appended to an alkoxy radical.

The term "dihydroxyalkoxy" as used herein refers to an alkoxy radical which is disubstituted with -OH radicals.

The term "phenylalkoxy" as used herein refers to a phenyl group appended to an alkoxy radical, including, but not limited to benzyloxy and the like.

The term "(substituted phenyl)alkoxy" as used herein refers to a substituted phenyl group appended to an alkoxy radical, including, but not limited to 4-chlorobenzyloxy and the like.

The term "naphthylalkoxy" as used herein refers to a naphthyl group appended to an alkoxy radical.

The term "(substituted naphthyl)alkoxy" as used herein refers to a substituted naphthyl group appended to an alkoxy radical.

The term "polyalkoxy" as used herein refers to $R_{40}O$-, wherein $R_{40}$ is a straight or branched chain containing 1-5, $C_n$-O-$C_{n'}$ linkages wherein n and n′ are independently 1-3.

The term "halo" as used herein refers to Cl, Br, F or I substituents.

The term "halobenzyl" as used herein refers to a halo substituent appended to the phenyl ring of a benzyl radical.

The term "halophenyl" as used herein refers to a halo substituent appended to a phenyl radical.

The term "substituted phenyl" as used herein refers to a phenyl ring substituted with one, two or three substituents chosen from the group loweralkoxy, loweralkyl, amino, loweralkylamino, dialkylamino, hydroxy, halo, mercapto, nitro, thioalkoxy, carboxaldehyde, carboxy, carboalkoxy and carboxamide, including, but not limited to halophenyl, loweralkylphenyl, alkoxyphenyl and the like.

The term "substituted naphthyl" as used herein refers to a naphthyl ring substituted with one, two or three substituents chosen from the group loweralkoxy, loweralkyl, amino, loweralkylamino, dialkylamino, hydroxy, halo, mercapto, nitro, thioalkoxy, carboxaldehyde, carboxy, carboalkoxy and carboxamide, including, but not limited to halonaphthyl, alkoxynaphthyl and the like.

The term "heterocyclic group" or "heterocyclic" as used herein refers to any 3- or 4-membered ring containing a heteroatom selected from oxygen, nitrogen, and sulfur, or a 5- or 6-membered ring containing from one to three heteroatoms selected from the group consisting of nitrogen, oxygen, and sulfur; wherein the 5-membered ring has 0-2 double bonds and the 6-membered ring has 0-3 double bonds; wherein the nitrogen and sulfur heteroatoms may optionally be oxidized; wherein the nitrogen heteroatom may optionally be quaternized; and including any bicyclic group in which any of the above heterocyclic rings is fused to a benzene ring or another 5- or 6-membered heterocyclic ring independently as defined above. Heterocyclics in which nitrogen is the heteroatom are preferred. Fully saturated heterocyclics are also preferred. Preferred heterocyclics include: pyrryl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, N-methyl piperazinyl, azetidinyl, N-methyl azetidinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl, triazolyl and benzothienyl.

Heterocyclics can be unsubstituted or monosubstituted or disubstituted with substituents independently selected from hydroxy, oxo (=O), alkylimino ($R^*N=$ wherein $R^*$ is a loweralkyl group), amino, alkylamino, dialkylamino, alkoxy, polyalkoxy, loweralkyl or haloalkyl.

The most preferred heterocyclics include imidazolyl, pyridyl, piperazinyl, N-methyl piperazinyl, azetidinyl, N-methyl azetidinyl, thiazolyl, thienyl, triazolyl and the following:

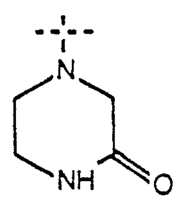 , 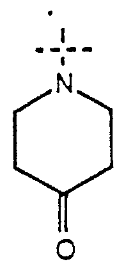 , 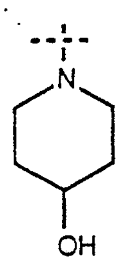 , 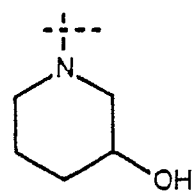 ,

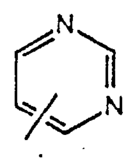 , 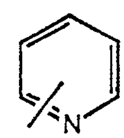 , 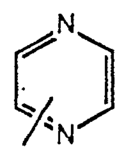 , 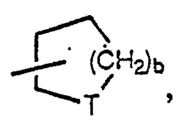 , 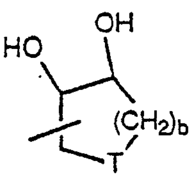

wherein b is 1 or 2 and Tis N, NH, O, S, provided that T is the point of connection only when T is N,

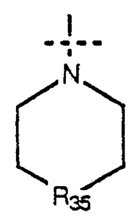

wherein $R_{35}$ is NH, N-loweralkyl, O, S, or $SO_2$, or

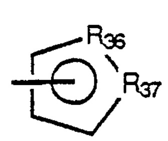 , 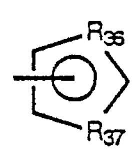 , 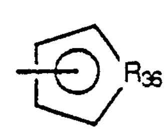

(i) (ii) (iii)

wherein the symbols (i), (ii) and (iii) represent 5-membered heterocycles containing one or more heteroatoms and containing 2 double bonds; wherein $R_{36}$ is N, O, or S and not the point of connection and $R_{37}$ is N when it is the point of connection or NH, O or S when it is not the point of connection.

The term "N-protecting group" or "N-protected" as used herein refers to those groups intended to protect the N-terminus of an amino acid or peptide or to protect an amino group against undesirable reactions during synthetic procedures or to prevent the attack of exopeptidases on the compounds or to increase the solubility of the compounds and includes but is not limited to sulfonyl, acyl, acetyl, pivaloyl, t-butyloxycarbonyl (Boc), carbonylbenzyloxy (Cbz), benzoyl or an L- or D-aminoacyl residue, which may itself be N-protected similarly.

The term "O-protecting group" as used herein refers to a substitutent which protects hydroxyl groups against undesirable reactions during synthetic procedures and includes but is not limited to substituted methyl ethers, for example methoxymethyl, benzyloxymethyl, 2-methoxyethoxymethyl, 2-(trimethylsilyl)-ethoxymethyl, benzyl and triphenylmethyl; tetrahydropyranyl ethers; substituted ethyl ethers, for example, 2,2,2-trichloroethyl and t-butyl; silyl ethers, for example, trimethylsilyl, t-butyldimethylsilyl and t-butyldiphenylsilyl; cyclic acetals and ketals, for example, methyl acetal, acetonide and benzylidene acetal; cyclic ortho esters, for example, methoxymethylene; cyclic carbonates; and cyclic boronates.

The terms "Ala", "His", "Leu", "Phe", "norLeu", "Met" and "Tyr" as used herein refer to alanine, histidine, leucine, phenylalanine, norleucine, methionine and tyrosine, respectively. In general, the amino acid abbreviations used herein follow the IUPAC-IUB Joint Commission on Biochemical Nomenclature for amino acids and peptides (Eur. J. Biochem. 1984, 158, 9-31).

The compounds of the invention can be made as shown in Schemes 1-3. Intermediates (1) and (7) can be prepared according to methods described in copending U.S. patent application, USSN 191,714, filed May 9, 1988, which is hereby incorporated by reference. Other intermediates (1) can be prepared as disclosed in PCT Patent Application No. WO88/05050, published July 14, 1988. In the schemes A, $R_1$, $R_2$, $R_3$, $R_4$, $R_5$, $R_6$, $R_7$, $R_{12}$, W, U and Z are as defined above.

In particular, the process shown in Scheme 1 discloses the coupling of an N-functionalized amino acid (1) with a diamino diol (2) to provide (3). The coupling reaction is accomplished using the diimide method which employs N-ethyl-N′-(3-dimethylaminopropyl) carbodiimide, 1-hydroxybenzotriazole, and N-methylmorpholine. When $R_{12}$ is hydrogen, the secondary amino group of (2) can be protected with an N-protecting group, which is removed after the coupling reaction.

Alternatively, Scheme 2 discloses the coupling of N-protected amino acid (4) with diamino diol (2). Deprotection provides amine (5) which is coupled with carboxylic acid (6) to provide (3).

The process shown in Scheme 3 discloses methods for preparing diamino diols (2). Epoxide (7), wherein $P_1$ is an N-protecting group and $P_2$ is an O-protecting group, can be treated with sodium azide, followed by reduction, to give the primary amine (9). Acylation of (9) provides amide (10) wherein R′ is loweralkyl, monohaloloweralkyl or polyhaloloweralkyl. Reduction of (10) with $BH_3$/THF, followed by removal of $P_1$ and $P_2$, provides (2) wherein $R_{12}$ is hydrogen and $R_7$ is loweralkyl or haloalkyl.

Alternatively, epoxide (7) can be reacted with an amine (11) to give, after removal of $P_1$ and $P_2$, (2) directly. A final alternative involves reaction of epoxide (7) with a primary amine (13) to provide (14), which is then acylated to give (15) wherein R″ is loweralkyl, monohaloloweralkyl or polyhaloloweralkyl. Amide (15) can then be reduced and $P_1$ and $P_2$ removed to provide (2).

In the schemes -NR7R12 can be replaced by a heterocyclic group to provide the compounds of the invention wherein Z is heterocyclic.

Other methods known in the art can be used to accomplish the amide bond forming coupling reactions. In particular, activated derivatives of the carboxylic acids can be used in the coupling reactions. Such activated derivatives include acid halides such as acid chlorides, and activated esters including, but not limited to, formic and acetic acid derived anhydrides, anhydrides derived from alkoxycarbonyl halides such as isobutyloxycarbonylchloride and the like, N-hydroxysuccinimide derived esters, N-hydroxyphthalimide derived esters, N-hydroxybenzotriazole derived esters, N-hydroxy-5-norbornene-2,3-dicarboxamide derived esters, 2,4,5-trichlorophenol derived esters and the like.

Scheme 1

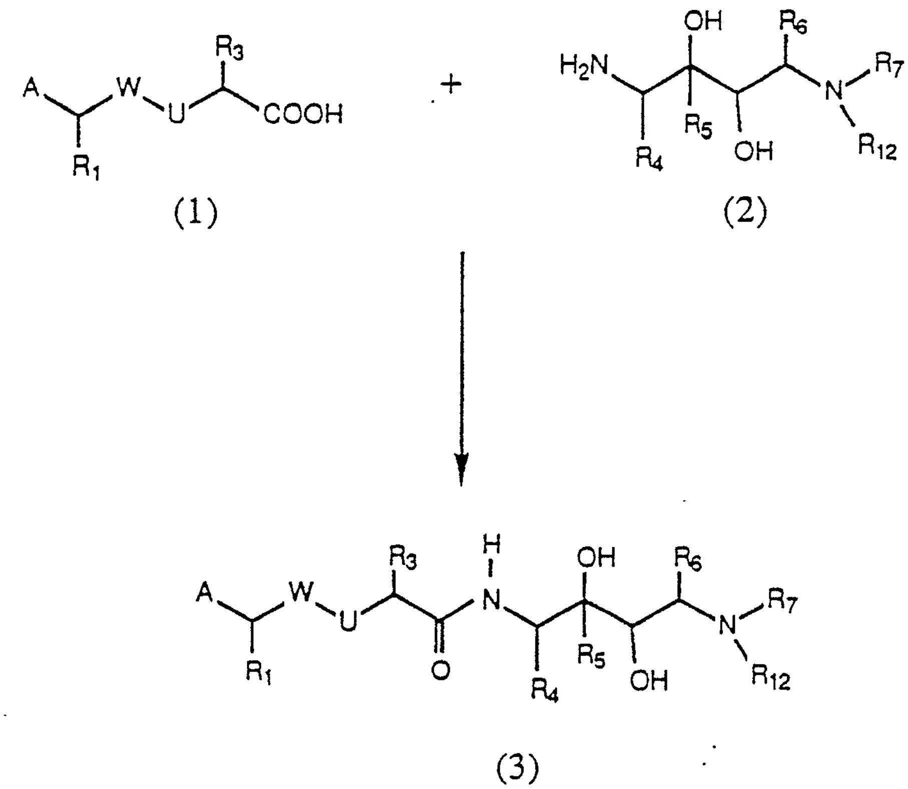

Scheme 2

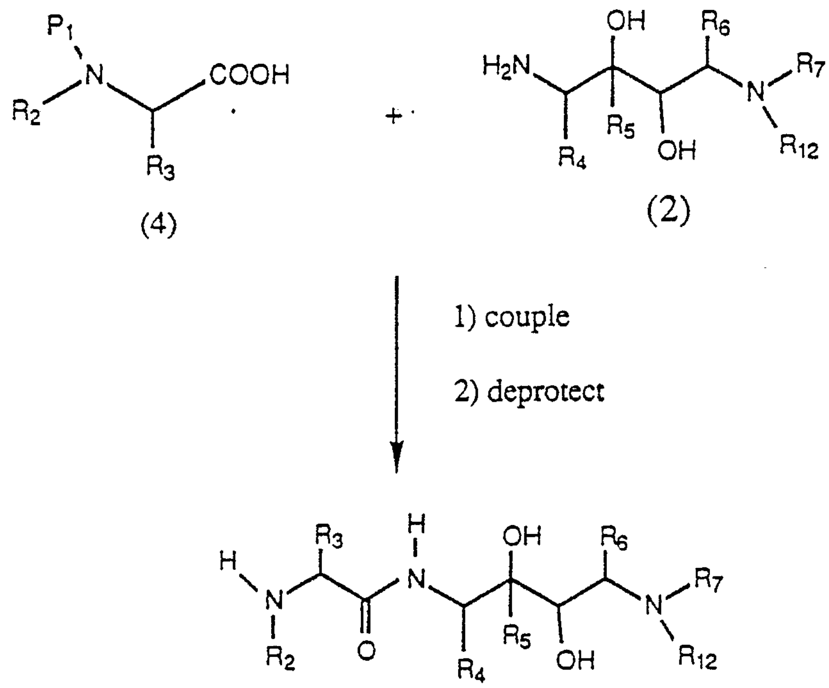


(4) (2)

(5)

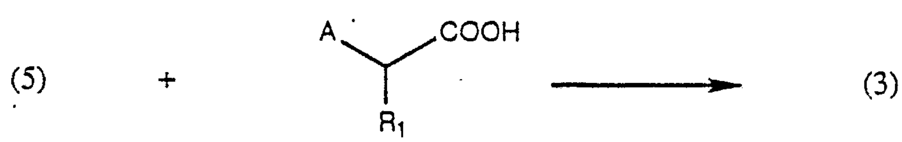

(5) + (6) → (3)

(6)

Scheme 3

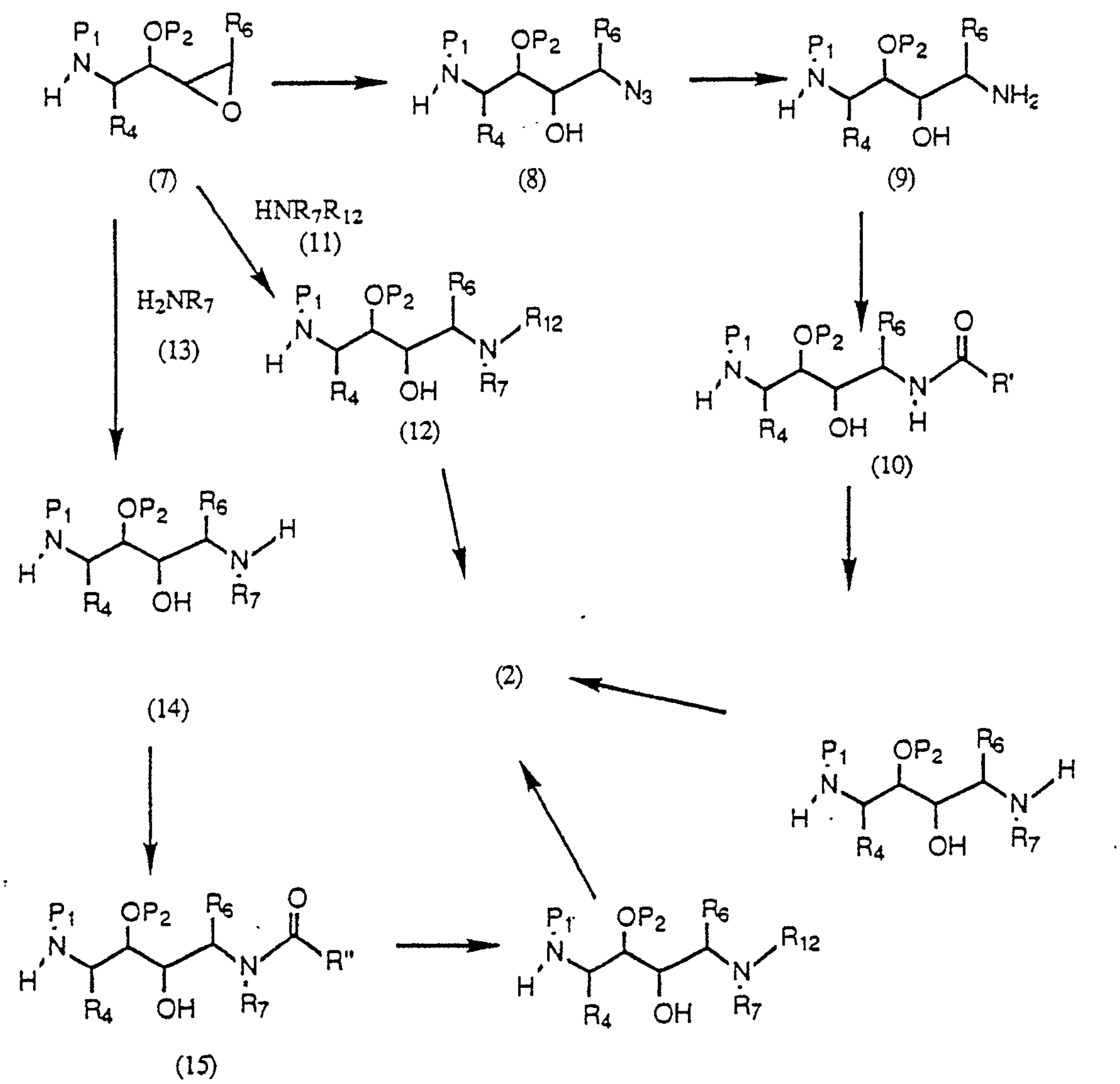


The following Examples will serve to further illustrate preparation of the novel compounds of the invention.

## Example 1

4(S)-t-Butyloxycarbonylamino-5-cyclohexyl-3(R,S)-hydroxy-1-pentene

To a stirred -78° C solution of Boc-cyclohexylalanine methyl ester (10.2 g. 35.8 mmol) in dry toluene (60 ml) was added diisobutylaluminum hydride (34 ml of a 1.5 M solution in toluene). After 30 min., vinyl magnesium bromide (108 ml of 1 M solution in tetrahydrofuran (THF)) was added. After stirring for 15 hours at 0° C, the mixture was carefully quenched with methanol, treated with Rochelle salts (22 ml of saturated aqueous solution in 140 ml H2O), and filtered. After extracting the solids 5 times with ethyl acetate, the extracts and filtrate were combined and the organic phase was washed with brine, dried, filtered and evaporated to an oil (10.2g). Chromatography on silica gel eluting with hexane/ethyl acetate mixtures provided 6.1 g (60%) of the desired product.

Analysis calculated for C16H29NO3 1/4H2O: C, 66.8; H, 10.3; N, 4.9. Found: C,66.9; H, 10.2; N, 4.7.

### Example 2

### 4(S)-Cyclohexylmethyl-5(R,S)-vinyl-2-oxazolidinone

The resultant product of Example 1 (2.80 g, 9.88 mmol) in dry dimethylformamide (DMF) (50 ml) was added to a stirred suspension of NaH (593 mg of a 60% dispersion in oil, 14.8 mmol, hexane washed) in dry DMF (50 ml). After 3 hours, the mixture was quenched (750 ml water + 100 ml brine) and extracted with ether (5 x 100 ml). The combined organic phase was washed with brine (3 x 50 ml), dried (MgSO4), filtered and evaporated to an oil (2.23 g). The NMR spectrum of the crude product revealed an 82:18 mixture of 5 S:5 R diastereomers. Silica gel chromatography gave 80% recovery of pure diastereomers. 5 S:

Analysis calculated for C12H19NO2: C, 68.9; H, 9.1; N, 6.7. Found: C, 68.4; H, 9.2; N, 6.5. Mass spectrum: (M+1)+ = 210. 5 R: Mass spectrum: (M+1)+ = 210.

### Example 3

### (3S,4S)-3-Hydroxy-4-amino-5-cyclohexyl-1-pentene

To the resultant 5S-diasteriomer from Example 2 (2.06 g, 9.84 mmol) in dioxane (180 mL) and water (120 mL) was added barium hydroxide octahydrate (6.24 g, 19.8 mmol). The mixture was refluxed for 18 hours, cooled, filtered, concentrated, taken up in water and extracted with ethyl acetate which was dried over Na2SO4 and evaporated to afford 1.64 g (91%) of the desired product, m.p. 59-61 °C.

Analysis calculated for C11H21NO: C, 72.08; H, 11.55; N, 7.64. Found: C, 71.67; H, 11.68; N, 7.36.

### Example 4

### (3S,4S)-3-Hydroxy-4-tert-butoxycarbonylamino-5-cyclohexyl-1-pentene

To the resultant compound from Example 3 (1.62 g, 8.84 mmol) in methylene chloride (20 mL) was added di-tert-butyldicarbonate (1.93 g, 8.84 mmol). The mixture was stirred for 14 hours, diluted with ethyl acetate, washed sequentially with 0.5 M H3PO4, saturated NaHCO3 solution and brine, then dried over Na2SO4 and evaporated to afford 2.51 g (100%) of the desired compound.

### Example 5

### (3S,4S)-3-Methoxymethoxy-4-tert-butyloxycarbonylamino-5-cyclohexyl-1-pentene

To the resultant compound from Example 4 (1.60 g, 56.5 mmol) in methylene chloride (30 mL) was added diisopropylethylamine (9.8 mL, 56 mmol) and chloromethylmethyl ether (4.3 mL, 56 mmol). After 12 h, the mixture was concentrated, taken up in ethyl acetate, washed sequentially with 0.5 M H3PO4,

saturated $NaHCO_3$ solution and brine, then dried over $Na_2SO_4$ and evaporated to afford 1.82 g (98%) of the desired product as an oil.

Analysis calculated for $C_{18}H_{33}NO_4$: C, 66.02; H, 10.16; N, 4.28. Found: C, 65.96; H, 10.36; N, 4.26.

## Example 6

### (2RS,3R,4S)-3-Methoxymethoxy-4-tert-butyloxycarbonylamino-5-cyclohexyl-1,2-oxopentane

The resultant compound from Example 5 (1.82 g.) and 3-chloroperbenzoic acid (3.60g) in methylene chloride (30 mL) were stirred for 40 h. The solvent was evaporated, and the residue dissolved in ethyl acetate and washed sequentially with cold 10% $Na_2SO_4$ solution, saturated $NaHCO_3$ solution, then brine. After drying over $Na_2SO_4$ the solvent was evaporated to provide 1.81 g (95%) of the desired product as an oil.

Analysis calculated for $C_{18}H_{33}NO_5$: C, 62.95; H, 9.68; N, 4.08. Found: C, 63.08; H, 10.16; N, 4.07.

## Example 7

### (2S,3R,4S)-1-Azido-2-hydroxy-3-methoxymethoxy-4-tert-butyloxycarbonylamino-5-cyclohexylpentane

The resultant compound from Example 6 (1.68 g., 4.89 mmol), sodium azide (0.82 g, 12.6 mmol) and $NH_4Cl$ (0.51 g, 9.53 mmol) in methanol (40 mL) were heated at reflux for 36 h. The mixture was concentrated, taken up in ethyl acetate, washed with water and brine then dried over $Na_2SO_4$ and evaporated. Chromatography on silica gel with ether-hexane mixtures afforded 0.73 g (38%) of the desired 2-S isomer followed by 0.36 g (19%) of the 2-R isomer.

2-S isomer: 1H-NMR ($CDCl_3$, TMS) 4.66 (d,1H), 4.08 (m,1H), 3.39 (s,3H), 1.45 (s,9H) 2-R isomer: 1H-NMR ($CDCl_3$, TMS) 4.78 (d,1H), 4.66 (d,1H), 3.45, (s,3H).

## Example 8

### (2S, 3R, 4S)-4-t-Butyloxycarbonylamino-5-cyclohexyl-2-hydroxy-1-N-methoxyamino-3-methoxymethoxylpentane

The resultant compound from Example 6 (4.01g, 11.7 mmol) was added to a premixed solution of methoxylamine hydrochloride salt (2.93 g, 35.0 mmol) and sodium carbonate (3.71 g, 35 mmol) in 45 mL of 10% $H_2O$:isopropanol in a resealable tube fit with a magnetic stirring bar. The resulting solution was flushed with $N_2$, tightly sealed, and heated at 115°C for 18 h. After cooling to room temperature, the reaction solution was filtered and the filtrate concentrated under vacuum. MPLC (200 g silica gel; 20 ml fractions; 30% ethyl acetate/chloroform) provided, in order of elution: the (2S, 3R, 4S)-isomer (1.51 g, 3.87 mmol, 33%), mixed fractions (0.35 g, 0.90 mmol, 8%), and the (2R, 3R, 4S)-isomer (0.75 g, 1.92 mmol, 16%). Recrystalization of the (2S, 3R, 4S)-isomer from ether/hexanes provided an analytical sample; m.p. 68.5-69.5°C. Analysis calculated for $C_{19}H_{38}N_2O_6 \cdot 0.25\ H_2O$: C, 57.77; H, 9.82; N, 7.09. Found: C, 58.07; H, 9.58; N, 6.74.

## Example 9

(2S,3R,4S)-1-Dimethylamino-2-hydroxy-3-methoxymethoxy-4-tertbutyloxycarbonylamino-5-cyclohexylpentane

The resultant compound from example 7 (167.0 mg, 0.432 mmol), 10% palladium on carbon (83 mg) and 37% aqueous formaldehyde solution (1 mL) in methanol (5 mL) were stirred under a hydrogen atmosphere for 24 h. After filtration and solvent evaporation the residue was chromatographed on silica gel with methanol-chloroform mixtures to provide 51.7 mg (31%) of the desired product as an oil.

1H-NMR (CDCl3, TMS) 4.95 (d, 1H), 4.68 (s, 2H), 3.67 (m, 1H), 3.40 (s, 3H), 2.34 (s, 6H), 1.45 (s, 9H).

Example 10

(2S,3R,4S)-1-Amino-2-hydroxy-3-methoxymethoxy-4-tert-butyloxycarbonylamino-5-cyclohexylpentane

The resultant compound from Example 7 (508.3 mg, 1.32 mmol) in methanol (7 mL) was treated with triethylamine (0.55 mL, 3.9 mmol) and propane 1,3-dithiol (0.40 mL, 4.0 mmol). After 36 h the mixture was poured into 0.5 M H3PO4, washed with ether, made basic with K2CO3 and extracted into ethyl acetate which was dried over Na2SO4 and evaporated to afford 397.6 mg (84%) of the desired product as a white solid.

Analysis calculated for C18H36N2O5: C, 59.97; H, 10.07; N, 7.7 Found: C, 59.91; H, 10.14; N, 7.68.

Example 11

(2S,3R,4S)-1-Isopropylcarbonylamino-2-hydroxy-3-methoxymethoxy-4-tert-butyloxycarbonylamino-5-cyclohexylpentane

The resultant compound from example 10 (193.2 mg, 0.536 mmol) in methylene chloride (3 mL) at 0° C was treated with triethylamine (115 ul, 0. 21 mmol) and isobutyric anhydride (100 ul, 0.603 mmol). After 30 min. at 0° C and 45 min. at ambient temperature the mixture was poured into ethyl acetate and washed sequentially with 0.5 M H3PO4, satured NaHCO3 solution and brine, then dried over Na2SO4 and evaporated. Chromatography of the residue on silica gel with hexane-ethyl acetate mixtures afforded 206.3 mg (89%) of the desired product as an oil.

1H-NMR (CDCl3, TMS) 4.69 (d, 1H), 4.62 (d, 1H), 4.04 (m, 1H), 3.34 (ddd, 1H), 3.46 (s, 3H), 2.38 (m, 1H), 1.44 (s, 9H), 1.17 (d, 3H), 1.14 (d, 3H)

Example 12

(2S,3R,4S)-1-(Benzyloxycarbonyl)isobutylamino-2-hydroxy-3-methoxymethoxy-4-tert-butyloxycarbonylamino-5-cyclohexylpentane

The resultant compound from Example 11 (196.0 mg, 0.455 mmol) in tetrahydrofuran (5 mL,THF) at -23° C was treated with BH3 in THF (1.4 mL, 1.0 M). The mixture was stirred at 0° C and then quenched with methanol (10 mL). After solvent evaporation the residue was dissolved in methylene chloride (5 mL), cooled to 0° C, and treated with triethylamine (100 ul, 0.71 mmol) and benzyl chloroformate (65 ul, 0.46 mmol). After 45 min. at 0° C and 1 h at room temperature the product was isolated and chromatographed as described in Example 11 to provide 83.1 mg (33%) of the desired product as an oil.

Analysis calculated for C30H50N2O7.0.5H2O: C. 64.37; H, 9.18; N, 5.00. Found: C, 64.18; H, 9.30; N,

5.11.

## Example 13

### (2S,3R,4S)-1-(Trifluoroethyl(methylamino-2-hydroxy-3-methoxymethoxy-4-tert-butyloxycarbonylamino-5-cyclohexylpentane

The resultant epoxide from Example 6 was stirred in methylamine in a sealed tube for 72 h. After removing the solvent with ethanol chasers the material was acylated with trifluoroacetic anhydride according to the procedure of Example 11. After isomer separation by chromatography on silica gel the desired 2S isomer was reduced according to the procedure of Example 12 to provide the desired product.

## Example 14

### Boc-Phe-His Amide of (2S,3R,4S)-1-Azido-2,3-dihydroxy-4-amino-5-cyclohexylpentane

The resultant compound from Example 6 (0.20 mmol) was stirred for 1 h in 4 M HCl in ethanol and the solvent was evaporated with ether chasers. This residue, Boc-Phe-His-OH (0.21 mmol), and 1-hydroxybenztriazole (0.54 mmol) were dissolved in DMF (2 mL), treated with N-methylmorpholine (0.44 mmol), cooled to -23° C, and treated with 1-(3-dimethylaminopropyl)-3-ethylcarbodiimide hydrochloride (0.28 mmol). After 2 h the cooling bath is removed and after 16 h the mixture is poured into saturated NaHCO3 solution and extracted into ethyl acetate which was washed with water and then brine, dried over Na2SO4 and evaporated. Chromatography of the residue on silica gel with methanol-chloroform mixtures afforded the desired product, m.p. 158-163° C.

Exact Mass calculated for C31H47N8O6 (M+H) : 627.3618. Found: 627.3605.

## Example 15

### Isobutyryl-Phe-Leu Amide of (2S,3R,4S)-1-Azido-2,3-dihydroxy-4-amino-5-cyclohexylpentane

Using the procedure of Example 14 but using isobutyryl-Phe-Leu-OH in place of Boc-Phe-His-OH provided the desired produce, m.p. 193-194° C.

Exact Mass calculated for C30H49N6O5 (M+H) : 573.3764. Found: 573.3757.

## Example 16

### Boc-Phe-His (4-Amino)Amide of (2S,3R,4S)-1,4-Diamino-2,3-dihydroxy-5-cyclohexylpentane Acetic Acid Salt

The resultant compound from Example 14 (35.1 mg, 0.0478 mmol) and 10% palladium on carbon (21 mg) in acetic acid (3 mL) were stirred under a hydrogen atmosphere for 90 min. and then filtered and evaporated to provide 27.6 mg (96%) of the desired produced as a white solid, m.p. 145-148° C.

Analysis calculated for C33H52N6O8: C, 57.98; H, 7.93; N, 12.72. Found: C, 59.92; H, 7.89; N, 12.75.

## Example 17

### Isobutyryl-Phe-Leu (4-Amino)Amide of (2S,3R,4S)-1,4-Diamino-2,3-dihydroxy-5-cyclohexylpentane

Using the procedure of Example 16 with the resultant compound of Example 15 and dissolving the resultant compound in ethyl acetate and washing with sodium carbonate solution and then drying the organic solution

## Example 20

### Boc-Phe-His Amide of (2S,3R,4S)-1-(Benzyloxycarbonyl)isobutylamino-2,3-dihydroxy-4-amino-5-cyclohexylpentane

Using the procedure of Example 14 with the resultant compound from Example 12 provided the desired product, m.p. 100-105 °C.

Analysis calculated for $C_{43}H_{62}N_6O_8 \cdot 0.25\ H_2O$: C, 64.92; H, 7.92; N, 10.56. Found: C, 64.78; H, 7.82; N, 10.36.

## Example 21

### Boc-Phe-His (4-Amino)Amide of (2S,3R,4S)-1-Isobutylamino-2,3-dihydroxy-4-amino-5-cyclohexylpentane Acetic Acid Salt

Using the procedure of Example 16 with the resultant compound from Example 20 provided the desired product, m.p. 110-113 °C.

Analysis calculated for $C_{37}H_{60}N_6O_8$: C, 61.99; H, 8.44; N, 11.72. Found: C, 61.62; H, 8.24; N, 11.78.

## Example 22

### Boc-Phe-His Amide of (2S,3R,4S)-1-Isobutyl methylamino-2,3-dihydroxy-4-amino-5-cyclohexylpentane

Using the procedure of Example 9 with the resultant compound from Example 20 provided the desired product, m.p. 103-107 °C.

Exact mass calculated for $C_{36}H_{59}N_6O_6$(M+H); 671.4496. Found: 671.4496.

## Example 23

Boc-Phe-His Amide of (2S,3R,4S)-1-Trifluoroethylmethylamino-2,3-dihydroxy-4-amino-5-cyclohexylpentane

Using the procedure of Example 14 with the resultant compound from Example 13 provided the desired product, m.p. 113-115 °C.

Analysis calculated for C34H51N6O6F3. H2O; C, 57.13; H, 7.47; N, 11.76. Found: C, 57.43; H, 7.24; N, 11.40.

Example 24

Boc-Phe-His-(4-Amino)Amide of (2S,3R,4S) 1-Methoxylamino-2,3-dihydroxy-4-amino-5-cyclohexylpentane

Using the procedure of Example 14 with the resultant compound from Example 8 provided the desired product and the bis-Boc-Phe-His (1-methoxylamino,4-amino)amide. The mixture was treated with excess K2CO3 in refluxing absolute methanol for 1h. cooled, and filtered. The filtrate was concentrated in vacuo and purified by silica gel chromatography as previously described to provide the desired product, m.p. softening at 117 °C, melts 119-122 °C.

Example 25

Boc-His Amide of 1-Dimethylamino-2,3-dihydroxy-4-amino-5-cyclohexylpentane

Using the procedure of Example 14 with the resultant compound from Example 9 and replacing Boc-Phe-His-OH with Boc-His-OH provided the desired product, m.p. 95-99 °C.

Mass Spectrum: (M + H) = 482.

Example 26

2(R,S)-(4-Morpholinylcarbonylmethyl)-3-phenyl-propionic Acid

Ethyl alpha-carboxymethylcinnamate was prepared as reported (Cohen, S.G. and Milovanovic, A. Biochemistry, 1968, 3495) and hydrogenated (1 atmosphere H2) with 10% Pd/C in methanol for 5 h. The resulting dihydrocinnamate was coupled to morpholine using the procedure of Example 20. Ester hydrolysis with LiOH in dioxane/water provided the desired compound. Mass spectrum: (M + H) + = 278.

Analysis calculated for C15H19NO4.1/8 H2O: C, 64.4; H, 6.9; N, 5.0. Found: C, 64.4; H, 6.8; N, 4.9.

Example 27

(2-R)-4-Morpholinylcarbonyl methyl-3-phenylpropionyl-His Amide of (2S,3R,4S)-1-Dimethylamino-2,3-dihydroxy-4-amino-5-cyclohexylpentane

Using the procedure of Example 14 with the resultant compound from Example 25 and using the

resultant compound from Example 26 in place of Boc-Phe-His-OH provided the desired product, m.p. 90-95 °C.

Exact Mass calculated for C34H53N6O6 (M + H) : 641.4026. Found: 641.4008.

## Example 28

### (3S,4S)-3-Methoxyethoxymethoxy-4-tert-butyloxycarbonylamino-5-cyclohexyl-1-pentene.

To the resultant compound from Example 4 (2.51 g, 8.84 mmol) in methylene chloride (20 ml) was added diisopropyl ethylamine (4.60 ml, 26.4 mmol) and methoxyethoxy chloromethane (3.00 ml, 26.3 mmol). After stirring at room temperature for 24 h the mixture was concentrated, diluted with ethyl acetate, washed with 0.5 M $H_3PO_4$, saturated $NaHCO_3$ solution, then brine, dried over $Na_2SO_4$, and evaporated. Chromatography on silica gel with ethyl acetate/hexane mixtures afforded 2.63 g (80%) of the desired product as an oil. EI-MS: $M^+$ = 371.

Anal. ($C_{20}H_{37}NO_5$)

Calcd: C, 64.66; H, 10.04; N, 3.77.

Found: C, 64.94; H, 9.97; N, 3.74.

## Example 29

### (2RS,3R,4S)-3-Methoxyethoxymethoxy-4-tert-butyloxycarbonylamino-5-cyclohexyl-1,2-oxopentane.

To the resultant compound from Example 5 (5.41 g, 14.56 mmol) in methylene chloride (50 ml) was added 3-chloroperbenzoic acid (6.28 g). After stirring at room temperature for 60 h the mixture was concentrated, diluted with ethyl acetate, washed with cold 1:1 15% aqueous $Na_2SO_3$ solution/saturated $NaHCO_3$ solution (2 x 200 ml), saturated $NaHCO_3$ solution (3 x 100 ml) then brine (1 x 100 ml), dried over $Na_2SO_4$, and evaporated to afford 4.57 g (81%) product as an oil. EI-MS: $M^+$ = 387.

Anal. ($C_{20}H_{37}NO_6$)

Calcd: C, 61.99; H, 9.62; N, 3.61.

Found: C, 61.84; H, 9.56; N, 3.55.

## Example 30

### (2S,3R,4S)-4-t-Butyloxycarbonylamino-5-cyclohexyl-2-hydroxy-1-(N-methoxy-N-methylamino)-3-(methoxyethoxymethoxy)pentane.

The resultant compound from Example 29 (454.7 mg, 1.17 mmol) in dioxane (4 mL) was treated with N-methyl-O-methylhroxylamine hydrochloride (1.14 g, 11.7 mmol) in water (4 mL). Solid $NaHCO_3$ (1.00 g, 11.9 mmol) was added, the reaction was sealed and heated at 90 °C for 60 h. The reaction was cooled, poured into saturated $NaHCO_3$ solution and extracted into ethyl acetate which was dried over $Na_2SO_4$ and evaporated. Chromatography of the residue on silica gel (60 g) with 60% ethyl acetate in hexane afforded 306.0 mg (58%) of the 2S-isomer (followed by the 2R-isomer). TLC (5% methanol/95% chloroform) $R_f$ = 0.53; $^1H$ NMR ($CDCl_3$) $\delta$ 4.89 (1H,d), 4.77 (2H,s), 4.02-4.12 (1H,m), 3.02 (3H,s), 3.39 (3H,s), 2.91 (1H,dd), 2.63 (3H,s), 1.45 (9H,s).

## Example 31

(2S,3R,4S)-4-Amino-5-cyclohexyl-2,3-dihdroxy-1-(N-methoxy-N-methylamino)pentane.

The resultant compund from Example 30 (1.00 mmol) was stirred for 1 h in 4M HCl/ethanol. The mixture was evaporated and the residue was disolved in water which was made basic with solid $K_2CO_3$ and was then saturated with NaCl. The mixture was extracted with chloroform which was dried over $Na_2SO_4$ and evaporated to afford the desired product.

## Example 32

(2S,3R,4S)-4-Amino-5-cyclohexyl-2,3-dihydroxy-1-(N-methyl-N-trifluoroethylamino)pentane.

The resultant compound from Example 13 was treated according to the procedure for Example 31 to provide the desired product.

## Example 33

H-His Amide of (2S,3R,4S)-4-Amino-5-cyclohexyl-2,3-dihydroxy-1-(N-methoxy-N-methylamino)pentane.

To the resultant compound from Example 31 (1.00 mmol), Boc-His-OH (1.10 mmol) and 1-hydroxybenzotriazole (2.70 mmol) in DMF (5 mL) was added N-methylmorpholine (1.20 mmol). The mixture was cooled to -23° C and treated with 1-(3-dimethylaminopropyl)-3-ethylcarbodiimide hydrochloride (1.43 mmol). After 2 h at -23° C and 12-16 h at room temperature the mixture was poured into saturated $NaHCO_3$ solution and extracted with ethyl acetate which was washed with water and brine, then dried over $Na_2SO_4$ and evaporated. Chromatography of the residue on silica gel with methanol/chloroform mixtures afforded the intermediate Boc-protected compound. This material was dissolved in methylene chloride (5 mL), cooled to 0° C, treated with trifluoroacetic acid (5 mL), stirred at 0° C for 3 h, and evaporated. The residue was dissolved in water, made basic with solid $K_2CO_3$, saturated with NaCl and extracted with chloroform which was dried over $Na_2SO_4$ and evaporated to afford the desired product.

## Example 34

H-Nle Amide of (2S,3R,4S)-Amino-5-cyclohexyl-2,3-dihydroxy-1-(N-methoxy-N-methylamino)pentane.

Boc-Nor-Leucine (Boc-Nle-OH, 1.00 mmol) in methylene chloride (10 mL) at -10° C was treated with N-methylmorpholine (1.10 mmol) and then with isobutylchloroformate (1.00 mmol). After 3 min. the resultant amine from Example 31 (1.00 mmol) was added and the reaction was stirred at -10° C for 10 min and at room temperature for 2 h. The mixture was evaporated, taken up in ethyl acetate, washed with water, saturated $NaHCO_3$ solution, and brine, then dried and evaporated. Chromatography of the residue on silica gel followed by N-deprotection according to the procedure of Example 33 provided the desired product.

Example 35

Boc-L-(1-Pyrazolyl)alanine.

Pyrazole (700 mg, 10.3 mmol) and N-(tert-Butyloxycarbonyl)-L-serine-β-lactone (1.707 g, 9.117 mmol, Arnold et al., J. Am. Chem. Soc. 1985, 107, 7105) in $CH_3CN$ (75 ml) were heated at 52° C for 72 h. The solvent was evaporated and the residue was dissolved in hot methanol (8 ml) and then water (24 ml) was added with heating until the mixture became turbid. The mixture was cooled to room temperature with rapid stirring, and after stirring overnight 745 mg (32%) of the desired product was collected as a white solid. $^1$H NMR ($CDCl_3$) δ 7.65 (d,1H), 7.41 (d,1H), 6.30 (dd,1H), 5.48 (br,1H), 4.82 (dd,1H), 4.67 (dd,1H), 4.48 (m,1H), 1.47 (s,9H).

Example 36

H-L-(1-Pyrazolyl)Ala Amide of (2S,3R,4S)-4-Amino-5-cyclohexyl-2,3-dihydroxy-1-(N-methyl-N-trifluoroethylamino)-pentane.

Using the procedure of Example 33 with the resultant compound from Example 32 and replacing Boc-His-OH with the resultant compound from Example 35 gave the desired product.

Example 37

3-Benzyloxycarbonylamino-3-methylbutanoic Acid.

A solution of 2,2-dimethyl-3-carbomethyoxypropionic acid [LeMaul, Bull. Soc. Chim. Fr., 828 (1965), 20 g, 0.125 mol], diphenylphosphorylazide (34.3 g, 0.125 mol) and triethylamine was heated in toluene (150 ml) at 100°C for 2 h. After cooling to 5° C, the toluene solution was washed successively with 0.5 M HCl, aqueous $NaHCO_3$ and brine. Evaporation of the dried solution gave a residue which was chromatographed on silica gel eluting with 60/40 hexane-ether. There was obtained 13 g of methyl 3-isocyanato-3-methylbutanoate as a mobile liquid. A solution of this material in toluene (20 ml) was treated with benzyl alcohol (13 ml) and the resulting mixture heated at reflux for 40 h. Evaporation of the toluene left a residue which was dissolved in methanol (125 ml) and then treated with a solution of NaOH (6.6 g, 0.165 mmol) in 22 ml of water. After 5 h, the reaction mixture was partially evaporated, washed with ether and acidified with 6N HCl. Extraction with methylene chloride and evaporation gave 21 g of the desired product. NMR (300 MHz, $CDC1_3$): δ 1.42 (s,6H), 2.78 (s,2H), 5.08 (s,2H).

Example 38

Cbz-[(β,β-di-Me)-β-Ala]-Phe-$OCH_3$.

A 4.0 g sample of 3-benzyloxycarbonylamino-3-methylbutanoic acid was coupled to phenylalanine methyl ester hydrochloride (3.43 g) using the mixed anhydride procedure described in Example 34. Purification of the crude product by flash chromatography eluting with 65/35 ether/hexane gave an 86%

yield of product. NMR (300 MHz, $CDCl_3$): δ 1.32 (s,3H), 1.34 (s,3H), 2.46 (d,1H), 2.63 (d,1H), 2.98 (dd,1H), 3.09 (dd,1H), 3.70 (s,3H), 4.86 (dd,1H), 4.97 (d,1H), 5.2 (d,1H), 5.3 (s,1H), 6.13 (d,1H).

## Example 39

### Cbz[β,β-di-Me)-β-Ala]-Phe-OH.

To a 0° C solution of Cbz-[(β,β-di-Me)-β-Ala]-Phe-OMe (1.5 g, 3.63 mmol) in dioxane (15 ml) was added a solution of lithium hydroxide (0.174 g, 4.15 mmol) in water 7.5 ml). After stirring for 1 h at 0-5° C, the reaction mixture was diluted with cold water and extracted two times with ether. The aqueous portion was acidified with 6N HCl and extracted with ether. The organic extract was washed with brine and evaporated to give an 87% yield of product as a viscous liquid.

## Example 40

### Cbz-[(β,β-di-Me)-β-Ala]-(Me)Tyr-$OCH_3$.

Using the procedure of Example 38 and replacing phenylalanine methyl ester hydrochloride with O-methyltyrosine methyl ester hydrochloride provided the desired material.

## Example 41

### Cbz-[(β-β-di-Me)-β-Ala]-(Me)Tyr-OH.

Using the procedure of Example 39 with the resultant compound from Example 40 gave the desired material.

## Example 42

### 2(S)-[[(4-Morpholinyl)carbonyl]oxy]-3-phenylpropionic Acid Methyl Ester.

To L-phenyllactic acid methyl ester (3.2 g) was added 150 ml of 12.5% phosgene in toluene and 25 drops of dimethylformamide. After stirring for 16 h at room temperature, the solvent was evaporated and the residue chased several times with benzene. The resulting product was dissolved in methylene chloride (50 ml), cooled to 0° C and treated by dropwise addition with 3.86 g (0.044 mmol) of morpholine. The reaction mixture was stirred for 2 h at 0-5° C and then distributed between 0.5 N HCl and methylene chloride. The organic phase was washed with aqueous $NaHCO_3$ and brine and evaporated to a residue. Flash chromatography on silica gel eluting with 2:1 ether/hexane gave a 65% yield of product. NMR (300 MHz): δ 3.08 (dd,1H), 3.20 (dd,1H), 3.8 (s,3H), 5.19 (dd,1H).

## Example 43

2(S)-[(4-Morpholinyl)carbonyl]oxy-3-phenylpropionic Acid.

Using the hydrolysis procedure of Example 39, the title compound was obtained in 90% yield.

Example 44

(4R)-3-(3-Phenylpropionyl)-4-(2-propyl)-oxazolidine-2-one.

To a stirred solution of 4-(2-propyl)-oxazolidine-2-one in anhydrous tetrahydrofuran (250 ml) under a nitrogen atmosphere at -78° C were added in a dropwise fashion a solution of n-butyllithium in hexane (50 ml, 77.4 mmol) over 5 to 10 min. After stirring an additional 20 min at -78° C 3-phenylpropionyl chloride (12.7 ml, 85.2 mmol) was added neat. The reaction was warmed to room temperature and stirred 1 to 2 h. The reaction was quenched by adding 100 ml of saturated aqueous ammonium chloride and the volatiles removed by rotary evaporation. The resulting aqueous residue was extracted three times with ether and the combined organic phases were washed with brine, dried ($Na_2SO_4$), filtered and concentrated in vacuo. Recrystallization from hexanes/ethyl acetate provided the title compound (16.6 g, 82%). m.p. = 86.5 to 87.5° C. Mass spectrum: $(M+NH_4)^+$ = 279, $(M+H)^+$ = 262.

Example 45

(4R)-3-[(2R)-3-t-butyloxycarbonyl-2-benzylpropionyl]-4-(2-propyl)-oxazolidine-2-one.

To a stirred solution of the product resulting from Example 44 (2.28 g, 8.72 mmol), in anhydrous tetrahydrofuran (30 ml) under a nitrogen atmosphere at -78° C was added a solution of sodium hexamethyldisilylamide (9.6 ml, 9.59 mmol) in tetrahydrofuran. After stirring for 30 min at -78° C, t-butyl bromoacetate (2.21 g, 11.34 mmol) was added in anhydrous tetrahydrofuran and the resulting solution stirred 1 h at -78° C. The reaction was quenched by adding 20 ml of saturated aqueous ammonium chloride and partitioned between water and ether. The aqueous layer was drawn off and extracted with ether. The combined organic phases were washed with 10% aqueous HCl, saturated aqueous $NaHCO_3$, and brine, dried ($Na_2SO_4$), filtered, and concentrated in vacuo. Recrystallization from acetone/hexanes provided the desired purified product (2.59 g, 79%). m.p. = 167-168° C. Mass spectrum: $(M + NH_4)^+$ = 393, $(M+H)^+$ = 376.

Example 46

Benzyl-(2R)-3-t-butyloxycarbonyl-2-benzylpropionate.

To a stirred solution of dry benzyl alcohol (0.55 ml, 5.33 mmol) in anhydrous tetrahydrofuran (18 ml) under a nitrogen atmosphere at 0° C was added a hexane solution of N-butyllithium (2.58 ml; 4.00 mmol). To this solution was added the product from Example 45 in anhydrous tetrahydrofuran (10 ml). After stirring 1 h at 0° C the reaction was quenched by adding excess saturated aqueous ammonium chloride. The volatiles were removed by rotary evaporation and the resulting aqueous residue extracted two times with

ether. The combined organic layers were washed with brine, dried ($Na_2SO_4$), filtered, and concentrated *in vacuo* provided an oil which was purified by chromatography on $SiO_2$ (15% ethyl acetate/hexanes) to provide the desired product (0.89 g, 94%) as a colorless oil. Mass spectrum: $(M)^+$ = 354.

## Example 47

### Benzyl-(2R)-3-carboxy-2-benzylpropionate.

The product from Example 46 (0.52 g, 1.47 mmol) was dissolved in a 1:1 (v:v) solution (6 ml) of trifluoracetic acid and dichloromethane and stirred at room temperature for 1 h. The volatiles were removed *in vacuo* to provide the title compound (0.437 g, 100%) as an oil which crystallized on standing. The unpurified material was of sufficient purity to employ in subsequent steps. Mass spectrum: $(M)^+$ = 298.

## Example 48

### Benzyl-(2R)-2-benzyl-3-(N-morpholinocarbamoyl)-propionate.

The product from Example 47 (0.438 g, 1.47 mmol), diphenylphosphoryl azide (317 µl, 1.47 mmol), and triethylamine (205 µl, 1.47 mmol) in dry benzene (6 ml) were refluxed for 3 to 5 h to provide a solution of the derived isocyanate which was cooled to 0°C and treated with morpholine (141 µl, 1.62 ml). The cooling bath was removed and the reaction stirred for 1 h. The reaction mixture was poured into 10% aqueous HCl and extracted two times with ether. The combined organic layers were washed successively with saturated aqueous $NaHCO_3$ and brine, dried ($Na_2SO_4$), filtered and concentrated *in vacuo* to provide the unpurified product. The desired product (0.403 g, 72%) was obtained in pure form after chromatography on $SiO_2$ (3% methanol/chloroform) as a thick oil which formed an amorphous solid on standing. Mass spectrum: $(M)^+$ = 382. NMR (300 MHz, $CDCl_3$, ppm, TMS as internal standard) δ 7.12-7.40 (m,10H), 5.18 (AB; J = 12.6 Hz; 2H), 4.8 (dd; J = 5.7 Hz; 1H), 3.59 (dd; J = 6.0, 6.0 Hz; 4H), 3.55 (d,d,d; J = 3.0, 6.0, 14.4 Hz; 1H), 3.37 (d,d,d; J = 5.4, 8.4, 14.4 Hz; 1H), 3.13 (d,d; J = 6.0, 6.0 Hz; 4H), 2.8-3.10 (m,3H).

## Example 49

### Benzyl-(2R)-2-benzyl-3-(ethoxycarbamoyl)-propionate.

The procedure as described in Example 48 was followed except absolute ethanol was employed in lieu of morpholine. Mass spectrum: $(M)^+$ = 341. NMR (300 MHz, $CDCl_3$, ppm, TMS as internal standard) δ 7.1-7.4 (m,10H), 5.17 (s,2H), 4.96 (br s,1H), 4.07 (d,d,d; J = 6.6, 6.6, 6.6 (Hz,2H), 3.25-3.5 (2 br ABX,2H), 2.9-3.05 (m,2H), 2.75-2.88 (br m,1H), 1.23 (d,d; J = 6.6, 6.6 Hz; 3H).

## Example 50

### (2R)-2-Benzyl-3-(morpholinocarbamoyl)-propionic Acid.

The product from Example 49 (0.315 g, 0.86 mmol) was dissolved in ethyl acetate (5 ml) and syringed into a flask charged with 10% Pd/C (~0.3 g). The resulting suspension was exposed to 1 atm of gaseous hydrogen for 2 to 4 h. The catalyst was removed by filtration through a celite pad. The filtrate was concentrated *in vacuo* to provide the desired compound (0.21 g, 88%) as a cream colored foam which was employed without further purification. Mass spectrum: $(M+H)^+$ = 278.

## Example 51

### (2R)-2-Benzyl-3-ethoxycarbamoylpropionic Acid.

The procedure as described in Example 50 was followed employing the product from Example 49 in lieu of that from Example 48. Mass spectrum: $(M+H)^+$ = 252.

## Example 52

### Benzyl (2R)-2-Benzyl-3-morpholinocarbonylpropionate.

The product of Example 47 and morpholine were converted to the title compound using the mixed anhydride method of coupling as described in Example 34. Mass spectrum: $(M)^+$ = 367.

## Example 53

### (2R)-2-Benzyl-3-morpholinocarbonylpropionic Acid.

The product from Example 52 was converted to the title compound following the procedure described in Example 50. Mass spectrum: $(M)^+$ = 277.

## Example 54

### (2R)-3-t-Butyloxycarbonyl-2-benzylpropionic Acid.

The resultant compound from Example 46 and an equal weight of 10% palladium on carbon in methanol were stirred under a hydrogen atmosphere for 1 h. The mixture was filtered and evaporated to provide the desired product. $^1H$ NMR ($CDCl_3$, TMS) $\delta$ 7.25 (m,5H), 3.07 (m,2H), 2.73 (dd,1H), 2.56 (dd,1H), 2.33 (dd,1H), 1.40 (s,9H).

## Example 55

### α-Isocyanato-L-(O-methyl)tyrosine.

A suspension of (O-methyl)tyrosine methyl ester hydrochloride (6 g) in toluene (125 ml) was heated at 100°C while phosgene was bubbled into the reaction mixture. After 2 h the mixture became homogeneous and the phosgene was continued for an additional 15 min. The mixture was cooled and evaporated with several benzene chasers to provide the desired product.

Example 56

1-Benzyloxycarbonylamino-2,3-propanediol.

1-Amino-2,3-propanediol (15.2 g, 167 mmol) and NaOH (8.1 g, 204 mmol) in water (70 ml) at -10°C was treated dropwise with benzyl chloroformate (28.5 ml, 200 mmol) in ether (30 ml) over 20 min. The reaction was stirred at 0°C for 30 min then at room temperature for 2 h. The mixture was acidified with 2 M HCl and extracted with ethyl acetate which was washed with 0.5 M $H_3PO_4$ and brine, then dried over $Na_2SO_4$ and evaporated. Recrystallization of the residue from benzene afforded 16.59 g (44%) of the desired product as a white powder. NMR (300 MHz, $CD_3OD$, ppm): δ 3.12 (dd,1H), 3.28 (dd,1H), 3.50 (m,2H), 3.68(m,1H), 5.08 (s,2H), 7.35 (m,5H).

Example 57

1-Methylamino-2,3-propanediol.

Lithium aluminum hydride (7.20 g, 189 mmol) in tetrahdyrofuran (THF, 300 ml) was heated to reflux and the resultant compound from Example 56 (17.0 g, 75.5 mmol) in THF (150 ml) was added dropwise over 10 min. The mixture was refluxed for 2 h, cooled, quenched sequentially with water (10 ml), 3 M NaOH (40 ml) and water (20 ml), then filtered and concentrated. The residue was dissolved in water which was washed with ether and evaporated. Bulb to bulb distillation of the residue afforded 2.0 g (25%) of the desired compound as an oil. NMR (300 MHz, $CDCl_3$, ppm): δ 2.45 (s,3H), 2.68 (dd,1H), 2.77 (dd,1H), 3.61 (dd,1H), 3.72 (dd,1H), 3.78 (m,1H).

Example 58

(N-Methyl-2,3-dihydroxypropylamino)carbonyl-(0-methyl)tyrosine Methyl Ester.

To the resultant compound from Example 55 (1.53 g, 6.5 mmol) in dioxane (5 ml) at 0°C was added the resultant compound from Example 57 (0.684 g, 6.5 mmol). The reaction was stirred at 0°C for 1 h then at room temperature for 1 h, evaporated and chromatographed on silica gel with 5% methanol in chloroform to afford 1.855 g (84%) of the desired product as an oil. NMR (300 MHz, $CDCl_3$, ppm), δ 2.88, 2.89 (s,3H total), 3.05 (m,2H), 3.26-3.60 (m,5H), 3.73 (s,3H), 3.80 (s,3H), 4.70 (m,1H), 5.07 (broad t,1H), 6.83 (dd,1H), 7.02 (dd,1H).

Example 59

(N-Methyl-2,3-dihydroxypropylamino)carbonyl-(0-methyl)tyrosine.

The resultant compound from Example 58 (114 mg, 0.355 mmol) in dioxane (4 ml) and water (2 ml) at 0 °C was treated with LiOH monohydrate (42.0 mg, 1 mmol). After 90 min, 2M HCl (0.6 ml, 1.2 mmol) was added and the mixture was evaporated to a foam which was used without further purification; DCI-MS: $(M+H)^+$ = 327.

## Example 60

3,3-Dimethylglutaric Acid Mono t-Butyl Ester.

3,3-Dimethylglutaric anhydride (455 mg, 3.2 mmol) in tetrahydrofuran (THF, 5 ml) was treated with sublimed potassium t- butoxide (395 mg, 3.5 mmol). After 30 min the solution was concentrated, poured into saturated $NaHCO_3$ solution and washed with ether. The aqueous phase was acidified to pH 4 with 0.5 M $H_3PO_4$ and extracted with chloroform which was dried over $Na_2SO_4$ and evaporated to afford 179 mg (26%) of the desired product as an oil. NMR (300 MHz, $CDCl_3$, ppm) δ 1.13 (s,6H), 1.47 (s,9H), 2.33 (s, 2H), 2.45 (s,2H).

## Example 61

(4-t-Butyloxycarbonyl-3,3-dimethyl)butanoylphenylalanine Benzyl Ester.

Prepared according to the procedure from Example 34 from the resultant compound from Example 60 and phenylalanine benzyl ester p-toluenesulfonic acid salt. NMR (300 MHz, $CDCl_3$, ppm) δ 0.96 (s,3H), 1.00 (s,3H), 1.44 (s,9H), 1.90 (d,1H), 2.16 (d,1H), 2.25 (d,1H), 2.29 (d,1H), 3.03 (dd,1H), 3.17 (dd,1H), 4.92 (m,1H), 5.12 (d,1H), 5.16 (d,1H), 7.10-7.40 (m,10H).

## Example 62

(4-t-Butyloxycarbonyl-3,3-dimethyl)butanoylphenylalanine.

Using the procedure of Example 54 with the resultant compound of Example 61 gave the desired product as an oil. NMR (300 MHz, $CDCl_3$, ppm), δ 0.93 (s,3H), 0.99 (s,3H), 1.45 (s,9H), 1.77 (d,1H), 2.10 (d,1H), 2.19 (d,1H), 2.25 (d,1H), 3.02 (dd,1H), 3.33 (dd,1H), 4.72 (m,1H), 7.25 (m,5H).

## Example 63

2(R,S)-(4-morpholinylcarbonylmethyl)-3-(1′-naphthyl)-propionic Acid.

To 50 ml of absolute ethanol was added 2 g of sodium metal. The suspension was stirred vigorously until all the sodium dissolved and the evolution of hydrogen ceased. To this solution of sodium ethoxide

was added a solution of 11.6 g of diethylsuccinate in 10.4 g of 1-naphthaldehyde. The solution was heated to reflux for 3 h, at which time it was cooled to room temperature and concentrated. The residue was dissolved in 320 ml of water and extracted 6 times with 100 ml portions of ether. The aqueous layer was acidified with 2N HCl and extracted with 2 x 300 ml of ether and dried with anhydrous magnesium sulfate. Evaporation of the solvent gave a yellow gummy solid which was hydrogenated to the saturated acid using Pd/C as catalyst. Coupling of the resulting saturated acid to morpholine using the mixed anhydride method described in Example 34 followed by ester hydrolysis using the procedure of Example 39 gave the desired acid. Mass spectrum: $(M+H)^+ = 328$.

## Example 64

### [(4-Thiomorpholinyl)carbonyl]-Phe Methyl Ester.

A suspension of L-phenylalanine methyl ester hydrochloride (6 g) in toluene (125 ml) was heated to 100 °C and phosgene gas was bubbled into the reaction mixture. After approximately 1.5 h, the mixture became homogeneous. The bubbling of phosgene was continued for 10 more min. The solvent was then evaporated and the residue chased with benzene several times. The residue was then dissolved in ~100 ml of methylene chloride and cooled to ~0 °C, and 1.1 equivalent of thiomorpholine was added dropwise. After 10 min the solution was washed with 1N HCl and the organic layer was dried with $MgSO_4$. Evaporation of solvent gave 5.5 g of product. Mass spectrum: $M^+ = 308$.

## Example 65

### [(4-Sulphonylmorpholinyl)carbonyl]-Phe Methyl Ester.

To 2 g of the product from Example 64 in 100 ml of methylene chloride was added 2.94 g of meta-chloroperbenzoic acid at 0°C. After 30 min the solvent was evaporated, dissolved in ether, and the ether solution was washed with 10% sodium sulfite solution and then with saturated sodium bicarbonate several times. The organic layer was dried with $MgSO_4$ and evaporation of the solvent gave a white solid which was purified by silica gel column chromatography (20% EtOAc/80%/$CH_2Cl_2$) to give 2.10 g (95%) of pure product. Mass spectrum: $M^+ = 340$.

## Example 66

### [(4-sulfonylmorpholinyl)carbonyl]-Phe-OH.

Using the procedure described in Example 39, with the resultant compound from Example 65 gave the desired compound.

## Example 67

### Boc-6-aminohexanoic Acid.

A mixture of 3.0 g (0.02 mol) of 6-aminohexanoic acid, 5.04 g (0.02 mol) of di-t-butyldicarbonate and 3.84 g (0.05 mol) of $NaHCO_3$ in 160 ml of 1:1 $H_2O$/tetrahydrofuran was stirred vigorously for 24 h. After concentration of the solvent, the mixture was acidified with HCl, saturated with NaCl, extracted with ethyl acetate, dried over $MgSO_4$ and concentrated in vacuo to give the desired product ($R_f$ 0.48, 9:1 chloroform/methanol).

Example 68

Boc-6-aminohexanoyl-(Me)Tyr-$OCH_3$.

The resulting compound from Example 67 and the hydrochloride salt of O-methyltyrosine methyl ester were reacted according to the procedure of Example 34 to provide the desired product as an oil.

Example 69

Boc-6-aminohexanoyl-(Me)Tyr-OH.

The resulting compound from Example 68 was hydrolized according to the procedure of Example 39 to provide the desired product.

Example 70

N-Methyl-N-(2-(N,N-dimethylamino)ethyl)carbamoyl-(0-methyl)tyrosine Methyl Ester.

A solution of 0.5 g (2.1 mmol) of the resultant compound of Example 55 in 50 ml of dichloromethane was cooled to 0°C and treated with 0.3 ml (2.3 mmol) of N,N,N′-trimethylethylenediamine. After being allowed to stir for 16 h, the solution was concentrated and the desired compound was isolated by flash column chromatography using 1% methanol/2% isopropylamine in chloroform.

Example 71

N-Methyl-N-(2-(N,N-dimethylamino)ethyl)carbamoyl-(0-methyl)tyrosine Lithium Salt.

A solution of the resultant compound of Example 70 in dioxane was cooled to 0°C, treated with 1.05 equivalent of aqueous lithium hydroxide (0.5 M) and stirred for 1.5 h. The resulting solution was concentrated in vacuo to give the desired compound as a white solid.

Example 72

Isobutyryl (O-methyl)tyrosine.

To (O-methyl)tyrosine (126 mg, 0.647 mmol) in dioxane (3 ml) was added NaOH (52 mg, 1.3 mmol) in water (3 ml). Isobutyric anhydride (0.11 ml, 0.66 mmol) was added and the mixture was stirred at 0° C for 2 h, poured into saturated $NaHCO_3$ solution and extracted with ether.

The aqueous phase was acidified with concentrated HCl then extracted with ethyl acetate which was dried over $Na_2SO_4$ and evaporated. Trituration with hot ethyl acetate afforded 132 mg (77%) of the desired product as a solid.

Anal. Calcd. for $C_{14}H_{19}NO_4$ 0.25 $H_2O$: C, 62.32; H, 7.28; N, 5.19.
Found: C, 62.68; H, 7.29; N, 5.18.

Example 73

Isobutyryl (phenylmethyl)alanine.

Prepared from phenylmethylalanine according to the procedure of Example 72 to give the desired product as an oil.

Example 74

Isobutyryl (O-benzyl)threonine.

Prepared according to the procedure of Example 72 from (O-benzyl)threonine to give the desired product as a solid.

Anal. Calcd. for $C_{15}H_{21}NO_4$: C, 64.50; H, 7.58; N, 5.01.
Found: C, 64.65; H, 7.51; N, 4.75.

Example 75

Morpholinocarbonyl-(O-methyl)tyrosine Methyl Ester.

To the resultant compound from Example 55 in methylene chloride at 0° C was added one equivalent of morpholine. After 1 h the solvent was evaporated and the residue was chromatographed on silica gel with ethyl acetate/hexane mixtures to provide the desired produce.

Example 76

Morpholinocarbonyl-(O-methyl)tyrosine.

Using the procedure from Example 39 with the resultant compound from Example 75 gave the desired product.

Anal. Calcd. for $C_{15}H_{20}N_2O_5$ 0.25 $H_2O$: C, 57.59; H, 6.66; N, 8.95.
Found: C, 57.79; H, 6.57; N, 8.93.

Example 77

Phenethylmethylaminocarbonyl-(O-methyl)tyrosine Methyl Ester.

Prepared according to the procedure of Example 75 replacing morpholine with N-methyl-2-phenylethylamine. $^1$H NMR ($CDCl_3$,TMS) δ 7.25 (m,5H), 7.02 (m,2H), 6.82 (m,2H), 4.73 (m,2H), 3.78 (s,3H), 3.73 (s,3H), 3.45 (m,2H), 3.03 (d,2H), 2.81 (d,2H), 2.77 (s,3H).

Example 78

Phenethylmethylaminocarbonyl-(O-methyl)tyrosine.

Using the procedure from Example 39 with the resultant compound from Example 77 gave the desired product. $^1$H NMR ($CDCl_3$,TMS) δ 7.28 (m,3H), 7.10 (m,4H), 6.82 (m,2H), 4.40 (m,2H), 3.78 (s,3H), 3.38 (m,2H), 2.70 (s,3H).

Example 79

Diisopropylaminocarbonyl-Phe-$OCH_3$.

Using the procedure of Example 64 but replacing the thiomorpholine with diisopropylamine provided the desired product. $^1$H NMR ($CDCl_3$,TMS) δ 7.10-7.40 (m,5H), 4.83 (m,1H), 4.66 (d,2H), 3.88 (m,2H), 3.74 (s,3H), 3.20 (dd,1H), 3.12 (dd,1H), 1.19 (d,6H), 1.15 (d,6H).

Example 80

Diisopropylaminocarbonylphenylalanine.

Using the procedure of Example 39 with the resultant compound of Example 79 gave the desired product. $^1$H NMR ($CDCl_3$,TMS) δ 7.28 (m,5H), 4.48 (m,2H), 3.72 (s,3H), 3.69 (m,2H), 3.34 (dd,1H), 3.19 (dd,1H), 1.11 (d,6H), 1.05 (d,6H).

Example 81

2-Ethyl-2-methylbutanoyl-Phe-OH.

Using the procedure of Example 72 and replacing isobutyric anhydride with 2-ethyl-2-methylbutanoyl chloride gave the desired compound. $^1H$ NMR ($CDCl_3$,TMS) $\delta$ 7.15-7.35 (m,5H), 6.00 (d,1H), 4.78 (m, 1H), 3.27 (dd,1H), 3.08 (dd,1H), 1.55 (m,2H), 1.35 (m,2H), 1.00 (s,3H), 0.70 (m,6H).

## Example 82

### Benzyl (2R)-2-Benzyl-3-[N-(2-hydroxylethyl)-N-methyl]aminocarbonylpropionate.

Using the mixed anhydride coupling procedure of Example 34 with the resultant compound from Example 47 and N-methylethanolamine gave the desired compound. $^1H$ NMR ($CDCl_3$,TMS) $\delta$ 7.10-7.40 (m,10H), 5.10 (m,2H), 3.00, 2.92 (s,total 3H).

## Example 83

### (2R)-2-Benzyl-3-[N-(2-hydroxyethyl)-N-methyl]aminocarbonylpropionic Acid.

Using the procedure of Example 54 with the resultant compound of Example 82 gave the desired compound. $^1H$ NMR ($CDCl_3$,TMS) $\delta$ 7.25 (m,5H), 2.96,2.93 (s, total 3H).

## Example 84

### Benzyl (2R)-2-Benzyl-3-methylaminocarbonylpropionate.

Using the mixed anhydride coupling procedure of Example 34 with the resultant compound from Example 47 and methylamine gave the desired product. $^1H$ NMR ($CDCl_3$,TMS) $\delta$ 7.05-7.40 (m,10H), 5.42 (broad s,1H), 5.12 (d,1H), 5.06 (d,1H), 3.29 (m,1H), 3.02 (dd,1H), 2.83 (dd,1H), 2.72 (d,3H), 2.48 (dd,1H), 2.27 (dd,1H).

## Example 85

### (2R)-2-Benzyl-3-methylaminocarbonylpropionic Acid.

Using the procedure of Example 54 with the resultant compound of Example 84 gave the desired product. $^1H$ NMR ($CDCl_3$,TMS) $\delta$ 7.15-7.35 (m,5H), 5.83 (m,1H), 2.72 (d,3H).

## Example 86

### 2-N-methyl-benzyloxycarbonylaminoethanol.

To N-methylethanolamine (149 mmol) in methylene chloride (100 ml) at 0°C was added benzyl chloroformate (10.0 ml, 70 mmol). The mixture was stirred at 0°C for 30 min, then at room temperature for 1 h, poured into ethyl acetate, washed with 2 M HCl, saturated $NaHCO_3$ solution, and brine, then dried over $Na_2SO_4$ and evaporated to provide 12.91 g (94%) of the desired compound. $^1H$ NMR ($CDCl_3$,TMS) δ 7.36 (m,5H), 5.14 (s,2H), 3.78 (m,2H), 3.47 (m,2H), 3.01(s, 3H).

## Example 87

### 1-Methoxymethoxy-2-(N-methylbenzyloxycarbonylamino)ethane.

Using the procedure of Example 5 with the resultant compound from Example 86 gave the desired product. $^1H$ NMR ($CDCl_3$,TMS) δ 7.37 (m,5H), 5.14 (s,2H), 4.09 (m,2H), 3.67 (m,2H), 3.50 (m,2H), 3.33 (m,3H), 3.02 (s,3H).

## Example 88

### 1-Methoxyethoxymethoxy-2-(N-methylbenzyloxycarbonylamino)ethane.

Using the procedure of Example 5 with the resultant compound from Example 86 and replacing chloromethyl methyl ether with 2-methoxyethoxy methylchloride gave the desired compound, b.p. 150-170°C (0.3 mm).

## Example 89

### (3,4-cis-Dihydroxypyrrolidinylcarbonyl)-(O-methyl)tyrosine Methyl Ester.

A suspension of L-(O-methyl)tyrosine methyl ester hydrochloride (10 g) in toluene (f200 ml) was heated to 100°C while phosgene gas was bubbled into the reaction mixture. After approximately 2 h the mixture became homogeneous. The bubbling of phosgene was continued for 15 more minutes keeping the temperature at ~100°C. The toluene was then evaporated and the residue chased with benzene several times. The isocyanate from L-(Me)Tyr-$OCH_3$ was then dissolved in ~100 ml of methylene chloride and 1.1 equivalent of 3-pyrroline (75% pure) was added dropwise at 0°C. After 15 min, the reaction mixture was washed with 0.5 N HCl and methylene chloride. The organic layer was washed with aqueous $NaHCO_3$ and dried over $MgSO_4$. Evaporation of the solvent gave 3-pyrrolinylcarbonyl-(Me)Tyr-methyl ester which was cis-hydroxylated under the following conditions: 2.5 g of the 3-pyrrolinylcarbonyl-(Me)Tyr-methyl ester was dissolved in 50 ml of THF and 1 ml of a 2.5% solution of $OsO_4$ in t-butanol was added, followed by 1.15 g of N-methylmorpholine-N-oxide. After 1 h, the solvent was evaporated and the residue dissolved in 150 ml of ethyl acetate and washed with dilute $Na_2SO_3$ solution, saturated $NaHCO_3$ solution and then dried with $MgSO_4$. Evaporation of the solvent gave a gummy solid which was purified by $SiO_2$ column chromatography (5% MeOH/$CH_2Cl_2$) to give the desired compound (65% yield). Mass spectrum: $M^+$ = 338.

## Example 90

(3,4-cis-Dihydroxypyrrolidinylcarbonyl)-(O-methyl)tyrosine.

Using the procedure from Example 39 with the resultant compound from Example 89 and replacing ether extractions with chloroform extractions gave the desired product. Mass spectrum: $M^+$ = 324.

Example 91

1-Benzyloxycarbonyl-3,4-cis-dihydroxypyrrolidine.

3-Pyrroline (75% pure) was reacted with benzyl chloroformate according to the procedure of Example 86 and the resulting product was cis-hydroxylated according to the procedure of Example 89 to give the desired product.

Example 92

1-Benzyloxycarbonyl-3,4-cis-dimethoxymethoxypyrrolidine.

Using the procedure of Example 5 with the resultant compound from Example 91 gave the desired product. $^1$H NMR ($CDCl_3$,TMS) δ 7.35 (m,5H), 5.16 (d,1H), 5.13 (d,1H), 4.73 (m,4H), 4.21 (m,2H), 3.60 (m,4H), 3.40 (s,3H), 3.38 (s,3H).

Example 93

1-Methylamino-2-methoxymethoxyethane.

Using the procedure of Example 54 with the resultant compound from Example 87 gave the desired product, b.p. 60-80 °C (60 mm).

Example 94

1-Methylamino-2-methoxyethoxymethoxyethane.

Using the procedure of Example 54 with the resultant compound from Example 88 gave the desired product, b.p. 130-140 °C (45 mm).

Example 95

Cis-3,4-dimethoxymethoxypyrrolidine.

Using the procedure of Example 54 with the resultant compound from Example 92 gave the desired product. $^1$H NMR ($CDCl_3$,TMS) δ 4.71 (m,4H), 4.12 (m,2H), 3.38 (s,6H), 3.09 (m,2H), 2.90 (m,2H).

Example 96

Benzyl (2R)-2-Benzyl-3-(3,4-cis-dimethoxymethoxypyrrolidinocarbonyl) propionate.

Using the procedure of Example 34 with the resultant compounds from Example 47 and Example 95 gave the desired product. $^1$H NMR ($CDCl_3$,TMS) δ 7.10-7.35 (m,10H), 5.10 (m,2H), 4.72 (m,4H), 4.18 (m,2H), 3.37 (m,6H), 3.05 (m,1H), 2.82 (m,1H), 2.62 (m,1H), 2.30 (m,1H).

Example 97

Benzyl (2R)-2-Benzyl-3-(N-Methyl-N-2-methoxyethoxymethoxyethylaminocarbonyl)propionate.

Using the procedure of Example 34 with the resultant compounds from Example 47 and Example 94 gave the desired product.
Anal. Calcd. for $C_{25}H_{33}NO_6$: C, 67.70; H, 7.50; N, 3.16.
Found: C, 67.79; H, 7.12; N, 3.15.

Example 98

(2R)-2-Benzyl-3-(3,4-cis-dimethoxymethoxypyrrolidinocarbonyl) propionic Acid.

Using the procedure of Example 54 with the resultant compound from Example 96 gave the desired product. $^1$H NMR ($CDCl_3$,TMS) δ 7.26 (m,5H), 4.70 (m,4H), 4.20 (m,2H), 2.75 (dd,1H).

Example 99

(2R)-2-Benzyl-3-(N-Methyl-N-2-methoxyethoxymethoxyethylaminocarbonyl) propionic Acid.

Using the procedure of Example 54 with the resultant compound from Example 97 gave the desired product. $^1$H NMR ($CDCl_3$,TMS) δ 7.27 (m,5H), 4.70,4.55 (m, total 2H), 3.40,3.41 (s, total 3H), 2.97,2.93 (s, total 3H).

Example 100

N-Methyl-(2-methoxymethoxyethyl)aminocarbonyl-(O-methyl)tyrosine Methyl Ester.

Using the procedure of Example 75 and replacing morpholine with the resultant compound from Example 93 gave the desired product. $^1$H NMR ($CDCl_3$,TMS) δ 7.06 (m,2H), 6.82 (m,2H), 5.52 (broad d,1H), 4.70 (m,1H), 4.53 (s,2H), 3.89 (s,3H), 3.82 (s,3H), 3.29 (s,3H), 2.92 (s,3H).

Example 101

N-methyl-(2-methoxymethoxyethyl)aminocarbonyl-(O-methyl)tyrosine.

Using the procedure of Example 39 with the resultant compound from Example 100 gave the desired product. $^1$H NMR ($CDCl_3$,TMS) δ 7.13 (m,2H), 6.84 (m,2H), 4.53 (d,1H), 4.51 (d,1H), 3.80 (s,3H), 3.60 (m,2H), 3.29 (s,3H), 2.89 (s,3H).

Example 102

(N-butyl, 4-$OCH_3$)-phenylalanine.

To a stirred 0 °C suspension of (4-$OCH_3$)-phenylalanine (1.00 g, 5.12 mmol) and butyraldehyde (0.406 g, 110 M%) in methanol (10 ml) was added sodium cyanoborohydride (241 mg, 75 M%). The mixture was warmed to room temperature for 23 h and filtered. The solid was washed with methanol and suction dried to give 1.07 g (83%) of the desired product. Mass spectrum: $M^+$ = 251.
Anal. Calcd. for $C_{14}H_{21}NO_3 \cdot 1/3\ H_2O$: C, 65.3; H, 8.5; N, 5.4.
Found: C, 65.1; H, 8.3; N, 5.6.

Example 103

Ethyl hydrogen (α,α-dimethylbenzyl)malonate.

Diethyl (α,α-dimethylbenzyl)malonate was prepared by the conjugate addition of phenyl magnesium bromide to diethyl isopropylidenemalonate as described by C. Holmberg [Liebigs Ann. Chem., 748 (1981)]. A solution of this diester (42.1 g, 0.15 mmol) in ethanol (100 ml) was treated by dropwise addition with a solution of potassium hydroxide (8.48 g, 0.13 mmol) in 100 ml of ethanol. After heating at 90 °C for 1 h and at 50 °C for 20 h, the reaction mixture was evaporated on the rotary evaporator to a residue. The residue was diluted with water and extracted with ether to remove unreacted starting material. The aqueous phase was cooled to 5 °C, acidified to pH 3 with 6N HCl, and extracted with methylene chloride. The organic layer was washed with brine solution and dried over magnesium sulfate. Evaporation of the solvent gave 27.3 g (84%) of liquid product. $^1$HMR ($CDCl_3$): δ 1.05 (t,3H), 1.6 (s,6H), 3.78 (s,1H), 3.96 (m,2H), 7.2-7.4 (m,5H).

Example 104

Ethyl 2(R,S)-[[(4-morpholinyl)carbonyl]amino]-3,3-dimethyl-3-phenylpropionate.

To a solution of ethyl hydrogen (α,α-dimethylbenzyl)malonate (4 g, 0.016 mmol) in toluene was added triethylamine (2.33 ml, 0.016 mmol) and diphenylphosphoryl azide (4.4 g, 0.016 mmol). The reaction mixture was heated at 100° C for 2.5 h, cooled to 5° C, and treated with 1.4 ml (0.016 mmol) of morpholine. After stirring overnight at room temperature, the toluene solution was washed successively with 1N HCl and aqueous sodium bicarbonate solution. The dried organic solution was evaporated to a residue which was purified by column chromatography on silica gel. There was obtained 3.7 g (69%) of product after trituration with hexane, m.p. 93-94° C.
Anal. Calcd. for $C_{\cdot 3}H_{26}N_2O_4$: C, 64.55; H, 7.84; N, 8.38.
Found: C, 64.72; H, 7.95; N, 8.33.

## Example 105

### 2(R,S)-[[4-Morpholinyl)carbonyl]amino-3,3-dimethyl-3-phenylpropionic Acid.

A solution of the product from Example 104 (2 g, 5.99 mmol) in dioxane (10 ml) was treated with 0.26 g (6.5 mmol) of sodium hydroxide in 5 ml of water. After stirring for 16 h at 35° C, the reaction was worked up as described in Example 39 to give a 93% yield of product.

## Example 106

### Cbz-[(β,β-di-Me)-β-Ala]-Phe-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 39 gave the desired compound.

## Example 107

### [(β,β-di-Me)-β-Ala]-Phe-His Amide of (2S, 3R, 4S)-4-Amino-5-cyclohexyl-2,3-dihydroxy-1-(N-methoxy-N-methylamino)pentane.

Using the procedure of Example 54 with the resultant compound of Example 106 gave the desired product.

## Example 108

### Cbz-[(β,β-di-Me)-β-Ala]-(Me)Tyr-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from

Example 41 gave the desired compound.

Example 109

[(β,β-di-Me)-β-Ala]-Phe-His Amide of (2S, 3R, 4S)-4-Amino-5-cyclohexyl-2,3-dihydroxy-1-(N-methoxy-N-methylamino)pentane.

Using the procedure of Example 54 with the resultant compound of Example 107 gave the desired product.

Example 110

2(S)-[(4-Morpholinyl)carbonyl]oxy-3-phenylpropionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 43 gave the desired compound.

Example 111

(2R)-2-Benzyl-3-(morpholinocarbamoyl)-propionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 50 gave the desired compound.

Example 112

(2R)-2-Benzyl-3-ethoxycarbamoylpropionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 51 gave the desired compound.

Example 113

(2R)-2-Benzyl-3-morpholinocarbonylpropionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 53 gave the desired compound.

## Example 114

(2R)-3-t-Butyloxycarbonyl-2-benzylpropionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 54 gave the desired compound.

## Example 115

(N-Methyl-2,3-dihydroxypropylamino)carbonyl-(0-methyl)tyrosine-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 59 gave the desired compound.

## Example 116

(4-t-Butyloxycarbonyl-3,3-dimethyl)butanoylphenylalanine-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 62 gave the desired compound.

## Example 117

(4-Carboxy-3,3-dimethyl)butanoylphenylalanine-His Amide of (2S, 3R, 4S)-4-Amino-5-cyclohexyl-2,3-dihydroxy-1-(N-methoxy-N-methylamino)pentane Trifluoroacetic Acid Salt.

Using the procedure of Example 47 with the resultant compound from Example 116 gave the title product.

## Example 118

2(R,S)-(4-morpholinylcarbonylmethyl)-3-(1′-naphthyl)-propionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 63 gave the desired compound as a mixture of diasteriomers which could be separated by chromatography on silica gel.

## Example 119

**[(4-sulfonylmorpholinyl)carbonyl]-Phe-His Amide of (2S, 3R, 4S)-4-Amino-5-cyclohexyl-2,3-dihydroxy-1-(N-methoxy-N-methylamino)pentane.**

Using the coupling procedure of Example 33 and replacing Boc-His-OH with the resultant acid from Example 66 gave the desired compound.

## Example 120

**N-Methyl-N-(2-(N,N-dimethylamino)ethyl)carbamoyl-(0-methyl)tyrosine-His Amide of (2S, 3R, 4S)-4-Amino-5-cyclohexyl-2,3-dihydroxy-1-(N-methoxy-N-methylamino)pentane.**

Using the coupling procedure of Example 33 and replacing Boc-His-OH with the resultant acid from Example 71 gave the desired compound.

## Example 121

**Isobutyryl (O-methyl)tyrosine-His Amide of (2S, 3R, 4S)-4-Amino-5-cyclohexyl-2,3-dihydroxy-1-(N-methoxy-N-methylamino)pentane.**

Using the coupling procedure of Example 33 and replacing Boc-His-OH with the resultant acid from Example 72 gave the desired compound.

## Example 122

**Isobutyryl (phenylmethyl)alanine-His Amide of (2S, 3R, 4S)-4-Amino-5-cyclohexyl-2,3-dihydroxy-1-(N-methoxy-N-methylamino)pentane.**

Using the coupling procedure of Example 33 and replacing Boc-His-OH with the resultant acid from Example 73 gave the desired compound.

## Example 123

Isobutyryl (O-benzyl)threonine-His Amid of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 74 gave the desired compound.

Example 124

Morpholinocarbonyl-(O-methyl)tyrosine-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 76 gave the desired compound.

Example 125

Phenethylmethylaminocarbonyl-(O-methyl)tyrosine-His Amid of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 78 gave the desired compound.

Example 126

Diisopropylaminocarbonylphenylalanine-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 80 gave the desired compound.

Example 127

2-Ethyl-2-methylbutanoyl-Phe-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 81 gave the desired compound.

Example 128

(2R)-2-Benzyl-3-[N-(2-hydroxyethyl)-N-methyl]-aminocarbonylpropionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 83 gave the desired compound.

## Example 129

(2R)-2-Benzyl-3-methylaminocarbonylpropionyl-Phe-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His- OH with the resultant acid from Example 85 gave the desired compound.

## Example 130

(3,4-cis-Dihydroxypyrrolidinylcarbonyl)-(O-methyl)tyrosine-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 90 gave the desired compound.

## Example 131

[(2R)-2-Benzyl-3-(3,4-cis-dimethoxymethoxypyrrolidinocarbonyl) propionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 98 gave the desired compound.

## Example 132

(2R)-2-Benzyl-3-(N-Methyl-N-2-methoxyethoxymethoxyethylaminocarbonyl) propionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 99 gave the desired compound.

## Example 133

N-methyl-(2-methoxymethoxyethyl)aminocarbonyl-(O-methyl)tyrosine-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 101 gave the desired compound.

Example 134

(N-butyl, 4-$OCH_3$)-phenylalanine-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 102 gave the desired compound.

Example 135

Ethyl hydrogen (α,α-dimethylbenzyl)malonate-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 103 gave the desired compound.

Example 136

2(R,S)-[[4-Morpholinyl)carbonyl]amino]-3,3-dimethyl-3-phenylpropionyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 105 gave the desired compound as a mixture of diasteriomers which could be separeted by chromatography on silica gel.

Example 137

Dibenzylacetyl-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with dibenzylacetic acid gave the desired compound.

Example 138

(2R,4R,5S)-2-Isobutyl-4-hydroxy-5-tert-butyloxycarbonylamino-6-phenylhexanoic Acid Amide of (2S, 3R, 4S)-4-Amino-5-cyclohexyl-2,3-dihydroxy-1-(N-methoxy-N-methylamino)pentane.

Using the procedure of Evans, et al. (J. Org. Chem. 1985, 50, 4615) with the resultant compound of Example 31 and (3R,5R,1′S)-5-(1-(t-butyloxycarbonylamino)-2-phenylethyl)-3-isobutyldihydrofuran-2-(3H-one (D.J. Kempf, J. Org. Chem. 1986, 51 3921) gave the desired compound after purification by column chromatography.

## Example 139

(2R,4R,5S)-2-(4-Pentenyl)-4-hydroxy-5-tert-butyloxycarbonylamino-6-phenylhexanoic Acid Amide of (2S, 3R, 4S)-4-Amino-5-cyclohexyl-2,3-dihydroxy-1-(N-methoxy-N-methylamino)pentane.

Using the procedure of Evans, et al. (J. Org. Chem. 1985, 50, 4615) with the resultant compound of Example 31 and (3R,5R,1′S)-5-(1-(t-butyloxycarbonylamino)-2-phenylethyl)-3-(4-pentenyl)dihydrofuran-2-(3H)-one (D.J. Kempf, J. Org. Chem. 1986, 51, 3921) gave the desired compound after purification by MPLC.

## Example 140

Benzyl (2R)-2-Benzyl-3-(4-trifluoroethylpiperazin-1-ylcarbonyl)propionate.

The resultant acid from Example 47 (0.500 g, 1.68 mmol) in $CH_2Cl_2$ (7 ml) at -10° C was treated with N-methylmorpholine (0.20 ml, 1.82 mmol) and then isobutyl chloroformate (0.22 ml, 1.68 mmol). After 5 min 1-trifluoroethylpiperazine (0.30 g, 1.78 mmol) was added and the mixture was stirred at -10° C for 15 min and then at room temperature for 2 h. The solvent was evaporated and the residue was taken up in ethyl acetate which was washed with saturated $NaHCO_3$ solution, water and brine, and then dried over $Na_2SO_4$ and evaporated. Chromatography of the residue on silica gel with 20-33% ethyl acetate in hexane provided 0.61 g (81%) of an oil. $^1H$ NMR ($CDCl_3$) $\delta$ 7.10-7.40 (m,10H), 5.15 (d,1H), 5.05 (d,1H), 3.25-3.70 (m,5H), 3.04 (dd,1H), 2.97 (q,2H), 2.81 (dd,1H), 2.72 (dd,1H), 2.60 (m,4H), 2.32 (dd,1H).

## Example 141

(2R)-2-Benzyl-3-(4-trifluoroethylpiperazin-1-ylcarbonyl)propionic Acid.

The resultant compound from Example 140 (610 mg), and 10% palladium on carbon (300 mg) in methanol were stirred under an $H_2$ atmosphere for 2 h. Filtration and solvent evaporation afforded 470 mg (96%) of a solid, m.p. 96-98° C.

## Example 142

Benzyl (2R)-2-Benzyl-3-(4-methylpiperazin-1-ylcarbonyl)propionate.

Using the procedure of Example 140 but replacing 1-trifluoroethylpiperazine with 1-methylpiperazine provided the desired product as an oil. $^1$H NMR ($CDCl_3$ $\delta$ 7.10-7.40 (m,10H), 5.16 (d,1H), 5.05 (d,1H), 3.25-3.70 (m,5H), 3.03 (dd,1H), 2.82 (dd,1H) 2.72 (dd,1H), 2.35 (m,5H), 2.28 (s,3H).

Example 143

(2R)-2-Benzyl-3-(4-methylpiperazin-1-ylcarbonyl)propionic Acid.

Prepared from the resultant compound of Example 142 according to the procedure of Example 141. $^1$H NMR ($CDCl_3$) $\delta$ 7.15-7.35 (m,6H), 2.50-2.80 (m,4H), 2.47 (s,3H), 2.30 (dd,1H).

Example 144

Benzyl (2R)-2-Benzyl-3-((2-pyridin-2-ylethyl)methylaminocarbonyl)propionate.

Using the procedure of Example 140 but replacing 1-trifluoroethylpiperazine with 2-(2-methylaminoethyl)pyridine provided the desired product as an oil. $^1$H NMR ($CDCl_3$ $\delta$ 8.48 (m,1H), 7.57 (m,1H), 6.95-7.40 (m,12H), 5.00-5.20 (m,2H), 2.87, 2.82 (2s,total 3H), 2.31, 2.18 (2dd,total 1H).

Example 145

(2R)-2-Benzyl-3-((2-pyridin-2-ylethyl)methylaminocarbonyl)propionic Acid.

Prepared from the resultant compound of Example 144 according to the procedure of Example 141. $^1$H NMR ($CDCl_3$) $\delta$ 8.49 (m,1H), 7.58 (m,1H), 6.95-7.32 (m,7H), 2.87, 2.72 (2s,total 3H).

Example 146

Benzyl (2R)-2-Benzyl-3-((N-pyridin-4-yl)methylaminocarbonyl)propionate.

Using the procedure of Example 140 but replacing 1-trifluoroethylpiperazine with 4-methylaminopyridine provided the desired product $^1$H NMR ($CDCl_3$ $\delta$ 8.6 (m,2H), 7.4-7.0 (m,12H), 5.1 (q,2H), 3.3 (m,1H), 3.2 (s,3H), 3.0 (dd,1H), 2.7 (dd,1H), 2.6 (dd,1H), 2.25

Example 147

(2R)-2-Benzyl-3-((N-pyridin-4-yl)methylaminocarbonyl)propionic Acid.

Prepared from the resultant compound of Example 146 according to the procedure of Example 141, m.p. 88-92 °C.

Example 148

Benzyl (2S)-2-(4-morpolinyl)-3-phenylpropionate.

2.5-Dihydrofuran (0.78 ml, 10.3 mmol) in methanol (4 ml) and $CH_2Cl_2$ (16 ml) at -60 °C was treated with ozone until a blue color persisted, and the excess ozone was removed under a stream of $N_2$. To this solution was added $NaCNBH_3$ (456 mg, 7.26 mmol). After 15 min at -60 °C, H-Phe-OBn p-toluenesulfonic acid salt (2.22 g, 5.19 mmol) in methanol (20 ml) was added over 5 min, and the mixture was stirred at -60 °C for 15 min and at 0 °C for 20 h. The mixture was quenched with acetic acid (0.30 ml, 5.2 mmol), stirred at 0 °C for 30 min and the solvent was evaporated. The residue was taken up in saturated $NaHCO_3$ solution and extracted into $CH_2Cl_2$ which was dried over $Na_2SO_4$ and evaporated. Chromatography of the residue on silica gel with 20% ethyl acetate in hexane afforded 1.374 g (81%) of an oil. $^1H$ NMR ($CDCl_3$) δ 7.10-7.35 (m,10H), 5.03 (s,2H), 3.62-3.75 (m,4H), 3.48 (dd,1H), 3.08 (dd,1H), 2.97 (dd,1H), 2.58-2.80 (m,4H).

Example 149

(2S)-2-(4-Morpholinyl)-3-phenylpropionic Acid.

Prepared from the resultant compound of Example 148 according to the procedure of Example 141. $^1H$ NMR ($CD_3OD$) δ 7.15-7.35 (m,5H), 3.78 (m,4H), 3.57 (m,1H), 3.12 (m,2H), 3.03 (m,4H).

Example 150

Benzyl 2(R)-2-Benzyl-3-chloromethylcarbonylpropionate.

The resultant acid from Example 47 (500 mg, 1.68 mmol) in $CH_2Cl_2$ (8 ml) at 0 °C was treated with oxalyl chloride (0.160 ml, 1.83 mmol) and dimethylformamide (0.0065 ml). After 2 h at 0 °C, the solvent was evaporated and the residue was dissolved in ether (6 ml), cooled to 0°C and treated with an ether solution of $CH_2N_2$. After 2 h at 0 °C the solvent was evaporated and the residue was dissolved in ether (6 ml), cooled to -10 °C, and treated with 4.0 M HCl/ dioxane (0.6 ml, 2.4 mmol). After 1 h the solvent was evaporated and the residue was chromatographed on silica gel with 10% ethyl acetate in hexane to afford 476.8 mg (83%) of a colorless oil. $^1H$ NMR ($CDCl_3$) δ 7.08-7.40 (m,10H), 5.12 (d,1H), 5.08 (d,1H), 4.02 (s,2H), 3.30 (m,1H), 3.10 (dd,1H), 2.97 (dd,1H), 2.78 (dd,1H), 2.55 (dd,1H).

Example 151

Benzyl (2R)-2-Benzyl-3-thiazol-4-ylpropionate.

The resultant compound from Example 150 (476.8 mg, 1.44 mmol) and thioformamide (176 mg, 2.88 mmol) in acetone (6 ml) were stirred at room temperature for 108 h. N-methylmorpholine (0.16 ml, 1.40 mmol) was added and after 20 min the mixture was diluted with ether, filtered, evaporated, and chromatographed on silica gel with 20% ethyl acetate in hexane to afford 369 mg (76%) of an oil $^1H$ NMR ($CDCl_3$) $\delta$ 9.70 (d,1H), 7.05-7.35 (m,10H), 6.90 (d,1H), 5.00 (d,1H), 4.95 (d,1H), 3.28-3.35 (m,1H), 3.19 (dd,1H), 2.95-3.10 m,2H), 2.88 (dd,1H).

## Example 152

### (2R)-2-Benzyl-3-thiazol-4-ylpropionic Acid.

The resultant compound from Example 151 (364 mg) was stirred for 2h in 30% HBr in acetic acid (5 ml). The solvent was evaporated and the residue was dissolved in 1 M HCl and washed with ether. The aqueous phase was adjusted to pH 4 with solid $NaHCO_3$ and extracted with chloroform which was dried over $Na_2SO_4$ and evaporated to afford 186.5 mg (70%) of an oil. $^1H$ NMR ($CDCl_3$) $\delta$ 8.78 (d,1H), 7.15-7.35 (m,5H), 6.99 (d,1H), 3.00-3.30 (m,4H), 2.81 (dd,1H).

## Example 153

### Benzyl (2R)-2-Benzyl-5-tert-butylmercapto-4-oxopentanoate.

To tert-butylmercaptan (0.11 ml) in dimethylformamide (5 ml) at 0°C was added potassium bis-(trimethylsilyl)amide in toluene (1.80 ml, 0.90 mmol, 0.5 M) followed by the resultant compound from Example 150 (259.6 mg, 0.785 mmol) in dimethylformamide (3 ml). After 16 h at room temperature the mixture was diluted with ethyl acetate, washed with water and brine, then dried over $Na_2SO_4$ and evaporated. Chromatography of the residue on silica gel with 10% ethyl acetate in hexane afforded 219.6 mg (73%) of a colorless oil. $^1H$ NMR ($CDCl_3$ $\delta$ 7.10-7.40 (m,10H), 5.07 (s,2H), 3.25 (s,2H), 3.18-3.29 (m,1H), 2.97-3.07 (m,2H), 2.78 (dd,1H), 2.71 (dd,1H), 1.25 (s,9H).

## Example 154

### Benzyl (2R)-2-Benzyl-5-tert-butylsulfinyl-4-oxopentanoate.

The resultant compound from Example 153 (44.4 mg, 0.115 mmol) in $CH_2Cl_2$ (2 ml) at -10°C was treated with meta-chloroperbenzoic acid (25.0 mg, 0.116 mmol, 80% pure). After 2 h at -10-0°C the solvent was evaporated and the residue was dissolved in ethyl acetate which was washed with 1:1 10% $Na_2SO_3$ solution saturated $NaHCO_3$ solution, saturated $NaHCO_3$ solution and brine, and then dried over $Na_2SO_4$ and evaporated to afford 46.0 mg (99%) of a colorless oil. $^1H$ NMR ($CDCl_3$) $\delta$ 7.05-7.40 (m,10H), 5.08 (m,2H), 3.36-3.53 (m,2H), 3.30 (m,1H), 2.95-3.18 (m,2H), 2.80 (2dd,total 1H), 2.69 (2dd,total 1H), 1.24 (s,9H).

## Example 155

Benzyl (2R)-2-Benzyl-5-tert-butylsulfonyl-4-oxopentanoate.

The resultant compound from Example 153 (171.9 mg, 0.447 mmol) in $CH_2Cl_2$ (5 ml) was treated with meta-chloroperbenzoic acid (290 mg, 1.34 mmol, 80% pure). After 75 min at room temperature the product was isolated as described in Example 154 to afford 184.0 mg (59%) of a colorless oil. $^1H$ NMR ($CDCl_3$) $\delta$ 7.08-7.35 (m,10H), 5.07 (s,2H), 3.98 (d,1H), 3.88 (d,1H), 3.23-3.33 (m,1H), 3.18 (dd,1H), 3.03 (dd,1H), 2.88 (dd,1H), 2.82 (dd,1H), 1.38 (s,9H).

## Example 156

(2R)-2-Benzyl-5-tert-butylsulfinyl-4-oxopentanoic Acid.

Prepared from the resultant compound from Example 154 according to the procedure of Example 141. $^1H$ NMR ($CDCl_3$) $\delta$ 7.15-7.35 (m,5H), 1.23 (s,9H).

## Example 157

(2R)-2-Benzyl-5-tert-butylsulfonyl-4-oxopentanoic Acid.

Prepared from the resultant compound from Example 155 according to the procedure of Example 141. $^1H$ NMR ($CDCl_3$) $\delta$ 7.15-7.35 (m,5H), 3.94 (d,1H), 3.88 (d,1H), 2.90-3.30 (m,3H), 2.70-2.85 (m,2H), 1.39 (s,9H).

## Example 158

Benzyl (2R)-2-Benzyl-5-morpholin-4-yl-4-oxopentanoate.

The resultant compound from Example 150 (610 mg, 1.84 mmol) in dimethylformamide (10 ml) was treated with NaI (33 mg, 0.22 mmol) and morpholine (0.60 ml, 6.88 mmol). After 2 h the mixture was diluted with ethyl acetate, washed with water and brine, and then dried over $Na_2SO_4$, and evaporated. Chromatography of the residue on silica gel with 60% ethyl acetate/40% hexane afforded 460 mg (65%) of an oil. $^1H$ NMR ($CDCl_3$) $\delta$ 7.05-7.40 (m,10H), 5.11 (d,1H), 5.06 (d,1H), 3.68 (m,4H), 3.22-3.32 (m,1H), 3.15 (d,1H), 3.08 (d,1H), 3.05 (m,1H), 2.87 (dd,1H), 2.77 (dd,1H), 2.35-2.50 (m,5H).

## Example 159

(2R)-2-Benzyl-5-morpholin-4-yl-4-oxopentanoic Acid.

Prepared from the resultant compound from Example 158 according to the procedure of Example 141. $^1H$ NMR ($CDCl_3$) $\delta$ 7.15-7.35 (m,5H), 3.60-3.75 (m,4H).

## Example 160

### Methyl α-Benzylacrylate.

α-Benzylacrylic acid (1.00 g, 6.17 mmol) in methanol (20 ml) was treated with $BF_3 \cdot Et_2O$ (2 ml). The mixture was heated to reflux for 14 h, cooled, and poured into saturated $NaHCO_3$ solution. Extraction with ether followed by drying over $Na_2SO_4$ and evaporation afforded 1.03 g (95%) of a mobile oil. $^1H$ NMR ($CDCl_3$)δ 7.17-7.35 (m,5H), 6.23 (m,1H), 5.47 (m,1H), 3.74 (s,3H), 3.63 (s,2H).

## Example 161

### Methyl (2RS)-2-benzyl-3-(N-methoxyl-N-methylamino)propionate.

The resultant compound from Example 160 (800 mg, 4.54 mmol), N-methyl,O-methylhydroxylamine hydrochloride (0.57 g, 5.4 mmol), and $NaHCO_3$ (0.46 g, 5.48 mmol) in dimethylsulfoxide (5 ml) were heated at 130°C for 20 h. The mixture was diluted with ethyl acetate, washed with water, saturated $NaHCO_3$ solution and brine, and then was dried over $Na_2SO_3$ and evaporated. Chromatography of the residue on silica gel with 10% ethyl acetate in hexane afforded 226 mg (21%) of a mobile oil. $^1H$ NMR ($CDCl_3$) δ 7.10-7.30 (m,5H), 3.60 (s,3H), 3.47 (s,3H), 2.80-3.10 (m,4H), 2.60 (dd,1H), 2.55 (s,3H).

## Example 162

### Methyl (2RS)-2-benzyl-3-pyrazol-1-ylpropionate.

Using the procedure of Example 161 but replacing N-methyl, O-methylhydroxylamine hydrochloride and $NaHCO_3$ with pyrazole provided the desired product as an oil. $^1H$ NMR ($CDCl_3$) δ 7.52 (d,1H), 7.10-7.35 (m,6H), 6.10 (dd,1H), 4.38 (dd,1H), 4.24 (dd,1H), 3.57 (s,3H), 3.37 (m,1H), 2.98 (dd,1H), 2.82 (dd,1H).

## Example 163

### (2RS)-2-Benzyl-3-pyrazol-1-ylpropionic Acid.

The resultant compound from Example 162 (100.0 mg, 0.409 mmol) in dioxane (2 ml) at 0°C was treated with $LiOH \cdot H_2O$ (22.0 mg, 0.524 mmol) in water (1 ml). After 1 h at 0°C and 30 min at room temperature the solvent was evaporated and the residue was taken up in water, the pH was adjusted to pH 3-4, and the mixture was extracted with $CHCl_3$ which was dried over $Na_2SO_4$ and evaporated to afford 96 mg (100%) of a solid. $^1H$ NMR ($CDCl_3$) δ 7.56 (d,1H), 7.10-7.35 (m,6H), 6.26 (dd,1H), 4.30 (m,2H), 3.34 (m,1H), 3.12 (dd,1H), 2.72 (dd,1H).

## Example 164

(2RS)-2-Benzyl-3-(N-methoxyl-N-methylamino)propionic Acid.

Using the procedure of Example 163 with the resultant compound from Example 161 gave the desired product $^1H$ NMR ($CDCl_3$) δ 7.10-7.35 (m,5H), 3.58 (s,3H), 2.62 (s,3H).

Example 165

Methyl (2RS)-2-benzyl-3-tert-butylmercaptopropionate.

To sodium (3.05 g, 133 mmol) in methanol (135 ml) was added tert-butylmercaptan (17.0 ml, 151 mmol). After 20 min methyl α-benzylacrylate (17.05 g, 96.8 mmol) in methanol (100 ml) was added and after 1 h at room temperature the mixture was heated at reflex for 17 h. After cooling, the mixture was acidified with 2 M HCl (70 ml), concentrated, taken up in ether, washed with water and brine, then dried over $MgSO_4$ and evaporated to 23.59 g (92%) of an oil. $^1H$ NMR ($CDCl_3$) δ 7.15-7.35 (m,5H), 3.63 (s,3H), 2.60-3.05 (m,5H), 1.28 (s,9H).

Example 166

Methyl (2RS)-2-benzyl-3-tert-butylsulfonylpropionate.

To the resultant compound from Example 165 (270 mg, 1.01 mmol) in methanol (6 ml) and water (5 ml) at 0° C was added potassium peroxymonosulfate (1.845 g, 6 mmol) in portions. After 15 min at 0° C and 24 h at room temperature the mixture was filtered, diluted with water, and extracted with $CH_2Cl_2$ which was washed with brine, dried over $MgSO_4$, and evaporated to 300 mg (99%) of an oil. $^1H$ NMR δ 7.15-7.35 (m,5H), 3.68 (s,3H), 3.45 (m,2H), 3.12 (dd,1H), 2.98 (m,2H), 1.37 (s,9H).

Example 167

(2RS)-2-Benzyl-3-tert-butylsulfonylpropionic Acid.

The resultant compound from Example 166 (282 mg, 0.95 mmol) in 6 M HCl (2 ml) and acetic acid (0.4 ml) was heated at reflux for 16 h. The mixture was cooled and filtered and the resulting solid was recrystallized from methycyclohexane/ethyl acetate to afford 152 mg (56%) of the desired product, m.p. 147-148° C.

Example 168

(2S)-2-Benzyl-3-tert-butylsulfonylpropionic Acid Amide of Histidine Benzyl Ester.

Using the carbodiimide coupling procedure of Example 33 with the resultant acid from Example 167 and histidine benzyl ester di-p-toluene sulfonic acid salt gave after workup the crude (2RS) amides.

Chromatography on silica gel with 1.5-2% $CH_3OH$ in $CHCl_3$ afforded 704 mg of the (2S)-isomer followed by 1.07 g of the (2R)-isomer.

(2S)-isomer: ¹H NMR ($CDCl_3$) δ 7.48 (d,1H), 7.10-7.45 (m,11H), 6.52 (s,1H), 6.40 (br,1H), 5.12 (d,1H), 5.07 (d,1H), 4.70 (m,1H), 3.78 (dd,1H), 3.10-3.25 (m,3H), 3.02 (dd,1H), 2.91 (dd,1H), 2.82 (dd,1H), 1.40 (s,9H).

(2R)-isomer: ¹H NMR ($CDCl_3$) δ 7.37 (s,1H), 7.20-7.40 (m,11H), 7.04 (br,1H), 6.43 (s,1H), 5.15 (d,1H), 5.07 (d,1H), 4.59 (m,1H), 3.48 (dd,1H), 3.23 (m,1H), 2.98-3.10 (m,4H), 2.83 (dd,1H), 1.38 (s,9H).

## Example 169

### (2S)-2-Benzyl-3-tert-butylsulfonylpropionic Acid Amide of (imidazole-Boc)Histidine Benzyl Ester.

To the resultant (2S)-isomer from Example 168 (700 mg, 1.37 mmol) in $CH_2Cl_2$ (4.6 ml) was added di-tert-butyldicarbonate (314 mg, 1.44 mmol). After 14 h the solvent was evaporated to afford the desired product. ¹H NMR ($CDCl_3$) δ 7.92 (d,1H), 7.15-7.40 (m,10H), 7.10 (d,1H), 7.06 (br,1H), 5.12 (d,1H), 5.08 (d,1H), 4.83 (m,1H), 3.58 (dd,1H), 3.22 (m,1H), 2.85-3.15 (m,5H), 1.60 (s,9H), 1.34 (s,9H).

## Example 170

### (2S)-2-Benzyl-3-tert-butylsulfonylpropionic Acid Amide of (imidazole-Boc)Histidine.

Using the procedure of Example 141 with the resultant product from Example 169 provided the desired product. ¹H NMR ($CDCl_3$) δ 8.13 (d,1H), 7.58 (s,1H), 7.15-7.35 (m,5H), 6.47 (br,1H), 4.62 (m,1H), 3.69 (dd,1H), 3.00-3.27 (m,4H), 2.28-2.42 (m,2H), 1.60 (s,9H), 1.37 (s,9H).

## Example 171

### Isonicitonyl-(O-methyl)tyrosine Benzyl Ester.

To (O-methyl)tyrosine benzyl ester hydrochloride salt (1.07 mmol) in $CH_2Cl_2$ (10 ml) was added isonicitonyl chloride hydrochloride (200 mg, 1.12 mmol) and N-methylmorpholine (0.42 ml, 3.82 mmol). After 3 h the mixture was concentrated, taken up in ethyl acetate, washed with saturated $NaHCO_3$ solution, water and brine, and then dried over $Na_2SO_4$ and evaporated. Chromatography of the residue on silica gel with 1:1 ethyl acetate/hexane afforded 132.5 mg (32%) of a solid. ¹H NMR ($CDCl_3$) δ 8.73 (m,2H), 7.54 (m,2H), 7.33-7.43 (m,5H), 6.89 (m,2H), 6.74 (m,2H), 6.61 (d,1H), 5.26 (d,1H), 5.17 (d,1H), 5.07 (m,1H), 3.77 (s,3H), 3.20 (m,2H).

## Example 172

### Isonicitonyl-(O-methyl)tyrosine.

Using the procedure of Example 141 with the resultant compound from Example 171 gave the desired

product. $^1$H NMR ($CD_3OD$) δ 8.65 (d,2H), 7.68 (dd,2H), 7.18 (m,2H), 6.82 (m,2H), 4.77 (dd,1H), 3.73 (s,3H), 3.32 (m,2H).

## Example 173

### 1-Methylisonipicotonyl-(O-methyl)tyrosine Benzyl Ester.

Using the carbodiimide coupling procedure of Example 33 with 1-methylisonipicotic acid and (O-methyl)tyrosine benzyl ester hydrochloride salt gave the desired product after chromatography on silica gel with 4-6% methanol in chloroform. $^1$H NMR ($CDCl_3$) δ 7.30-7.43 (m,5H), 6.87 (m,2H), 6.73 (m,2H), 5.89 (d,1H), 5.20 (d,1H), 5.12 (d,1H), 4.89 (m,1H), 3.77 (s,3H), 3.07 (m,2H), 2.87 (m,2H), 2.28 (s,3H), 1.65-2.15 (m,7H).

## Example 174

### 1-Methylisonipicotonyl-(O-methyl)tyrosine.

Using the procedure of Example 141 with the resultant compound from Example 173 provided the desired product as a waxy solid. $^1$H NMR ($CD_3OD$) δ 7.12 (m,2H), 6.81 (m,2H), 4.55 (dd,1H), 3.74 (s,3H), 2.82 (s,3H).

## Example 175

### 1-Methylpiperazin-4-ylcarbonyl-)O-methyl)tyrosine Methyl Ester.

To a-isocyanato-L-(O-methyl)tyrosine methyl ester (0.250 g) in $CH_2Cl_2$ (5 ml) was added 1-methylpiperazine (0.12 ml, 1.08 mmol). After 2 h the solvent was evaporated and the residue was chromatographed on silica gel with 2% methanol in chloroform to afford the desired product as a solid $^1$H NMR ($CDCl_3$) δ 7.02 (m,2H), 6.83 (m,2H), 4.84 (d,1H), 4.76 (m,1H), 3.79 (s,3H), 3.73 (s,3H), 3.37 (m,4H), 3.07 (m,2H), 2.37 (m,4H), 2.31 (s,3H).

## Example 176

### 1-Methylpiperazin-4-ylcarbonyl-(O-methyl)tyrosine.

To the resultant compound from Example 175 (375.0 mg, 1.12 mmol) in dioxane (6 ml) at 0 °C was added $LiOH \cdot H_2O$ (56.3 mg, 1.34 mmol) in $H_2O$ (3 ml). After 70 min the reaction was quenched with 2 M HCl (0.67 ml, 1.34 mmol), concentrated diluted with brine, and extracted into chloroform which was dried over $Na_2SO_4$ and evaporated. The residue was suspended in ethanol, filtered, and the filtrate was evaporated to afford 344.8 mg (96%) of a white solid. $^1$H NMR ($CD_3OD$) δ 7.13 (m,2H), 6.81 (m,2H), 4.42 (m,1H), 3.75 (s,3H), 3.50 (m,4H), 3.33 (m,1H), 2.93 (m,1H), 2.82 (m,4H), 2.61 (s,3H).

## Example 177

### Methyl (2S)-(4-methylpiperazin-1-yl)carbonyloxy-3-phenylpropionate.

Using the procedure of Example 42 but replacing morpholine with 1-methylpiperazine and modifying the isolation procedure to omit the HCl washes afforded, after chromatography on silica gel with 2% methanol in chloroform, the desired product as a colorless oil. $^1$H NMR ($CDCl_3$) δ 7.17-7.33 (m,5H), 5.18 (dd,1H), 3.73 (s,3H), 3.46 (m,4H), 3.18 (dd,1H), 3.09 (dd,1H), 2.34 (m,4H), 2.28 (s,3H).

## Example 178

### (2S)-(4-Methylpiperazin-1-yl)carbonyloxy-3-phenylpropionic Acid.

Using the procedure of Example 39 but replacing ether extractions with chloroform extractions, with the resultant compound from Example 177 afforded the desired product.
$^1$H NMR ($CD_3OD$) δ 7.15-7.30 (m,5H), 4.98 (m,1H), 2.78 (s,3H).

## Example 179

### Benzyl (2R)-2-benzyl-3-((2-morpholin-4-ylethyl)methylaminocarbonyl)propionate.

To benzyl (2R)-2-benzyl-3-(N-(2-hydroxyethyl)-N-methyl)aminocarbonylpropionate (110 mg, 0.31 mmol) in $CH_2Cl_2$ (2 ml) at -78° C was added triethylamine (0.070 ml, 0.50 mmol) and methanesulfonyl chloride (0.036 ml, 0.047 mmol). After 1 h morpholine (0.085 ml, 0.97 mmol) was added and the mixture was stirred at room temperature for 5 h. The solvent was evaporated and the residue was suspended in ethyl acetate, washed with saturated $NaHCO_3$ solution, water and brine, then dried over $Na_2SO_4$ and evaporated. Chromatography of the residue on silica gel with 2:1 ethyl acetate in hexane afforded 90.0 mg (68%) of the desired produce. $^1$H NMR ($CDCl_3$) δ 7.10-7.37 (m,10H), 5.00-5.20 (m,2H), 3.60-3.73 (m,4H), 2.94, 2.90 2s,total 3H).

## Example 180

### (2R)-2-Benzyl-3-((2-morpholin-4-ylethyl)methylaminocarbonyl)propionic Acid.

Using the procedure of Example 141 with the resultant compound from Example 179 gave the desired product. $^1$H NMR ($CDCl_3$) δ 7.17-7.33 (m,5H), 3.60-3.70 (m,4H), 2.92, 2.86 (2s,total 3H).

## Example 181

### Benzyl (2R)-2-benzyl-3-((2-imidazol-1-ylethyl)methylaminocarbonyl)propionate.

Using the procedure of Example 179 and replacing morpholine with imidazole and changing stirring at room temperature for 5 h to heating at reflux for 4 h gave, after chromatography on silica gel with 0.5% methanol in chloroform, the desired product.
$^1$H NMR ($CDCl_3$) δ 7.42 (s,1H), 7.00-7.40 (m,11H), 6.89 (s,1H), 5.18 (d,1H), 5.08 (s,1H), 4.05 (m,2H), 3.61 (m,1H), 3.50 (m,1H), 3.32 (m,1H), 3.08 (m,1H), 2.60-2.85 (m,2H), 2.59 (s,3H), 2.27 (dd,1H).

## Example 182

### (2R)-2-Benzyl-3-((2-imidazol-1-ylethyl)methylaminocarbonyl)propionic Acid.

Using the procedure of Example 141 with the resultant compound from Example 181 gave the desired product $^1$H NMR ($CDCl_3$) δ 7.83 (s,1H), 7.15-7.32 (m,5H), 7.14 (s,1H), 6.93 (s,1H), 3.30 (m,2H), 3.09 (m,1H), 2.60-2.78 (m,2H, 2.60 (s,3H).

## Example 183

### Benzyl (2R)-2-benzyl-3-(2-(4-methylpiperazin-1-ylethyl)methylaminocarbonyl)propionate.

Using the procedure of Example 179 and replacing morpholine with 1-methylpiperazine and changing stirring at room temperature for 5 h to heating at reflux for 4 h gave, after chromatography on silica gel with 1-3% methanol in chloroform, the desired product.
$^1$H NMR ($CDCl_3$) δ 7.10-7.35 (m,10H), 5.00-5.20 (m,2H), 2.93, 2.89 (2s,total 3H), 2.28 (2s,total 3H).

## Example 184

### (2R)-2-Benzyl-3-(2-(4-methylpiperazin-1-ylethyl)methylaminocarbonyl)propionic Acid.

Using the procedure of Example 141 with the resultant compound from Example 231 gave the desired product $^1$H NMR ($CDCl_3$) δ 7.13-7.32 (m,5H), 2.92, 2.88 (2s,total 3H), 2.31, 2.33 (2s,total 3H).

## Example 185

### Benzyl (2R)-2-benzyl-3-((2-diethylaminoethyl)methylaminocarbonyl)propionate.

Using the procedure of Example 140 but replacing 1-trifluoroethylpiperazine with N,N-diethyl-N′-methylethylene diamine gave, after chromatography on silica gel with 3% methanol in chloroform, the desired product. $^1$H NMR ($CDCl_3$) δ 7.09-7.37 (m,10H), 5.02-5.20 (m,2H), 2.94, 2.88 (2s,total 3H), 0.91-1.08 (m,6H).

Example 186

(2R)-2-Benzyl-3-((2-diethylaminoethyl)methylaminocarbonyl)propionic Acid.

Using the procedure of Example 141 with the resultant product from Example 233 gave the desired product. $^1$H NMR ($CDCl_3$ δ 7.12-7.30 (m,5H), 3.00 (s,3H), 1.17 (t,6H).

Example 187

Benzyl (2R)-2-benzyl-3-((2-pyrazol-1-ylethyl)methylaminocarbonyl)propionate.

Using the procedure of Example 179 and replacing morpholine with pyrazole and changing stirring at room temperature for 5 h to heating at reflux for 4 h gave, after chromatography on silica gel with 0.5% methanol in chloroform, the desired product.
$^1$H NMR ($CDCl_3$) δ 7.51, 7.40(2d, total 1H), 7.18-7.38(m, 10H), 7.08-7.17(m, 1H), 6.21, 6.13(2dd, total 1H), 4.99-5.20(4d, total 2H), 2.78, 2.52(2s, total 3H).

Example 188

(2R)-2-Benzyl-3-((2-pyrazol-1-ylethyl)methylaminocarbonyl)propionic Acid.

Using the procedure of Example 141 with the resultant compound from Example 187 gave the desired product. $^1$H NMR ($CDCl_3$) δ 7.62(d, 1H), 7.51, 7.45(2d, total 1H), 7.15-7.37(m, 5H), 6.22, 6.19(2dd, total 1H), 2.82, 2.49(2s, total 3H).

Example 189

Benzyl α-Benzylacrylate.

α-Benzylacrylic acid (2.20 g, 13.6 mmol) in dry ether (40 mL) was treated with dicyclohexylcarbodiimide (2.60 g, 12.6 mmol), benzyl alcohol (1.30 mL, 12.6 mmol) and 4-dimethylaminopyridine (0.310 g, 2.54 mmol). After stirring at room temperature for 44 h, the mixture was filtered and evaporated. Chromatography of the residue on silica with 5% ethyl acetate in hexane afforded 2.70 g (85%) of a colorless oil. TLC (20% ethyl acetate:80% hexane) $R_f$ = 0.59; $^1$H NMR ($CDCl_3$) δ 7.15-7.40 (10H,m), 6.28 (1H,m), 5.49 (1H,m), 5.17 (2H,s), 3.67 (2H,s).

Example 190

Benzyl 3-Acetylmercapto-2-benzylpropionate.

The resulting compound from Example 189 (7.00 g, 27.7 mmol) in dry ether (10 mL) was treated with thiolacetic acid (3.00 mL, 42.0 mmol) and pyridine (2.30 mL, 28.4 mmol). After 114 h at room temperature the mixture was evaporated and chromatographed on silica gel (500 g) with 5-10% ethyl acetate in hexanes to afford 8.34 g (92%) of a mobile oil. TLC (20% ethyl acetate/80% hexane) $R_f$ = 0.40; $^1H$ NMR ($CDCl_3$) δ 7.05-7.40 (10H,m), 5.05 (2H,s), 2.87-3.20 (5H,m), 2.31 (3H,s).
Anal. ($C_{19}H_{20}O_3S \cdot 0.5\ H_2O$), calcd: C, 67.63; H, 6.27.
Found: C, 67.98; H, 6.04.

## Example 191

### 2-Benzyloxycarbonyl-3-phenyl-1-propylsulfonyl chloride.

Chlorine was bubbled into a mixture of the resultant compound from Example 190 (8.34 g, 25.4 mmol) in water (250 mL) for 30 min at room temperature followed by nitrogen which was bubbled through the mixture for 15 min. The mixture was extracted with methylene chloride which was dried over $MgSO_4$ to afford 8.55 g (95%) of an oil which was used without further purification. $^1H$ NMR ($CDCl_3$) δ 7.05-7.45 (10H,m), 5.13 (2H,s), 4.21 (1H,dd), 3.67 (1H,dd), 3.46-3.57 (1H,m), 3.16 (1H,dd), 2.94 (1H,dd).

## Example 192

### Benzyl 2-Benzyl-3-(4-methylpiperidin-1-ylsulfonyl)propionate.

The resultant compound from Example 191 (1.01 g) in methylene chloride (10 mL) at -10 °C was treated with 1-methylpiperazine (0.95 mL, 8.56 mmol). After 30 min at -10-0 °C, the mixture was evaporated, taken up in ethyl acetate, washed with saturated $NaHCO_3$ solution and brine, and was then dried over $Na_2SO_4$ and evaporated. Chromatography of the residue on silica gel (110 g) afforded 1.01 g (85%) of a white solid, m.p. 70-71 °C. TLC (5% $CH_3OH$/95% $CHCl_3$) $R_f$=0.36, $^1H$ NMR ($CDCl_3$) δ 7.07-7.43 (10H,m), 5.10 (2H,dd), 3.44 (1H,dd), 3.12-3.35 (5H,m), 3.06 (1H,dd), 2.86-2.98 (2H,m), 2.35-2.50 (4H,m), 2.30 (3H,s).

## Example 193

### Benzyl 2-Benzyl-3-(morpholin-4-ylsulfonyl)propionate.

To the resultant compound from Example 191 (0.99 g, 2.81 mmol) in methylene chloride (15 mL) at -10 °C was added morpholine (0.98 mL, 11.3 mmol). After 30 min at -10 °C the mixture was evaporated, taken up in ethyl acetate, washed with 2M HCl, saturated $NaHCO_3$ solution and brine, and then was dried over $Na_2SO_4$ and evaporated. Chromatography of the residue on silica gel (110 g) with 25-33% ethyl acetate in hexane afforded 1.02 g (90%) of a white solid, m.p. 83-85 °C. TLC (50% ethyl acetate/50% hexane) $R_f$ = 0.38; $^1H$ NMR ($CDCl_3$) δ 7.10-7.40 (10H,m), 5.10 (2H,s), 3.59-3.71 (4H,m), 3.46 (1H,dd), 3.23-3.33 (1H,m), 3.03-3.20 (5H,m), 3.85-3.98 (2H,m).

## Example 194

2-Benzyl-3-(1-methyl-piperidin-4-ylsulfonyl)propionic Acid.

The resultant compound from Example 192 (2.08 g, 4.99 mmol) and 10% Pd/C (0.850 g) in methanol (70 mL) and water (10 mL) were stirred under a hydrogen atmosphere for 2 1/2 h. The mixture was filtered with 7:1 methanol/water washes and evaporated to afford 1.52 g (93%) of a white solid, m.p. 190-194 °C. TLC (25% ethyl acetate/25% water/25% acetic acid/25% n-butanol) $R_f$ = 0.43;
Anal. ($C_{15}H_{22}N_2O_4S$•0.25 $H_2O$).
Calcd: C, 54.44; H, 6.85; N, 8.47.
Found: C, 54.77; H,6.53; N, 8.39.

Example 195

2-Benzyl-3-(morpholin-4-ylsulfonyl)propionic Acid.

The resultant compound from Example 193 (1.02 g, 2.53 mmol) and 10% Pd/C (0.50 g) in methanol (15 mL) were stirred under a hydrogen atmosphere for 3 h. The mixture was filtered and evaporated to afford 0.710 g (90%) of a white foam. $^1$H NMR ($CDCl_3$) δ 7.12-7.38 (5H,m), 3.63-3.77 (4H,m), 3.44 (1H,dd), 3.08-3.33 (6H,m), 2.97 (1H,dd), 2.92 (1H,dd).

Example 196

1-Benzyloxycarbonyl-3-hydroxyazetidine.

1-Diphenylmethyl-4-hydroxyazetidine (1.00 g, 4.18 mmol) and 10% Pd/C in methanol (10 mL) were stirred under a hydrogen atmosphere for 20 h. The mixture was filtered and evaporated, and the residue was dissolved in methylene chloride and cooled to 0 °C. After addition of triethylamine (0.64 mL, 4.57 mmol) and benzyl chloroformate (0.60 mL, 4.20 mmol), the mixture was stirred at room temperature for 90 min. The mixture was evaporated, taken up in ethyl acetate, washed with 2M HCl, saturated $NaHCO_3$ solution and brine, and then dried over $Na_2SO_4$ and evaporated. Chromatography of the residue on silica gel (120 g) with 50-60% ethyl acetate in hexane afforded 0.376 g (43%) of a colorless oil. TLC (50% ethyl acetate/50% hexane) $R_f$ = 0.13; $^1$H NMR ($CDCl_3$) δ 7.29-7.39 (5H,m), 5.10 (2H,s), 4.59-4.70 (1H,m), 4.26 (1H,dd), 4.23 (1H,dd), 3.91 (1H,dd), 3.88 (1H,dd), 2.15 (1H,d).

Example 197

3-Acetylmercapto-1-benzyloxycarbonylazetidine.

To triphenylphosphine (4.40 g, 16.8 mmol) in tetrahydrofuran (25 mL, THF) at -78 °C was added diethylazodicarboxylate (2.60 mL, 16.5 mmol) in THF (15 mL). After 7 min thiolactic acid (1.25 mL, 17.5 mmol) in THF (15 mL) was added followed by, after 7 min, the resultant compound from Example 196 (2.789 g, 13.46 mmol). The mixture was stirred at -78 °C for 1 h and then at room temperature for 20 h, and was then evaporated and chromatographed on silica gel (300 g) with 20% ethyl acetate in hexane afforded 3.250 g (91%) of a white solid, m.p. 94.5-95.5 °C. TLC (20% ethyl acetate/80% hexane) $R_f$ = 0.17; $^1$H NMR ($CDCl_3$) δ 7.28-7.41 (5H,m), 5.09 (2H,s), 4.48 (1H,d), 4.44 (1H,d), 4.15-4.26 (1H,m), 3.92 (1H,d), 3.89 (1H,d), 2.34 (3H,s).

Anal. ($C_{13}H_{15}NO_3S$).
Calcd: C, 58.85; H, 5.70, N, 5.28.
Found: C, 58.81; H, 5.70; N, 5.26.

## Example 198

### Methyl 2-Benzyl-3-(1-benzyloxycarbonyl-azetidin-3-ylmercapto)propionate.

A solution of sodium methoxide in methanol (3 mL) prepared with sodium bis(trimethylsilyl)amide (0.75 mL, 0.75 mmol, 1.0 M in THF) was added to the resultant compound from Example 197 (205.0 mg, 0.773 mmol) in methanol (3 mL). After 45 min, methyl α-benzylacrylate (150.0 mg, 0.851 mmol) in methanol (2 mL) was added. After 45 min the reaction was quenched with 2M HCl(0.38 mL, 0.76 mmol), evaporated, chromatographed on silica gel (30 g) with 20% ethyl acetate in hexane, to afford 280.6 mg (91%) of a colorless oil. TLC (20% ethyl acetate/80% hexane) $R_f$ = 0.13; $^1$H NMR ($CDCl_3$) δ 7.10-7.40 (10H,m), 5.08 (2H,s), 4.21-4.33 (2H,m), 3.77-3.90 (2H,m), 3.66 (3H,s), 3.53-3.63 (1H,m), 3.00 (1H,dd), 2.72-2.90 (3H,m), 2.63 (1H,dd).

## Example 199

### Methyl 2-Benzyl-3-(1-benzyloxycarbonyl-azetidin-3-ylsulfonyl)propionate.

The resultant compound from Example 198 (276.0 mg, 0.691 mmol) in methanol. (6 mL) and water (5 mL) was treated with OXONE (1.27 g, 2.07 mmol). After 14 h the mixture was diluted with methanol, filtered and concentrated to ca. 5 mL. After neutralization with solid $K_2CO_3$ the mixture was extracted into ethyl acetate which was washed with saturated $NaHCO_3$ solution, water, and brine, and then was dried over $Na_2SO_4$ and evaporated to afford 295.9 mg (99%) of a colorless oil. TLC (50% ethyl acetate/50% hexane) $R_f$ = 0.18; $^1$H NMR ($CDCl_3$) δ 7.10-7.40 (10H,m), 5.09 (2H,s), 4.22-4.35 (2H,m), 4.25 (1H,dd), 4.12 (1H,dd), 3.80-3.92 (1H,m), 3.73 (3H,s), 3.44 (1H,dd), 3.27-3.38 (1H,m), 3.14 (1H,dd), 2.92 (1H,dd), 2.87 (1H,dd).

## Example 200

### Methyl 2-Benzyl-3-(1-methylazetidin-3-ylsulfonyl)propionate.

The resultant compound from Example 199 (270.8 mg) and 10% Pd/C (150 mg) in methanol (6 mL) was treated with formaldehyde in water (0.25 mL, 37% formalin) and stirred under a hydrogen atmosphere for 3h. The mixture was filtered and evaporated to afford 194.3 mg (99%) of a colorless oil. TLC (15% $CH_3OH$/85% $CHCl_3$) $R_f$ - 0.60; $^1$H NMR ($CDCl_3$) δ 7.12-7.37 (5H,m), 3.77 (1H,dd), 3.71 (3H,s), 3.56 (1H,dd), 3.38-3.50 (4H,m), 3.26-3.36 (1H,m), 3.12 (1H,dd), 2.96 (1H,dd), 2.88 (1H,dd), 2.32 (3H,s).

## Example 201

### 2-Benzyl-3-(1-methylazetidin-3-ylsulfonyl)propionic Acid Hydrochloride.

The resultant compound from Example 200 (2.120 g, 6.81 mmol) in 2M HCl was stirred at 75° C for 20 h. The mixture was washed with ether, evaporated with water chasers, and lyophillized to afford 2.075 g (91%) of a white foam. TLC (25% ethyl acetate/25% water/25% acetic acid/25% n-butanol) $R_f$ = 0.50; $^1H$ NMR ($CD_3OD$) δ 7.17-7.35 (5H,m), 3.58-3.68 (2H,m), 2.95 (3H,s).

## Example 202

### Benzyl (2R)-2-Benzyl-3-(2-dimethylaminothiazol-4-yl)propionate.

The resultant compound from Example 150 (182.0 mg, 0.55 mmol) and N,N-dimethylthiourea (86 mg, 0.83 mmol) in acetone (4 ml) were stirred at room temperature for 48 h. The mixture was evaporated, taken up in ethyl acetate, washed with saturated $NaHCO_3$ solution and brine, then dried over $Na_2SO_4$ and evaported. Chromatography on silica gel (18g) with 20% ethyl acetate in hexane afforded 194 mg (93%) of an oil. TLC (50% ethyl acetate/50% hexane) $R_f$ = 0.66 $^1H$ NMR ($CDCl_3$) δ 7.10-7.30 (m,10H), 6.06 (s,1H), 5.01 (d,1H), 4.97 (d,1H), 3.15-3.30 (m,2H), 3.04 (s,6H), 2.88-3.00 (m,1H), 2.87 (dd,1H), 2.77(dd,1H).

## Example 203

### (2R)-2-Benzyl-3-(2-dimethylaminothiazol-4-yl)propionic Acid.

Using the procedure of Example 152 with the resultant compound from Example 202 gave the desired product as an oil. TLC (10% $CH_3OH$/90% $CHCl_3$) $R_f$ = 0.47; $^1H$ NMR ($CDCl_3$) δ 7.15-7.35 (m,5H), 5.95 (s,1H), 3.15(dd,1H), 3.12(s,6H), 3.00-3.15 (m,1H), 2.60-2.90 (m,3H).

## Example 204

### Benzyl (2R)-2-Benzyl-3-(2-methylimino-3-methyl-2,3-dihydrothiazol-4-yl(propionate.

The resultant compound from Example 150 (355.0 mg, 1.07 mmol) and N,N′-dimethylthiourea (98 mg, 0.94 mmol) in acetone (6 ml) were stirred at room temperature for 162 h. The mixture was evaporated, taken up in ethyl acetate, washed with 1.0 M $Na_2CO_3$ solution and brine, then dried over $Na_2SO_4$ and evaporated. Chromatography on silica gel (19g) with 3% methanol in chloroform afforded 319 mg (89%) of an oil. TLC (10% $CH_3OH$/90% $CHCl_3$) $R_f$ = 0.40; $^1H$ NMR ($CDCl_3$) δ 7.10-7.35 (m,10H), 5.51 (s,1H), 5.05 (d,1H), 5.00 (d,1H), 3.12(s,3H), 2.93-3.08 (m,2H), 2.97 (s,36H), 2.73-2.88 (m,2H), 2.51 (ddd,1H).

## Example 205

### (2R)-2-Benzyl-3-(2-methylimino-3-methyl-2,3-dihydrothiazol-4-yl)propionic Acid Hydrobromide.

The resultant compound from example from Example 204 (315mg, 0.828mmol) was stirred for 2h in 30% HBr in acetic acid (5 ml). The solvent was evaporated and the residue was dissolved in water which was washed with ether and lyophillized to afford 310mg (100%) of the desired product as a foam. $^1H$ NMR

($CD_3OD$) δ 7.20-7.35(m, 5H), 6.72(s, 1H), 3.44(s, 3H), 3.08(s, 3H), 2.70-3.20(m, 5H).

## Example 206

### Benzyl (2R) 2-Benzyl-3-(5,6-dihydroimidazo[2,1-b]thiazol-3-yl]propionate.

Using the procedure of Example 204 but replacing N,N′-dimethylthiourea with 2-imidazolidinethione gave the desired product as an oil. TLC (10% $CH_3OH$/90% $CHCl_3$) $R_f$ = 0.32; $^1H$ NMR ($CD_3OD$) δ 7.15-7.35 (m,10H), 6.42 (s,1H), 5.06 (d,1H), 5.01 (d,1H), 4.15-4.32(m,4H), 2.70-3.20 (m,4H), 2.77 (ddd,1H).

## Example 207

### (2R) 2-Benzyl-3-(5,6-dihydroimidazo[2,1-b]thiazol-3-yl]propionic Acid Hydrobromide

Using the procedure of Example 205 with the resultant compound from Example 206 gave the desired product as a foam. TLC (25% ethyl acetate/25% water 25% acetic acid/25% n-butanol) $R_f$ = 0.51; $^1H$ NMR ($CD_3OD$) δ 7.20-7.35(m, 5H), 6.51(s, 1H), 4.32(s, 4H), 3.00-3.15(m, 2H), 2.83-2.95(m, 2H), 2,72(ddd, 1H).

## Example 208

### tert-Butyl (2(S)-2-(tert-butyloxycarbamoyl)-3-phenylpropyl)sulfide.

tert-Butyl mercaptan (0.52 ml, 416 mg, 4.61 mmol) was added to a suspension of sodium hydride (111 mg, 4.62 mmol) in 8 ml of tetrahydrofuran (THF), cooled to 0° C under a nitrogen atmosphere. The resulting suspension was stirred at 0° C for 1 h, and for an additional 3 h at room temperature. To the resulting thick white suspension, cooled to 0° C, was added dropwise via cannula, a solution of 1-phenyl-3-trifluorosulfonyloxy-2-t-butyloxyamidopropane (1.63 g, 4.02 mmol) in 10 ml of THF. The resultant mixture was allowed to stir and slowly warm to room temperature over 18 h. The mixture was partitioned between 75 ml $Et_2O$ and 50 ml water. The organic phase was extracted with 50 ml saturated $NaHCO_3$, then the combined aqueous phases were extracted with 2 x 50 ml $Et_2O$. All organic phases were combined, washed with 50 ml brine, dried ($MgSO_4$), and the filtrate concentrated under reduced pressured to afford 1.40 g of orangish solid. Purification by recrystallization (hexanes, three crops) gave 1.16 g (89%) of white crystals; m.p. 67-69°C. $^1H$ NMR ($CDCl_3$) δ 1.31 (s,9H), 1.43 (s,9H), 2.57 (dd,1H), 2.66 (dd,1H), 2.84 (m,2H), 4.04 (bm,1H), 4.79 (bm,1H), 7.16-7.4 (m,5H).

## Example 209

### tert-Butyl (2(S)-2-(tert-butyloxycarbamoyl)-3-phenylpropyl)sulfone.

The resultant compound from Example 208 (863 mg, 2.67 mmol) was dissolved in 5 ml absolute ethanol and 5 ml THF, and 2.5 ml water and 5 ml pH 4.5 aqueous phosphate buffer was added. The mixture was cooled in ice and treated with OXONE (2.45 g, 8.00 mmol $KHSO_5$). The mixture was stirred

and allowed to warm to room temperature. After 60 h, the mixture was partitioned between 50 ml water and 50 ml $CH_2Cl_2$. The aqueous phase was further extracted with 3 x 50 ml $CH_2Cl_2$, and the combined organic extracts were washed with brine, dried ($MgSO_4$), filtered, and the filtrate concentrated under reduced pressure to give 942 mg of white solid. Recrystallization from $CH_2Cl_2$ $Et_2O$ (three crops) afforded 839 mg (88%) of white crystals; m.p, 169-170.5 °C. $^1$H NMR ($CDCl_3$) δ 1.38 (s,9H), 1.42 (s,9H), 3.1-3.3 (m,4H), 4.30 (bm,1H), 5.26 (bs,1H), 7.21-7.42 (m,5H). High resolution mass spectrum; calcd. for $C_{13}H_{33}NO_4S$ $(M+H)^+$:356.1895. Found: 356.1894.

## Example 210

### tert-Butyl (2(S)-2-amino-3-phenylpropyl) sulfone hydrochloride.

The resultant compound from Example 209 (741 mg, 2.09 mmol) was treated with 2.5 ml of 4.5 M HCl in dioxane at room temperature. After 24 h the volatiles were removed under reduced pressure, and the residue placed under high vacuum overnight, to afford 614 mg (100%) of the desired compound; m.p. >220 °C. $^1$H NMR ($CDCl_3$) δ 1.34 (s,9H), 3.20 (dd,1H), 3.26 (dd,1H), 3.70 (dd,1H), 3.89 (dd,1H), 4.25 (bm,1H), 7.25-7.35 (m,5H), 8.37 (bs,1H + $H_2O$).

## Example 211

### Diethyl 4-Thiazolylmethyl malonate.

Sodium metal (4.14 g, 180 mmol) was added to 150 ml of absolute ethanol and cooled in an ice-water bath under a nitrogen atmosphere. After the sodium dissolved diethyl malonate (28.83 g,. 180 mmol) was added and the solution warmed to room temperature and stirred for 2 h. The reaction mixture was recooled in an ice-water bath and 4-chloromethylthiazole (2.4 g, 18.0 mmol) in 5 ml of ethanol was added dropwise over 10 min and stirred for 20 h at room temperature. The crude reaction mixture was concentrated then partitioned between 50 ml $H_2O$ and 50 ml $CH_2Cl_2$ the organic layer was filtered through $MgSO_4$ and concentrated. The crude product was chromatographed on silica gel with 10% ethylacetate in hexane (EtOAc hex) to give 3.1 g (67%) of a clear oil. $^1$H NMR ($CDCl_3$, TMS) δ (t,6H), 3.4 (d,2H), 3.9 (t,1H), 4.15 (qd,4H), 7.0 (d,1H), 8.7 (d,1H); IR ($CDCl_3$) 2900, 2250, 1740, 1290, 1050 $cm^{-1}$. Mass spectrum: $(M+H)^+$ = 258.

Anal. Calcd for $C_{11}H_{15}NO_4S$: C, 51.34; H, 5.88; N, 5.44.
Found: C, 50.84; H, 5.82; N, 5.39.

## Example 212

### Ethyl 4-Aza-5(S)-benzyl-6-t-butylsulfonyl-3-oxo-2(R,S)-(4-thiazoylmethyl)hexanoate.

To a solution of 0.5 g (1.9 mmol) of the diethyl malonate (Example 214) in 25 ml of absolute ethanol cooled in an ice-water bath and stirred under a nitrogen atmosphere was added a solution of potassium hydroxide (0.11 g, 1.9 mmol) in 25 ml of absolute ethanol. Upon complete addition, the reaction was stirred for 72 h at room temperature and concentrated to afford the crude acid salt. The acid salt was redissolved into 30 ml of dimethylformamide (DMF), cooled in an ice-water bath. 1-Hydroxybenzotriazole (HOBT) (0.38 g, 2.88 mmol), 4-methyl morpholine (0.28 g, 2.8 mmol), tert-butyl-(2(S)-2-amino-3- phenylpropyl)sulfone hydrochloride (0.55 g, 1.9 mmol), and (1-(3-dimethyl-aminopropyl)-3-ethylcarbodiimide hydrochloride

(EDAC) (0.36 g, 1.9 mmol) were added. After 18 h, the solution was diluted with 50 ml saturated aqueous $NaHCO_3$ and extracted with 30 ml ethylacetate (EtOAc) (3X). The combined ethyl acetate extracts were washed with 25 ml 0.5 N HCl (2X), filtered through $MgSO_4$, and evaporated. The crude product was chromatographed on silica gel with 1:1 EtOAc/hex to give 0.78 g (88%) of a white solid. $^1H$ NMR ($CDCl_3$, TMS) δ 1.2 (td,6H), 1.4 (d,9H), 1.7 (s,2H($H_2O$)), 3.0-3.3 (m,4H), 3.35 (dd,2H), 3.74 (2dd,1H), 4.12 (m,4H), 4.54 (m,1H), 7.0 (dd,1H), &.1 (bd,1H), 7.25 (m,5H), 8.65 (dd,1H); IR ($CDCl_3$) 3420, 2900, 2250, 1740, 1670, 1520, 1290 1140 $cm^{-1}$. Mass spectrum: $(M+H)^+$ = 467.
Anal. Calcd. for $C_{22}H_{30}N_2O_5S_2$: C, 56.63; H, 6.48; N, 6.01.
Found: C, 56.79; H, 6.56; N. 6.01.

## Example 213

### Ethyl 4-Aza-5(R)-benzyl-6-t-butylsulfonyl-3-oxo-2(R,S)-(4-thiazoylmethyl)hexanoate.

Using the procedure for the synthesis of the ester in Example 212, but replacing tert-butyl-(2(S)-2-amino-3-phenylpropyl)sulfone hydrochloride with tert-butyl (2(R)-2-amino-3-phenylpropyl)sulfone hydrochloride gave 0.74 g (83%) of a white solid. $^1H$ NMR ($CDCl_3$, TMS) d 1.2 (td,3H), 1.35 (d,9H), 1.8 (bs,2H($H_2O$)), 3.0-3.3 (m,4H), 3.35 (dd,2H), 3.75 (t,1H), 4.15 (m,4H), 4.55 (bm,1H), 7.0 (dd,1H), 7.25 (m,5H), 8.7 (dd,1H); IR ($CDCl_3$) 3420, 2900, 2250, 1740, 1670, 1520, 1290, 1140 $cm^{-1}$. Mass spectrum: $(M+H)^+$ = 467.
Anal. Calcd. for $C_{22}H_{30}N_2O_5S_2$: C, 56.63; H, 6.48; N, 6.01.
Found: C, 56.64; H, 6.54; N, 5.99.

## Example 214

### Diethyl 2-Flouro-2-(4-thiazoylmethyl)malonate.

Sodium metal (0.15 g, 6.34 mmol) was added to 25 ml of absolute ethanol, cooled in an ice-water bath, and stirred under nitrogen until the metal had completely dissolved. Diethyl 2-fluoromalonate (1,13 g, 6.34 mmol) was added, and the solution was warmed to room temperature and stirred for 2 h. The ice bath was reapplied, and 4-chloromethylthiazole (0.85 g, 6.34 mmol) in 5 ml of ethanol was added. The solution was stirred for 18 h at room temperature, concentrated, and partitioned between 25 ml $H_2O$ and 25 ml $CH_2Cl_2$. The organic layer was filtered through $MgSO_4$ and evaporated. The crude product was chromatographed on silica gel with 10% EtOAc/hex to give 1.52 g (87%) of a colorless oil
$^1H$ NMR ($CDCl_3$, TMS) w 1.3 (t,6H), 3.75 (d,2H), 4.3 (q,4H), 7.22 (t,1H), 8.72 (d,1H); IR ($CDCl_3$) 1750 $cm^{-1}$. Mass spectrum: $(M+H)^+$ = 276.
Anal. Calcd. for $C_{11}H_{14}NO_4SF$: C, 47.99; H, 5.13; N, 5.09.
Found: C, 47.84; H, 5.19; N, 4.80.

## Example 215

### Ethyl 4-Aza-5(S)-benzyl-6-t-butylsulfonyl-2-fluoro-3-oxo-2(R,S)-(4-thiazoylmethyl)hexanoate.

Using the procedure for the synthesis of the ethyl ester in Example 212 and substituting the product from Example 214, the title compound was obtained in 92% yield. $^1H$ NMR ($CDCl_3$, TMS) δ 1.25 (m,3H), 1.35 (d,9H), 3.0-3.3 (m,5H), 3.75 (dt,2H), 4.25 (m,2H), 4.65 (m,1H), 7.1-7.35 (m,6H), 8.65 (t,1H); IR ($CDCl_3$) 3420, 2900, 2250, 1740, 1670, 1520, 1120, 1060 $cm^{-1}$. Mass spectrum: $(M+H)^+$ = 485.

Anal. Calcd. for $C_{22}H_{29}N_2O_5S_2F$: C, 54.52; H, 6.03; N, 5.78.
Found: C, 54.72; H, 6.10; N, 5.76.

## Example 216

### Ethyl 4-Aza-5(R)-benzyl-6-t-butylsulfonyl-2-fluoro-3-oxo-2(R,S)-(4-thiazoylmethyl)hexanoate.

Using the procedure for the synthesis of the ester in Example 215 but substituting tert-butyl (2(S)-2-amino-3-phenylpropyl)sulfone hydrochloride with tert-butyl (2(R)-2-amino-3-phenylpropyl)sulfone hydrochloride gave a 90% yield of the title compound. $^1$H NMR ($CDCl_3$, TMS) δ 1.25 (dt,3H), 1.4 (d,9H), 2.9-3.3 (m,5H), 3.75 (dt,2H), 4.25 (m,2H), 4.65 (m,1H), 7.1-7.35 (m,6H), 8.68 (t,1H); IR ($CDCl_3$) 3420, 2900, 2250, 1740, 1670, 1520, 1120, 1060 $cm^{-1}$. Mass spectrum: $(MM+H)^+$ = 485.
Anal. Calcd. for $C_{22}H_{29}H_2O_5S_2F$: C, 54.52; H, 6.03; N, 5.78.
Found: C, 54.68; H, 6.10; N, 5.79.

## Example 217

### 3(S)-Acetoxy-2(R,S)-benzyl-3-morpholinocarbonylpropionic Acid

To a solution of 0.55 g (1.9 mmol) of diethyl (2S,3R,S)-3-benzyl-2-hydroxysuccinate, prepared according to the procedure of D. Seebach, Org. Syn, 63, 109, in 8 ml of tetrahydrofuran, cooled in an ice-water bath was added 220 mg (5.2 mmol) of lithium hydroxide mono hydrate in 8 ml of water. The bath was removed and the reaction stirred for 18 h. The reaction mixture was acidified with concentrated HCl until pH 4 and the solvents removed under high vacuum to give a white solid. The crude diacid was warmed to 50°C in 4 ml of 1:1 acetic anhydride:acetyl chloride for 3 h. The excess acetic anhydride-acetyl chloride was removed under high vacuum. The residue was dissolved in dichloromethane, cooled in an ice-water bath, and 2 ml of morpholine was added. The reaction was stirred for 1 h, diluted with 1 N HCl, and the crude product extracted with chloroform. The combined chloroform extracts were dried ($MgSO_4$), filtered, and concentrated to give a brown oil. The product was purified by silica gel chromatography using 5:95:0.02 methanol:dichloromethane:acetic acid as eluant. Mass spectrum: $(M+H)^+$ = 336.

## Example 218

### Ethyl (2S,3R)-2-Benzyl-3-N-methylpiperazinocarbonyl-3-hydroxypropionate.

To a solution of 360 mg (1.28 mmol) of diethyl (2S,3R)-3-benzyl-2-hydroxypropionate, prepared according to the procedure of D. Seebach (example 217) in 1 ml tetrahydrofuran cooled in an ice-water bath was added a 1 ml solution of 50 mg (1.28 mmol) of lithium hydroxide monohydrate. The bath was removed and the reaction stirred for one hour. Aqueous saturated potassium hydrogen sulfate was added and the product acid was extracted with methylene chloride. The crude monoacid was dissolved into 2 ml methylene chloride and 0.15 ml of N-methylpiperazine, 0.2 ml ethyl diisopropylamine and 0.2 ml of diethyl phosphonocyanide. The reaction mixture was stirred for 18 h at room temperature, concentrated under high vacuum. The crude residue was sumitted to flash chromatography purification using 1:4 (methanol:ethyl acetate) as eluant. 150 mg of amide was obtained. $^1$H NMR ($CDCl_3$, TMS) 1.2 (t,3H), 2.3 (s,3H), 4.15 (q,2H), 7.2-7.35 (m,5H); IR 3400, 1735, 1640 $cm^{-1}$ Mass Spectrum; M+H (high resolution) Calcd. for $C_{18}H_{26}N_2O_4$: 334.1893: Observed: 334.1908.

Example 219

(2S,3R)-2-Benzyl-3-N-methylpiperazinocarbonyl-3-hydroxypropioic acid Lithium Salt.

A solution of 0.15 g (0.449 mmol) of ester from Example 218 in 3 ml tetrahydrofuran was treated with 21 mg (0.5 mmol) of lithium hydroxide mono hydrate dissolved in 3 ml water. The reaction was stirred at room temperature for 18 h. The solvents were removed under high vacuum to yield 142 mg of a white solid lithium salt which was used without further purification.

Example 220

4-Aza-5(S)-benzyl-6-t-butylsulfonyl-3-oxo-2(R,S)-(4-thiazolymethyl)hexane carboxamide of (2S,3R,4S)-4-Amino-5-cyclohexyl-2,3-dihydroxy-1-(N-methyl-N-trifluoroethylamino)pentane.

The ethyl ester (0.20 g, 0.4 mmol) obtained from Example 212 and potassium hydroxide (0.024 g, 0.4 mmol) in 2 ml of absolute ethanol is stirred at room temperature under nitrogen atmosphere for 18 h and concentrated. The resulting acid salt, 1-HOBT (0.043 g, 0.4 mmol), 4-methylmorpholine (0.12 g, 0.40 mmol), and the resultant compound from Example 32 are dissolved in 5 ml DMF, using the procedure of Example 33, to give the the title compound.

Example 221

4-Aza-5(R)-benzyl-6-t-butylsulfonyl-3-oxo-2(R,S)-(4-thiazoylmethyl)hexane carboxamide of (2S,3R,4S)-4-Amino-5-cyclohexyl-2,3-dihydroxy-1-(N-methyl-N-trifluoroethylamino)pentane.

Using the procedure for the synthesis of the title compound in Example 220, but substituting the ethyl ester in Example 213 for the ethyl ester in Example 212 gives the product after chromatography purification.

Example 222

4-Aza-5(S)-benzyl-6-t-butylsulfonyl-2-fluoro-3-oxo-2(R,S)-(4-thiazoylmethyl)hexane carboxamide of (2S,3R,4S)-4-Amino-5-cyclohexyl-2,3-dihydroxy-1-(N-methyl-N-trifluoroethylamino)pentane.

Using the procedure for the synthesis of the title compound in Example 220 but substituting the ethyl ester in Example 215 gives the product.

Example 223

4-Aza-5(R)-benzyl-6-t-butylsulfonyl-2-fluoro-3-oxo-2(R,S)-(4-thiazoylmethyl)hexane carboxamide of (2S,3R,4S)-4-Amino-5-cyclohexyl-2,3-dihydroxy-1-(N-methyl-N-trifluoroethylamino)pentane.

Using the procedure for the synthesis of the compound in Example 220 but substituting the ethyl ester in Example 216 gives the product.

Example 224

3(S)-Acetoxy-2(R,S)-benzyl-3-morpholinocarbonylpropionyl-L-(1-pyrazolyl)ala Amide of (2S,3R,4S)-4-Amino-5-cyclohexyl-2,3-dihydroxy-1-(N-methyl-N-trifluoroethylamino)pentane.

Using the coupling procedure of Example 33 with the resultant compound from Example 36 and replacing Boc-His-OH with the resultant compound from Example 217 gave the desired product.

Example 225

3(S)-Hydroxy-2(R,S)-benzyl-3-morpholinocarbonylpropionyl-L-(1-pyrazolyl)ala Amide of (2S,3R,4S)-4-Amino-5-cyclohexyl-2,3-dihydroxy-1-(N-methyl-N-trifluoroethylamino)pentane

Using the procedure of Example 39 with the resultant compound from Example 224 gave the desired product.

Example 226

(2S,3R)-2-Benzyl-3-N-methylpiperazinocarbonyl-3-hydroxypropionyl-L-(1-pyrazolyl)ala Amide of (2S,3R,4S)-4-Amino-5-cyclohexyl-2,3-dihydroxy-1-(N-methyl-N-trifluoroethylamino)pentane.

Using the coupling procedure of Example 33 with the resultant compound from Example 36 and replacing Boc-His-OH with the resultant compound from Example 219 gave the desired product.

Example 227

6-Aminohexanoyl-(Me)Tyr-His Amide of (2S, 3R, 4S)-4-Amino-5-cyclohexyl-2,3-dihydroxy-1-(N-methoxy-N-methylamino)pentane.

Using the coupling procedure of Example 33 and replacing Boc-His-OH with the resultant acid from Example 69 and then using the deprotection procedure of Example 33 gave the desired compound.

Example 228

(2R)-2-Benzyl-3-(4-trifluoroethylpiperazin-1-ylcarbonyl)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 141 gave the desired compound.

Example 229

(2R)-2-Benzyl-3-(4-methylpiperazin-1-ylcarbonyl)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 143 gave the desired compound.

Example 230

(2R)-2-Benzyl-3-((2-pyridin-2-ylethyl)methylaminocarbonyl)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 145 gave the desired compound.

Example 231

(2R)-2-Benzyl-3-((N-pyridin-4-yl)methylaminocarbonyl)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 147 gave the desired compound.

Example 232

(2S)-2-(4-Morpholinyl)-3-phenylpropionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 149 gave the desired compound.

Example 233

(2R)-2-Benzyl-3-thiazol-4-ylpropionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 152 gave the desired compound.

Example 234

(2R)-2-Benzyl-5-tert-butylsulfinyl-4-oxopentanoyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 156 gave the desired compound.

Example 235

(2R)-2-Benzyl-5-tert-butylsulfonyl-4-oxopentanoyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 157 gave the desired compound.

Example 236

(2R)-2-Benzyl-5-morpholin-4-yl-4-oxopentanoyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 159 gave the desired compound.

Example 237

(2RS)-2-Benzyl-3-pyrazol-1-ylpropionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 163 gave the desired compound as a mixture of diastereomers which could be separated by chromatography on silica gel.

Example 238

(2RS)-2-Benzyl-3-(N-methoxyl-N-methylamino)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 164 gave the desired compound as a mixture of diastereomers which could be separated by chromatography on silica gel.

## Example 239

(2S)-2-Benzyl-3-tert-butylsulfonylpropionyl-His Amide of (2S, 3R, 4S)-4-Amino-5-cyclohexyl-2,3-dihydroxy-1-(N-methoxy-N-methylamino)pentane.

Using the coupling procedure of Example 34 with the resultant compound from Example 34 and replacing Boc-Nle-OH with the resultant acid from Example 170 and then deprotecting the resulting imidazole-Boc material by stirring in 4:1:1 acetic acid/tetrahydrofuran/water at 45-50°C for 12h gave the desired compound after an extractive isolation.

## Example 240

Isonicitonyl-(O-methyl)tyrosine-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 172 gave the desired compound.

## Example 241

1-Methylisonipicotonyl-(O-methyl)tyrosine-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 174 gave the desired compound.

## Example 242

1-Methylpiperazin-4-ylcarbonyl-(O-methyl)tyrosine-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 176 gave the desired compound.

## Example 243

(2S)-(4-Methylpiperazin-1-yl)carbonyloxy-3-phenylpropionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 178 gave the desired compound.

## Example 244

(2R)-2-Benzyl-3-((2-morpholin-4-ylethyl)methylaminocarbonyl)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 180 gave the desired compound.

## Example 245

(2R)-2-Benzyl-3-((2-imidazol-1-ylethyl)methylaminocarbonyl)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 182 gave the desired compound.

## Example 246

(2R)-2-Benzyl-3-(2-(4-methylpiperazin-1-ylethyl)methylaminocarbonyl)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 184 gave the desired compound.

## Example 247

(2R)-2-Benzyl-3-((2-diethylaminoethyl)methylaminocarbonyl)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 186 gave the desired compound.

Example 248

(2R)-2-Benzyl-3-((2-pyrazol-1-ylethyl)methylaminocarbonyl)propionyl-Nle Amide of (2S, 3R, 4S)-4-Amino-5-cyclohexyl-2,3-dihydroxy-1-(N-methoxy-N-methylamino)pentane.

Using the coupling procedure of Example 33 with the resultant compound from Example 34 and replacing Boc-His-OH with the resultant acid from Example 188 gave the desired compound.

Example 249

(2R)-2-Benzyl-3-(1-methyl-piperidin-4-ylsulfonyl)propionyl-L-(1-pyrazolyl)ala Amide of (2S,3R,4S)-4-Amino-5-cyclohexyl-2,3-dihydroxy-1-(N-methyl-N-trifluoroethylamino)pentane.

Using the coupling procedure of Example 33 with the resultant compound from Example 36 and replacing Boc-His-OH with the resultant compound from Example 194 gave the desired product after diasteriomer separation by flash chromatography.

Example 250

(2R)-2-Benzyl-3-(morpholin-4-ylsulfonyl)propionyl-L-(1-pyrazolyl)ala Amide of (2S,3R,4S)-4-Amino-5-cyclohexyl-2,3-dihydroxy-1-(N-methyl-N-trifluoroethylamino)pentane.

Using the coupling procedure of Example 33 with the resultant compound from Example 36 and replacing Boc-His-OH with the resultant compound from Example 195 gave the desired product after diasteriomer separation by flash chromatography.

Example 251

(2R)-2-Benzyl-3-(1-methylazetidin-3-ylsulfonyl)propionyl-L-(1-pyrazolyl)ala Amide of (2S,3R,4S)-4-Amino-5-cyclohexyl-2,3-dihydroxy-1-(N-methyl-N-trifluoroethylamino)pentane.

Using the coupling procedure of Example 33 with the resultant compound from Example 36 and replacing Boc-His-OH with the resultant compound from Example 201 gave the desired product after diasteriomer separation by flash chromatography.

Example 252

(2R)-2-Benzyl-3-(2-dimethylaminothiazol-4-yl)propionnyl-L-(1-pyrazolyl)ala Amide of (2S,3R,4S)-4-Amino-5-cyclohexyl-2,3-dihydroxy-1-(N-methyl-N-trifluoroethylamino)pentane.

Using the coupling procedure of Example 33 with the resultant compound from Example 36 and replacing Boc-His-OH with the resultant compound from Example 203 gave the desired product.

Example 253

(2R)-2-Benzyl-3-(2-methylimino-3-methyl-2,3-dihydrothiazol-4-yl)propionyl-L-(1-pyrazolyl)ala Amide of (2S,3R,4S)-4-Amino-5-cyclohexyl-2,3-dihydroxy-1-(N-methyl-N-trifluoroethylamino)pentane.

Using the coupling procedure of Example 33 with the resultant compound from Example 36 and replacing Boc-His-OH with the resultant compound from Example 205 gave the desired product.

Example 254

(2R)2-Benzyl-3-(5,6-dihydroimidazo[2,1-b]thiazol-3-yl]propionyl-L-(1-pyrazolyl)ala Amide of (2S,3R,4S)-4-Amino-5-cyclohexyl-2,3-dihydroxy-1-(N-methyl-N-trifluoroethylamino)pentane.

Using the coupling procedure of Example 33 with the resultant compound from Example 36 and replacing Boc-His-OH with the resultant compound from Example 207 gave the desired product.

Example 255

H-Met Amide of (2S,3R,4S)-Amino-5-cyclohexyl-2,3-dihydroxy-1-(N-methoxy-N-methylamino)pentane.

Boc-Met-OH ( 1.00 mmol) in methylene chloride (10 mL) at -10 °C was treated with N-methylmorpholine (1.10 mmol) and then with isobutylchloroformate (1.00 mmol). After 3 min. the resultant amine from Example 31 (1.00 mmol) was added and the reaction was stirred at -10 °C for 10 min and at room temperature for 2 h. The mixture was evaporated, taken up in ethyl acetate, washed with water, saturated $NaHCO_3$ solution, and brine, then dried and evaporated. Chromatography of the residue on silica gel followed by N-deprotection according to the procedure of Example 33 provided the desired product.

Example 256

Boc-Phe-Met Amide of (2S,3R,4S)-Amino-5-cyclohexyl-2,3-dihydroxy-1-(N-methoxy-N-methylamino)pentane.

Using the coupling procedure of Example 255 with the resultant compound from Example 255 and

replacing Boc-Met-OH with Boc-Phe-OH gave the desired product.

Example 257

H-Phe-Met Amide of (2S,3R,4S)-Amino-5-cyclohexyl-2,3-dihydroxy-1-(N-methoxy-N-methylamino)pentane.

Using the deprotection procerure of Example 33 with the resultant compound from Example 256 gave the desired product.

Example 258

(2S,3R,4S)-4-t-Butyloxycarbonylamino-5-cyclohexyl-2-hydroxy-1-(morpholin-4-yl)-3-(methoxyethoxymethoxy)-pentane.

The desired compound was prepared according to the procedure of Example 30, but replacing N-methyl-O-methylhroxylamine hydrochloride and $NaHCO_3$ with morpholine.

Example 259

(2S,3R,4S)-4-t-Butyloxycarbonylamino-5-cyclohexyl-2-hydroxy-1-(imidazo-1-yl)-3-(methoxyethoxymethoxy)-pentane.

The desired compound was prepared according to the procedure of Example 30, but replacing N-methyl-O-methylhroxylamine hydrochloride and $NaHCO_3$ with imidazole.

Example 260

H-Nle Amide of (2S,3R,4S)-Amino-5-cyclohexyl-2,3-dihydroxy-1-(morpholin-4-yl)pentane.

The resultant compound from Example 258 was deprotected according to the procedure of Example 31 and then was treated according to the procedure of Example 34 to give the desired product.

Example 261

H-L-(1-Pyrazolyl)Ala Amide of (2S,3R,4S)-4-Amino-5-cyclohexyl-2,3-dihydroxy-1-(imidazol-1-yl)pentane.

The resultant compound from Example 259 was deprotected according to the procedure of Example 31 and then was treated according to the procedure of Example 36 to give the desired product.

## Example 262

(2R)-2-Benzyl-3-tert-butylsulfonylpropionyl-Nle Amide of (2S,3R,4S)-Amino-5-cyclohexyl-2,3-dihydroxy-1-(morpholin-4-yl)pentane.

Using the coupling procedure of Example 33 with the resultant compound from Example 260 and replacing Boc-His-OH with the resultant acid from Example 167 gave the desired compound as a mixture of diastereomers which could be separated by chromatography on silica gel.

## Example 263

(2R) 2-Benzyl-3-(1-methyl-piperidin-4-ylsulfonyl)propionyl-L-(1-Pyrazolyl)Ala Amide of (2S,3R,4S)-4-Amino-5-cyclohexyl-2,3-dihydroxy-1-(imidazol-1-yl)-pentane.

Using the coupling procedure of Example 33 with the resultant compound from Example 261 and replacing Boc-His-OH with the resultant acid from Example 194 gave the desired compound as a mixture of diastereomers which could be separated by chromatography on silica gel.

## Example 264

Boc-Phe-Leu (2S,3R,4S)-Amide of 1-Methoxylamino-2,3-dihydroxy-4-amino-5-cyclohexylpentane

Using the proccedure of Example 24, but replacing Boc-Phe-His-OH with Boc-Phe-Leu-OH, with the resultant compound from Example 8 provided the desired product. Analysis calculated for $C_{32}H_{54}N_4O_7$.0.5 $H_2O$: C, 62.41; H, 9.00; N, 9.09. Found: C, 62.17; H, 8.72; N, 8.69.
$^1$H NMR ($CD_3OD$) 7.17-7.30 (m, 5H), 4.45 (t, J=6.0, 6.0 Hz, 1H), 4.32 (dd, J=4.5 Hz, 9.5 Hz, 1H), 4.24 (br d, J=9.5 Hz, 1H), 3.49 (s, 3H), 3.2-3.4 (m obscured by $CD_3OD$), 3.16 (dd, J=14.0 Hz, 5.5 Hz, 1H), 2.82 (dd, J=15.0, 9.5 Hz, 1H), 2.77 (dd, J=14.0, 8.5 Hz, 1H), 1.55-1.87 (m, 8H), 3.36 (s, 9H), 1.13-1.45 (m, 4H), 0.75-1.03 (m, 7H).

## Example 265

(5-Amino-5-(methoxycarbonyl)pentyl)carbamoyl-(Me)Tyr-His Amide of (2S,3R,4S)-4-Amino-cyclohexyl-2,3-dihydroxy-1-(N-methoxy-N-methylamino)pentane

Using the procedure of Example 33, (5-(t-butyloxycarbonyl)amino-5-(methoxycarbonyl)pentyl)-carbamoyl-(Me)Tyr-OH is coupled to the product of Example 33 and deprotected to provide the desired product.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate,

lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl., and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other salts include salts with alkali metals or alkaline earth metals, such as sodium, potassium, calcium or magnesium or with organic bases.

The compounds of the present invention can also be used in the form of esters. Examples of such esters include a hydroxyl-substituted compound of formula I which has been acylated with a blocked or unblocked amino acid residue, a phosphate function, or a hemisuccinate residue. The amino acid esters of particular interest are glycine and lysine; however, other amino acid residues can also be used. These esters serve as pro-drugs of the compounds of the present invention and serve to increase the solubility of these substances in the gastrointestinal tract. These pro-drugs are metabolized in vivo to provide the hydroxyl-substituted compound of formula I. The preparation of the pro-drug esters is carried out by reacting a hydroxyl-substituted compound of formula I with an activated amino acyl, phosphoryl or hemisuccinyl derivative. The resulting product is then deprotected to provide the desired pro-drug ester.

The novel compounds of the present invention possess an excellent degree of activity and specificity in treating hypertension in a host. The compounds of the invention are also useful for treating congestive heart failure. The ability of the compounds of the invention to inhibit human renal renin can be demonstrated in vitro by reacting a selected compound at varied concentrations with human renal renin, free from acid proteolytic activity, and with renin substrate (human angiotensinogen) at 37 degress C and pH of 6.0. At the end of the incubation, the amount of angiotensin I formed is measured by radioimmunoassay and the molar concentration required to cause 50% inhibition, expressed as the $IC_{50}$, is calculated. When tested in accordance with the foregoing procedure, the compounds of the invention demonstrated $IC_{50}$'s in the range of $10^{-5}$ to $10^{-10}$ M.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing of wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional

substances other than inert diluents, e.g., lubricating agents such as magnesium stearate. In the case of capsules, tables, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The compounds of the present invention are also useful for treating glaucoma or reducing and/or controlling intraocular presure. Compositions for this purpose are administered as topical or systemic pharmaceutical compositions. These compositions are preferably administered as topical pharmaceutical compositions suitable for opthalmic administration, in a pharmaceutical vehicle such as pharmaceutically acceptable sterile aqueous or nonaqueous solutions, suspensions, emulsions, ointments and solid insersts.

Examples of suitable pharmaceutically acceptable vehicles for opthalmic administration are water, propylene glycol and other pharmaceutically acceptable alcohols, sesame or peanut oil and other pharmaceutically acceptable oils, petroleum jelly, water soluble opthalmically acceptable non-toxic polymers such as methyl cellulose, carboxymethyl cellulose salts, hydroxyethyl cellulose, hydroxypropyl cellulose; acrylates such as polyacrylic acid salts; ethylacrylates; polyacrylamides; natural products such as gelatin, alginates, pectins, tragacanth, karaya, agar, acacia; starch derivatives such as starch acetate, hydroxyethyl starch ethers, hydroxypropyl starch; as well as other synthetic derivatives such as polyvinyl alcohol, polyvinyl pyrrolidone, polyvinyl methyl ether, polyethylene oxide, carbopol and xanthan gum; and mixtures of these polymers. Such compositions may also contain adjuvants such as buffering, preserving, wetting, emulsifying and dispersing agents. Suitable preserving agents include antibacterial agents such as quaternary ammonium compounds, phenylmercuric salts, benzyl alcohol, phenyl ethanol; and antioxidants such as sodium metabisulfite, butylated hydroxyanisole and butylated hydroxytoluene. Suitable buffering agents include borate, acetate, gluconate and phosphate buffers.

The pharmaceutical opthalmic compositions of the invention amy also be in the form of a solid insert. A solid water soluble or water swellable polymer such as dextran, hydroxyloweralkyl dextran, carboxymethyl dextran, hydroxyloweralkyl cellulose, loweralkyl cellulose, carboxymethyl cellulose, polyvinyl alcohol, dextrin, starch, polyvinyl pyrrolidone and polyalkylene glycols may be used as the carrier for the drug.

Dosage levels of the active compound in the compositons for treating glaucoma or reducing and/or controlling intraocular pressure may be varied so as to obtain a desired therapeutic response to a particular composition. Generally, the active compound will be administered as an isotonic aqueous solution of from 0.00001 to 1.0 (w/v) percent concentration. More preferably, the active compound will be administered as an isotonic aqueous solution of from 0.00001 to 0.1 (w.v) percent concentration.

The term "controlling intraocular pressure" as used herein means the regulation, atenuation and modulation of increased intraocular tension. The term also means that the decrease, in the otherwise elevated intraocular pressure, obtained by the methods and compositions of the invention is maintained for a significant period of time as, for example, between consecutive doses of the composition of the invention.

The effect on intraocular pressure of the compounds of the invention can be determined in rabbits by using the following method.

Effects of Topically Administered Renin Inhibiting Compounds on Intraocular Pressure in Rabbits

a. Method The antiglaucoma activity of the compounds is tested by measuring the effect on intraocular pressure in rabbits as described by Tinjum, Acta Opthalmologica, 50 677 (1972). Male albino, New Zealand rabbits are placed in restraining devices and the intraocular pressure is measured with an applamatic tonometer. Exactly 0.1 ml of an isotonic saline solution containing a test compound is instilled into the conjunctival sac and the intraocular pressure is measured at 5, 15, 30, 60, 90, 120 and 180 minutes afterwards.

The foregoing is merely illustrative of the invention and is not intended to limit the invention to the disclosed compounds. Variations and changes which are obvious to one skilled in the art are intended to be within the scope and nature of the invention which are defined in the appended claims.

## Claims

1. A compound of the formula:

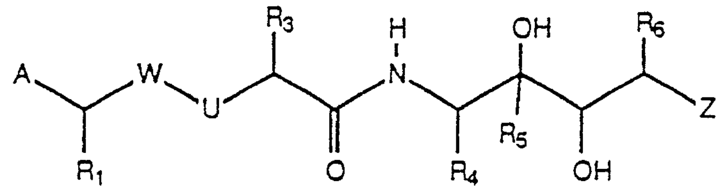

or a pharmaceutically acceptable salt, ester or prodrug thereof, wherein:
A is
(1) hydrogen,
(2) loweralkyl,
(3) carboxy,
(4) alkoxycarbonyl,
(5) $-OR_8$ or $-SR_8$
wherein $R_8$ is
(i) hydrogen,
(ii) loweralkyl,
(iii) aminoalkyl,
(iv) (alkyl)aminoalkyl,
(v) dialkylaminoalkyl,
(vi) (alkoxy)aminoalkyl,
(vii) (alkoxy) (alkyl)aminoalkyl,
(viii) phenylalkyl,
(ix) (substituted phenyl)alkyl wherein substituted phenyl is as defined above,
(x) naphthylalkyl,
(xi) (substituted naphthyl)alkyl wherein substituted naphthyl is as defined above,
(xii) heterocyclic,
(xiii) (heterocyclic)alkyl, or
(xiv) (heterocyclic)C(O)-,
(6) heterocyclic,
(7) functionalized alkyl,
(8) functionalized amino,
(9) functionalized carbonyl,
(10) functionalized sulfonyl or
(11) functionalized hydroxyalkyl;
W is
(1) -C(O)-,
(2) -CH(OH)- or
(3) $-N(R_2)-$ wherein $R_2$ is hydrogen or loweralkyl;
U is
(1) -C(O)-,
(2)$-CH_2-$ or
(3) $-N(R_2)-$ wherein $R_2$ is hydrogen or loweralkyl, with the proviso that when W is -CH(OH)-then U is $-CH_2-$ and with the proviso that U is -C(O)- or $-CH_2-$ when W is $-N(R_2)-$;
$R_1$ is
(1) loweralkyl,
(2) cycloalkylalkyl,
(3) benzyl,
(4) (alpha, alpha)-dimethylbenzyl,
(5) (substituted phenyl)methyl wherein substituted phenyl is independently as defined above,
(6) (alpha, alpha)-dimethyl-(substituted phenyl)methyl wherein substituted phenyl is independently as defined above,
(7) (1-naphthyl)methyl,

(8) (2-naphthyl)methyl,
(9) (heterocyclic)methyl,
(10) phenethyl,
(11) 1-benzyloxyethyl,
(12) phenoxy,
(13) thiophenoxy or
(14) anilino, provided that B is -CH2- or -CH(OH)- or A is hydrogen when $R_1$ is phenoxy, thiophenoxy or anilino:
$R_3$ is
(1) loweralkyl,
(2) loweralkenyl,
(3) hydroxyalkyl,
(4) alkoxyalkyl,
(5) ((alkoxy)alkoxy)alkyl,
(6) carboxyalkyl,
(7) (thioalkoxy)alkyl,
(8) azidoalkyl,
(9) aminoalkyl,
(10) (alkyl)aminoalkyl,
(11) dialkylaminoalkyl,
(12) (alkoxy)(alkyl)aminoalkyl,
(13) (alkoxy)aminoalkyl,
(14) benzyl or
(15) (heterocyclic)methyl;
$R_4$ is
(1) loweralkyl,
(2) cycloalkylmethyl or
(3) benzyl;
$R_5$ and $R_6$ are independently selected from
(1) hydrogen and
(2) loweralkyl; and
Z is
(1) -N($R_7$) ($R_{12}$) wherein
$R_7$ is
(1) loweralkyl,
(2) haloalkyl or
(3) alkoxy; and
$R_{12}$ is
(1) hydrogen,
(2) loweralkyl or
(3) haloalkyl; or
(2) heterocyclic.

2. The compound of Claim 1 wherein B is -NH-, W is -C(O)-, U is -NH-, $R_1$ is benzyl or 4-methoxybenzyl, $R_3$ is (4-imidazolyl)methyl, (pyrazolyl)methyl or (4-thiazolyl)methyl, R4 is cyclohexylmethyl, and Z is -$NR_7R_{12}$ wherein $R_7$ is alkoxy of haloalkyl and $R_{12}$ is hydrogen or loweralkyl, or Z is heterocyclic..

3. A pharmaceutical composition for treating hypertension or congestive heart failure, comprising a pharmaceutical carrier and a therapeutically effective amount of a compound of Claim 1.

4. A method of treating hypertension or congestive heart failure comprising administering to a host in need of such treatment a therapeutically effective amount of a compound of Claim 1.

5. A pharmaceutical composition for treating glaucoma or reducing and/or controlling intraocular pressure comprising a pharmaceutical vehicle suitable for topical or systemic administration and a therapeutically effective amount of a compound of Claim 1.

6. A method for treating glaucoma or reducing and/or controlling intraocular pressure comprising administering to a patient in need a therapeutically effective amount of a compound of Claim 1.

7. A process for the preparation of the compound of Claim 1 comprising coupling a carboxylic acid of the formula

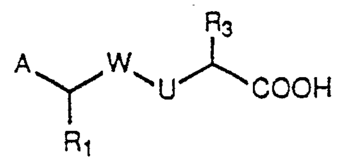

or an acid halide or activated ester derivative thereof wherein A, $R_1$, W, U and $R_3$ are as defined herein with a compound of the formula

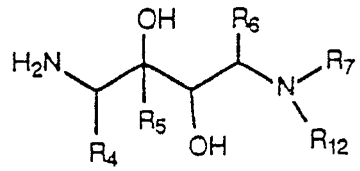

wherein $R_4$, $R_5$, $R_6$, $R_7$ and $R_{12}$ are as defined herein.

8. A process for the preparation of the compound of Claim 1 wherein W is -C(O)- and U is -N($R_2$)- comprising coupling an N-protected amino acid of the formula

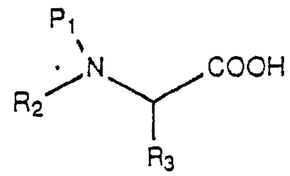

or an acid halide or an activated ester derivative thereof wherein $P_1$ is an N-protecting group and $R_2$ and $R_3$ are as defined above with a compound of the formula

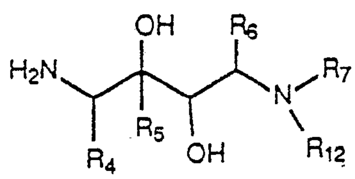

wherein $R_4$, $R_5$, $R_6$, $R_7$ and $R_{12}$ are as defined herein, followed by removal of the N-protecting group and coupling with a carboxylic acid of the formula

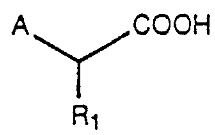

or an acid halide or activated ester derivative thereof wherein A and $R_1$ are as defined herein.